# EUROPEAN PATENT APPLICATION

(11) **EP 4 763 998 A1**
(43) Date of publication of application: **24.06.2026**
(21) Application number: 24221512.7
(22) Date of filing: 19.12.2024
(51) Int. Cl.: C12Q 1/6886

(54) **SMALL EXTRACELLULAR VESICLE (SEV) RNA SIGNATURE OF HEAD AND NECK SQUAMOUS CELL CARCINOMA (HNSCC) AS BIOMARKER FOR TUMOR ACTIVITY, TUMOR STAGE AND HPV STATUS**

(71) Applicant: Universität Ulm, 89081 Ulm (DE)
(72) Inventor: THEODORAKI, Marie-Nicole, 89081 Ulm (DE); HOFMANN, Linda, 89081 Ulm (DE); HOFFMANN, Thomas K., 89081 Ulm (DE)
(74) Representative: Michalski Hüttermann & Partner mbB

(57) **Abstract**

The present invention relates to a method for identyfying a head-and-neck cancer, comprising isolating small extracellular vesicles (sEVs) from a biological sample, isolating RNA molecules from the small extracellular vesicles (sEVs) obtained, wherein said RNA molecules comprises a biomarker set of messenger RNAs (mRNA) and/or microRNAs (miRNA), and determining the presence and/or amount of said mRNAs and/or miRNAs, and relating the presence and/or amount of said mRNAs and/or miRNAs to a reference. The present invention further relates to a method of detecting a set of biomarkers associated with the presence of head-and-neck cancer, comprising isolating small extracellular vesicles (sEVs) from a biological sample, contacting said small extracellular vesicles (sEVs) to one or more than one reagent that specifically binds to one or more than one biomarker; and determining whether or not the biomarker is differentially expressed in the sample by comparing the expression level of the biomarker in the biological sample to the expression level of said biomarker for at least one reference sample. The present invention also relates to the biomarker set associated with head-and-neck cancer, detected by the methods above, and represented by messenger RNAs (mRNAs) and/or microRNAs (miRNAs).

## Description

### Field of the invention

The present invention relates to a method for identyfying a head-and-neck cancer, comprising isolating small extracellular vesicles (sEVs) or "exosomes" from a biological sample, isolating RNA molecules from the small extracellular vesicles (sEVs) obtained, wherein said RNA molecules comprises a biomarker set of messenger RNAs (mRNA) and/or microRNAs (miRNA), and determining the presence and/or amount of said mRNAs and/or miRNAs, and relating the presence and/or amount of said mRNAs and/or miRNAs to a reference.

The present invention further relates to a method of detecting a set of biomarkers associated with the presence of head-and-neck cancer, comprising isolating small extracellular vesicles (sEVs) from a biological sample, contacting said small extracellular vesicles (sEVs) to one or more than one reagent that specifically binds to one or more than one biomarker; and determining whether or not the biomarker is differentially expressed in the sample by comparing the expression level of the biomarker in the biological sample to the expression level of said biomarker for at least one reference sample.

The present invention also relates to the biomarker set associated with head-and-neck cancer, detected by the methods above, and represented by messenger RNAs (mRNAs) and/or microRNAs (miRNAs).

### Background

Head and neck cancer (HNC) is a highly aggressive type of tumor characterized by delayed diagnosis, recurrence, metastasis, relapse, and drug resistance. The occurrence of HNC was associated with smoking, alcohol abuse (or both), human papillomavirus infection, and complex genetic and epigenetic predisposition (Liu *et al.* 2024).

Head and neck cancer is the 7^{th} most common tumor worldwide. About 90% of these cases are head and neck squamous cell carcinoma (HNSCC). According to GLOBOCAN, there are estimates of 900,000 new cases and 450,000 deaths per year worldwide. Every year, 30,000 new cases are registered in Germany, and 180,000 in Europe. Despite various innovations in tumor therapy, the survival of these patients remains poor, mainly because the carcinomas are detected late and at an advanced tumor stage.

HNC is characterized by a complex signaling network involving numerous genes and proteins. In HNC, there is an overexpression of four key cell surface receptors: EGFR, VEGFR, HER2, and MET. These receptors drive downstream pathways that are crucial for cell proliferation, apoptosis, immune escape and metastasis (Liu *et al.* 2024).

The immune system is known to be an important factor for successfully achieving cancer control and limiting its progression. Unfortunately, tumors have developed mechanisms to avoid this anti-tumor immune response, which is known as immune evasion. Advanced head and neck tumors with an early locoregional metastasis are generally known to be more immunosuppressive than early staged malignancies. Infiltration of tumor microenvironments (TME) with CD8+ cytotoxic T-cells (CTLs) predicts prolonged survival of patients with multiple types of tumors and is critical for the clinical effectiveness of different forms of immunotherapy. In contrast, intratumoral accumulation of regulatory T cells (Treg) and myeloid derived suppressor cells predicts resistance to treatments and accelerated cancer progression.

One way of immune suppression represent tumor derived extracellular vesicles (EV), especially small extracellular vesicles (sEVs). sEVs are small sized and differ by other EVs by their origin-unique cargo characteristics. They include exosomes and ectosomes. They are released by all cell types, but in the setting of stress, *e.g*., cancer disease, the production and release of EVs is higher compared to healthy donors. EVs are able to move in body fluids like the blood circulation and deliver signals from the host cell to other cells and other parts of the body.

Tumor derived small extracellular vesicles (sEVs) (TEX) have to be distinguished from all EVs circulating in the patient's plasma. TEX carry the cargo of proteins, nucleic acids, surface receptors and ligands resembling the tumor cell and can be therefore distinguished from EVs of normal human cells. TEX are virus-sized stable vesicles circulating in body fluids and being able to interact in different ways with immune cells. Through their ability to move freely in body fluids they can deliver suppressive but also stimulatory signals to different subsets of immune cells and induce a communication network between the host tumor cells and the immune cells. The characteristics of their cargo depend on the tumor tissue's conditions, in other words the tumor microenvironment (TME). Therefore, TEX are involved in cell signaling and inter-cellular interactions. Head and Neck Squamous Cell Carcinoma (HNSCC) are avid sEV producers and the plasma of HNSCC patients is enriched in TEX. sEVs have been shown to carry more than 10,000 RNAs with many of them being involved in immune regulation.

Genetic regulation through sEVs is represented by microRNAs (miRNAs), which are noncoding RNAs that suppress mRNA translation and therefore play a role in various cell pathways like proliferation and apoptosis. MiRNAs are highly represented in the EV cargo with 10% of miRNAs found in them. After internalization of TEX, miRNAs interfere with gene expression and can inhibit gene translation or even degrade specific mRNA.

Small extracellular vesicles (sEVs) have great potential as liquid biomarkers, and biomarkers are of high importance to improve HNSCC patient's outcome.

If a patient notices clinical symptoms, *e.g*., a sore throat or lymph node swelling in the neck, weight loss or shortness of breath, the family doctor or ear-, nose- and throat (ENT) doctor is consulted. The current gold standard for diagnosis consists of a clinical examination, a biopsy taken under general anesthesia, and imaging (computer tomography (CT) of neck/thorax/abdomen or magnet resonance imaging (MRI) of neck/CT thorax/abdomen). The process until final diagnosis can take from two to six weeks, depending on the hospital, at a cost of around 3,500 Euros.

So far, a liquid biomarker to facilitate and accelerate the diagnosis at lower cost and invasiveness was not yet available. All patients with a suspected tumor undergo the above-mentioned diagnostic cascade, even if ultimately no malignant tumor can be detected in some patients.

The present novel liquid biomarker from body fluids can be used to determine simply, atraumatically and quickly (within less than 24 hours) whether a patient has a carcinoma or not, even in the early stages of the disease.

Furthermore, the present test can be used during tumor follow-up to assess recurrence and to evaluate a response to therapy.

In addition, this test can also be used to detect whether an infection with a high-risk human papillomavirus (HPV) has occurred. This finding is crucial for the prognosis and is currently only determined by the pathologist. HPV infection is a major factor contributing to the rising incidence of HNC patients. Among all the chronic HPV infections in HNC patients, nearly 85% are caused by HPV16 or HPV18. HPV can cause changes in oncogenes, including amplification, rearrangement, deletion, and translocation, and it induces the expansion and expression of E5/E6/E7 proteins, leading to the initiation and progression of HNC (Liu *et al.* 2024).

The liquid biomarker developed by the present inventors is based on the RNA characterization of sEVs from body fluids. sEVs are tiny vesicles that are produced by every cell in the body - including tumor cells - and excreted into the circulation as in saliva and blood but also urine, cerebrospinal fluid and lymphatic system. In addition to blood and lymphatic system, saliva and urine can also be used to evaluate the RNA profile of sEVs. Saliva and urine sampling are entirely atraumatic.

The inventors have identified biomarkers and biomarker sets represented by mRNA, miRNA and a combined signature of these sEVs, with which a statement can be made regarding the presence of a malignant disease. This test has the potential to reduce patient suffering and save the healthcare system unnecessary costs.

### Summary of the invention

According to a first aspect, the present invention relates to a method for identyfying a head-and-neck cancer, comprising
a) isolating small extracellular vesicles (sEVs) from a biological sample;
b) isolating RNA molecules from the small extracellular vesicles (sEVs) obtained; wherein said RNA molecules comprises a biomarker set of messenger RNAs (mRNA) and/or microRNAs (miRNA);
c) determining the presence and/or amount of mRNAs and/or miRNAs; and
d) relating the presence and/or amount of mRNAs and/or miRNAs to a reference, wherein the head-and-neck cancer is identified on basis of a measurable and significant difference in the presence and/or amount of mRNAs and/or miRNAs as compared to the reference.

According to a second aspect, the present invention relates to a method of detecting a set of biomarkers associated with head-and-neck cancer, comprising
a) isolating small extracellular vesicles (sEVs) from a biological sample;
b) contacting said small extracellular vesicles (sEVs) to one or more than one reagent that specifically binds to one or more than one biomarker; and
c) determining whether or not the biomarker is differentially expressed in the sample by comparing the expression level of the biomarker in the biological sample to the expression level of said biomarker for at least one reference sample, wherein the reference sample is a comparable biological sample obtained from a disease-free subject.

According to a third aspect, the present invention relates to a biomarker set or biomarker sets associated with head-and-neck cancer, detected by the method according to the second aspect above.

With the method for identyfying a head-and-neck cancer, the method of detecting a set of biomarkers associated with head-and-neck cancer, and with developing respective biomarker set(s), the inventors for the first time provided the tools for reliable and reproducible detection of head and neck cancer by liquid biopsy via small extracellular vesicles (sEVs) from the patient's body fluids. The core of the invention comprises the identification of relevant RNA molecules which are capable of distinguishing between HNC and healthy humans. By use of said relevant sEV RNAs, also the disease stage (high and low tumor burden), the efficiency of medical anti-tumor treatment (wherein the patient is determined to be responsive to the treatment or not), and the presence or absence of human papillomavirus (HPV) infection can be differentiated.

In addition, with said RNAs, a respective algorithm (classifier) was programmed and developed in order to process the RNA data collected from tumor patients and healthy humans, and thus to distinguish between HNC or not, high or low tumor burden, and HPV infection or not.

Further advantages include the fast and non-traumatic tumor detection, the avoidance of general anesthesia and of operational risks, and the rapid results (within 24 hours).

These objects are met with the subject matter, methods and means according to the independent claims of the present invention. The dependent claims are related to preferred embodiments.

Additional details, features, characteristics and advantages of the object of the invention are disclosed in the dependent claims, and the following description of the respective figures and examples, which, in an exemplary fashion, show preferred embodiments of the present invention. However, these examples and drawings should by no means be understood as to limit the scope of the invention.

### Description of the Figures

**Fig. 1****.** Metrics (area under the curve (AUC), recall, specificity) of the initial model for (a) miRNA, (b) mRNA, and (c) miRNA/mRNA.
**Fig. 2****.** Top 20 features of (a) miRNA, (b) mRNA, and (c) miRNA/mRNA..
**Fig. 3****.** Metrics (area under the curve (AUC), recall, specificity) of Panel A for (a) miRNA, (b) mRNA, and (c) miRNA/mRNA.

### Detailed Description of the Invention

Before the invention is described in detail, it is to be understood that this invention is not limited to the particular compounds or methods described as such compounds or methods may vary. It is also to be understood that the terminology used herein is for purposes of describing particular embodiments only, and is not intended to be limiting. It must be noted that, as used in the specification and the appended claims, the singular forms "a," "an", and "the" include singular and/or plural referents unless the context clearly dictates otherwise. It is moreover to be understood that, in case parameter ranges are given which are delimited by numeric values, the ranges are deemed to include these limitation values. It is also to be understood that value ranges delimited by numerical values are to be understood to include said delimiting values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure and avoid lengthy repetitions.

According to a first aspect, the present invention relates to a method for identifying a head-and-neck cancer, comprising
a) isolating small extracellular vesicles (sEVs) from a biological sample;
b) isolating RNA molecules from the small extracellular vesicles (sEVs) obtained;
   wherein said RNA molecules comprises a biomarker set of messenger RNAs (mRNA) and/or microRNAs (miRNA);
c) determining the presence and/or amount of mRNAs and/or miRNAs;
d) relating the presence and/or amount of mRNAs and/or miRNAs to a reference, wherein the head-and-neck cancer is identified on basis of a measurable and significant difference in the presence and/or amount of mRNAs and/or miRNAs as compared to the reference.

According to a second aspect, the present invention relates to a method of detecting a set of biomarkers associated with head-and-neck cancer, comprising
a) isolating small extracellular vesicles (sEVs) from a biological sample;
b) contacting said small extracellular vesicles (sEVs) to one or more than one reagent that specifically binds to one or more than one biomarker; and
c) determining whether or not the biomarker is differentially expressed in the sample by comparing the expression level of the biomarker in the biological sample to the expression level of said biomarker for at least one reference sample, wherein the reference sample is a comparable biological sample obtained from a disease-free subject.

A "biomarker" can be defined as a measurable feature or characteristic which is an indicator of normal physiological processes, pathogenic processes, or an indicator of a response to an exposure or therapeutic intervention. Molecular, histologic, radiographic, or physiologic biomarkers represent different types of biomarkers (Aboy *et al.* 2019) by the nature of said measurable feature. FDA and NIH categorizes biomarkers further into prognostic, predictive, responsive, monitoring, safety, diagnostic and susceptibility/risk biomarkers (BEST Resource).

More specifically, a biomarker indicates a change that correlates with the risk or progression of a disease, or with the susceptibility of the disease to a given treatment. Once a proposed biomarker has been validated, it can be used to diagnose disease risk, presence of disease in an individual, or to tailor treatments for the disease in an individual (choices of drug treatment or administration regimes). In evaluating potential drug therapies, a biomarker may be used as a surrogate for a natural endpoint such as survival or irreversible morbidity. If a treatment alters the biomarker, which has a direct connection to improved health, the biomarker serves as a surrogate endpoint for evaluating clinical benefit.

The biomarker further can be of relevance as an indicator and for risk assessment of relapse or recurrence of the disease.

Anti-cancer drugs are sometimes judged for their effect on a "biomarker" or surrogate marker that may predict clinical benefit to the patient, such as regression of tumors. When monitoring a patient's response to an anti-cancer drug, a "biomarker" can be the basis for clear demonstration that the administered compound and a chosen dose and treatment schedule are capable and the levels reached are sufficient for an efficient inhibition of the targeted cancer. In the evaluation of molecularly targeted therapies, traditional clinical endpoints have proven difficult to apply; standard clinical trial endpoints that are used for cytotoxic compounds have been found to be insufficient for the evaluation of molecularly targeted anti-cancer agents. For their development, it is important to have endpoint assessments available for the efficacy of a compound in modulating the activity of its molecular target, and the relationship between target modulation and clinical response. Thus the availability of biomarkers indirectly indicating the effect of treatment on the disease state is one of the key prerequisites for the development of minimally or non-invasive techniques for assessment of an inhibitor's efficacy and, consequently, both for the clinical evaluation of the drug and in the long run for monitoring the efficacy of an approved drug in therapy.

In its broadest sense, a "biomarker" can be defined as a measurable feature which is an indicator of normal physiological processes, pathogenic processes, or an indicator of a response to an exposure or (therapeutic) intervention. Molecular, histologic, radiographic, or physiologic biomarkers represent different types of biomarkers and have been used in medicine: blood pressure or glucose levels are straightforward examples of a physiologic and a molecular biomarker, respectively.

Within the meaning of the present invention, a biomarker is used as an indicator of a biologic state. It is a characteristic that is objectively measured and evaluated as an indicator of normal biologic processes, pathogenic processes, or pharmacologic responses to a therapeutic intervention. This is in line with the definition given by an NIH study group in 1998.

The term "sample" as used herein refers to a bodily fluid sample, such as a blood sample. Preferably, said sample is a blood, blood plasma, or blood serum sample.

However, the terms "providing a sample", "a sample from a patient" or "a sample obtained from a patient" do not include the active step of extraction of the tissue or blood, but rather refer to the provision of an already extracted sample, *i.e.*, the provision of an *ex vivo* sample from a patient. The term "a sample obtained from a patient" as used herein refers to the sample *ex vivo*, *i.e.*, after collection, and is synonymous to "providing a sample from a patient" or "providing an *ex vivo* sample from a patient".

A blood sample may be a serum or plasma sample and is preferably a plasma sample. However, a blood sample may also include the cell fraction, *e.g*., for complete blood count and blood cell exam, such as a peripheral blood smear. Other bodily fluid samples in addition to blood include, without being limited to, mucous, seminal fluid, saliva, sputum, bronchial lavage, breast milk, bile and urine. A sample may further be an aspirate or a cerebrospinal fluid.

According to a third aspect, the present invention relates to a biomarker set associated with head-and-neck cancer, detected by the method as described in the second aspect above.

According to a fourth aspect, the present invention relates to a biomarker set which comprises biomarkers represented by mRNAs and/or miRNAs, preferably wherein the biomarker set consists of biomarkers represented by mRNAs and/or miRNAs.

In preferred embodiment(s) according to the third and/or fourth aspect(s) of the present invention, the biomarker set comprises biomarkers represented by mRNAs and miRNAs, wherein the biomarker set comprises C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, and RPS6, preferably wherein the biomarker set consists of C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, and RPS6.

In preferred embodiment(s) of the present invention, the biomarker set comprises biomarkers represented by mRNAs, wherein the biomarker set comprises MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, and LCK, preferably comprises STAT6, TLR10, S100A8, TNFSF11, CYLD, ITGAL, BATF, ICAM3, STAT2, RPS6, CDKN1A, TMEFF2, HLA-C, LCK, and PSMB9.

Said biomarker set may consist of biomarkers represented by mRNAs, wherein the biomarker set consists of MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, and LCK, preferably consists of STAT6, TLR10, S100A8, TNFSF11, CYLD, ITGAL, BATF, ICAM3, STAT2, RPS6, CDKN1A, TMEFF2, HLA-C, LCK, and PSMB9.

In preferred embodiment(s) of the present invention, the biomarker set comprises biomarkers represented by miRNAs, wherein the biomarker set comprises hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, and hsa-miR-216a-5p, preferably comprises hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, and hsa-miR-216a-5p.

Said biomarker set may consist of biomarkers represented by miRNAs, wherein the biomarker set consists of hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, and hsa-miR-216a-5p, preferably consists of hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, and hsa-miR-216a-5p.

In preferred embodiment(s) of the present invention, the biomarker set comprises biomarkers represented by a combination of mRNAs and miRNAs, wherein the biomarker set comprises hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let-7a-5p, hsa-miR-605-5p, hsa-miR-1246, hsa-miR-96-5p, C6, and hsa-miR-1286, preferably comprises hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, and hsa-miR-1286.

Said biomarker set may consist of biomarkers represented by a combination of mRNAs and miRNAs, wherein the biomarker set consists of hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let7a-5p, hsa-miR-605-5p, hsa-miR-1246, hsa-miR-96-5p, C6, and hsa-miR-1286, preferably consists of hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, and hsa-miR-1286.

According to a fifth aspect, the present invention relates to the use of a biomarker set as described above in the method for identification of a head-and-neck cancer.

In preferred embodiment(s) of the present invention, in said method for identifying a head-and-neck cancer as described above; said method of detecting a set of biomarkers as described above; the biomarker set as described above; and the use of a biomarker set as described above, the head-and-neck cancer is a head-and-neck squamous cell carcinoma (HNSCC).

In preferred embodiment(s) of the present invention, in said methods as described above, the biological sample is a sample selected from the group consisting of saliva, urine, whole blood, white blood cells, blood plasma, blood serum, cerebrospinal fluid, and lymphatic fluid.

In preferred embodiment(s) of the present invention, in said methods as described above, said small extracellular vesicles (sEVs) are shed from head-and-neck cancer cells.

In preferred embodiment(s) of the present invention, in said methods as described above, said small extracellular vesicles (sEVs) are isolated by use of size-exclusion chromatography, affinity selection using a binding reagent which specifically binds to an sEV surface antigen, ultrafiltration, (ultra)centrifugation and/or precipitation.

In preferred embodiment(s) of the present invention, in said methods as described above, said biomarkers are detected and/or identifed by use of nano-string analysis or polymerase chain reaction (PCR) analysis.

In preferred embodiment(s) of the present invention, in said method of detecting a set of biomarkers, in step c) an SVM (support vector machine)-based classifier and/or a logistic regression model is developed, validated and/or used for training.

In preferred embodiment(s) of the present invention, in said methods as described above, the methods are automated.

In preferred embodiment(s) of the present invention, in said method for identifying a head-and-neck cancer, and/or in said method of detecting a set of biomarkers, one or more than one biomarker or biomarker set identifies a particular disease level (stage) of the head-and-neck cancer.

In preferred embodiment(s) of the present invention, in said method for identifying a head-and-neck cancer, one or more than one biomarker or biomarker set identifies the presence of a human papillomavirus (HPV) associated with the head-and-neck cancer, preferably wherein the HPV-specific biomarker is an HPV-specific ribonucleic acid.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease.

According to a sixth aspect, the present invention relates to a biomarker set comprising biomarkers as above, wherein the sequence identity of each biomarker represented by an individual mRNA or miRNA in the biomarker set is at least 80%, or at least 85%, or at least 90%, or at least 95%, or at least 96%, or at least 97%, or at least 98%, or at least 99% as compared to the respective nucleotide sequence listed in the present Sequence Listing.

According to a seventh aspect, the present invention relates to a biomarker set, suited for the detection of cancer, preferably of a head-and-neck cancer, comprising five or more biomarkers, selected from the group of biomarkers consisting of the miRNAs hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p, hsa-miR-223-3p, hsa-miR-183-5p, hsa-miR-16-5p, hsa-miR-23a-3p, hsa-miR-325, hsa-miR-1305, hsa-let-7a-5p, hsa-miR-605-5p, hsa-miR-96-5p, hsa-miR-1286, hsa-miR-1204, hsa-miR-33a-5p, hsa-miR-590-5p, hsa-miR-574-5p and/or mRNAs MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK, C6, CLEC4A, IL13RA1, IL17RA, GZMH, PDGFRB.

The present invention further relates to the use of a biomarker set for the detection of cancer, preferably of a head-and-neck cancer, wherein the biomarker set comprises five or more biomarkers, selected from the group of biomarkers consisting of the miRNAs hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p, hsa-miR-223-3p, hsa-miR-183-5p, hsa-miR-16-5p, hsa-miR-23a-3p, hsa-miR-325, hsa-miR-1305, hsa-let-7a-5p, hsa-miR-605-5p, hsa-miR-96-5p, hsa-miR-1286, hsa-miR-1204, hsa-miR-33a-5p, hsa-miR-590-5p, hsa-miR-574-5p, and/or the mRNAs MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK, C6, CLEC4A, IL13RA1, IL17RA, GZMH, PDGFRB.

### Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus; all nucleic acid sequences disclosed herein are shown from 5' end to 3' end.

### Example 1: Panel Description

The compounds according to the present invention and their intermediates may be obtained using methods of synthesis which are known to the one skilled in the art and described in the literature of organic synthesis. Preferably, the compounds are obtained in analogous fashion to the methods of preparation explained more fully hereinafter, in which the substituents of the general formulae have the meanings given hereinbefore. These methods are intended as an illustration of the invention without restricting its subject matter and the scope of the compounds claimed to these examples. In some cases, the order in carrying out the reaction steps may be varied. Variants of the reaction methods that are known to the one skilled in the art but not described in detail here may also be used.

Where the preparation of starting compounds is not described, they are commercially obtainable or their synthesis is described in the prior art or they may be prepared analogously to known prior art compounds or methods described herein, *i.e.* it is within the skills of an organic chemist to synthesize these compounds. Substances described in the literature can be prepared according to the published methods of synthesis. Any functional groups in the starting materials or intermediates may be protected using conventional protecting groups. These protecting groups may be cleaved again at a suitable stage within the reaction sequence using methods familiar to the one skilled in the art.

The following RNA panels can be used for first cancer diagnosis, diagnosis of recurrence, therapy response and identification of HPV positivity.

### Panel A/B (20 features):

C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, RPS6

For each of the biomarker sets of mRNA, miRNA and mRNA/miRNA combined, a Panel A as "Main" Panel and a Panel B as "Core" Panel has been provided.

### mRNA Panel A (20 features):

MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK

### mRNA Panel B (15 features):

STAT6, TLR10, S100A8, TNFSF11, CYLD, ITGAL, BATF, ICAM3, STAT2, RPS6, CDKN1A, TMEFF2, HLA-C, LCK, PSMB9

### miRNA Panel A (20 features):

hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p

### miRNA Panel B (12 features):

hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, hsa-miR-216a-5p

### mRNA/miRNA combined Panel A (20 features):

hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let-7a-5p, hsa-miR-605-5p, hsa-miR-1246, hsa-miR-96-5p, C6, hsa-miR-1286

### mRNA/miRNA combined Panel B (10 features):

hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, hsa-miR-1286.

### Example 2: Methods

Plasma was obtained from citrate blood and saliva was collected using Salivette^{®} tubes. Small extracellular vesicles (sEVs) from biofluids were isolated by a combination of differential centrifugation, ultrafiltration, size exclusion chromatography, ultracentrifugation, immunoaffinity capture, and precipitation.

Total RNA, including miRNA, was isolated from sEVs using Qiazol-based kits. In the further course, the isolation of sEVs and RNA were to be combined, *e.g*., using column-based assays. RNA quality and quantity were assessed by automated electrophoresis and fluorometric spectrophotometry.

Downstream assays for RNA analysis comprised nanoString microarrays (nCounter^{®} PanCancer Immune Profiling Panel, nCounter^{®} Human v3 miRNA Assay, nCounter^{®} mRNA Custom CodeSet, nCounter^{®} miRNA Custom CodeSet), single-target and multiplexed SYBR Green-based RT-PCR, and (small) RNA sequencing. While the PanCancer Panel and miRNA Assay were ready-made microarrays, the Custom CodeSets cover the analysis of the applied target panels.

For sEV isolation, plasma from citrate blood was centrifuged at 2,000 g for 10 min and 12,000 g for 30 min at 4°C to remove debris and larger vesicles, followed by filtration through a 0.22 µm syringe-driven filters. The sample was diluted 1:1 with PBS to reduce viscosity. Pre-cleared and diluted plasma was ultracentrifuged at 110,000 g for 2h at 4°C. The resulting sEV pellet was resuspended in 1 mL PBS and again ultracentrifuged at 110,000 g for 70 min at 4°C. The washing step was repeated and resulting sEV pellet dissolved in Qiazol, thoroughly vortexed and stored at -80°C.

Total sEV RNA was isolated using the miRNeasy Micro Kit. Quality of sEV RNA was controlled by automated gel electrophoresis with a high-sensitivity RNA screen tape. The concentration of sEV RNA was determined by fluorometric spectrophotometry. sEV RNA was stored at -80°C.

SEV RNA profiling was performed using the nCounter^{®} SPRINT system. 500 pg sEV RNA each was applied to the Human v3 miRNA Assay (comprising the measurement of 827 human miRNAs), and to the PanCancer Immune Profiling Panel (comprising the measurement of 770 mRNAs). MiRNA data was normalized using the positive ligation controls included in the array, and mRNA data was normalized using stably expressed housekeeping genes included in the array. Normalized and background-corrected data were the input for statistical analysis as described in example 3.

### Example 3: Statistical Analysis for Panel Identification

The objective of this analysis was to develop and evaluate a panel and subpanel of features that could detect the presence of head and neck squamous cell carcinoma (HNSCC) based on sEV-RNA liquid biopsy. A pipeline was implemented to identify the most significant features contributing to the prediction model and to assess their diagnostic performance.

A logistic regression (LR) model was initially trained using 10-fold cross-validation with 1000 random states (Repeated Stratified CV). This approach was chosen to provide robust estimates of the model's performance metrics, particularly given the small dataset of 127 samples. The combination of 10-fold cross-validation and 1000 random states allows for a thorough evaluation of the model's performance, maximizing the use of available data for training while providing a robust estimate of how the model will likely perform on new, unseen data. The latter ensures that the model's performance metrics, such as AUC, recall, and sensitivity, are not just artifacts of a single random split of the data but are consistent across multiple iterations and data configurations. This leads to greater confidence in the reliability and generalizability of the model.

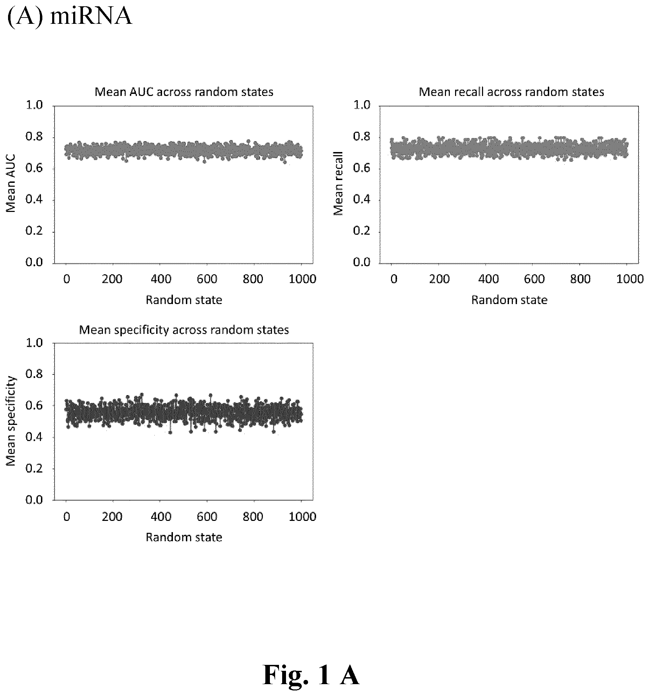

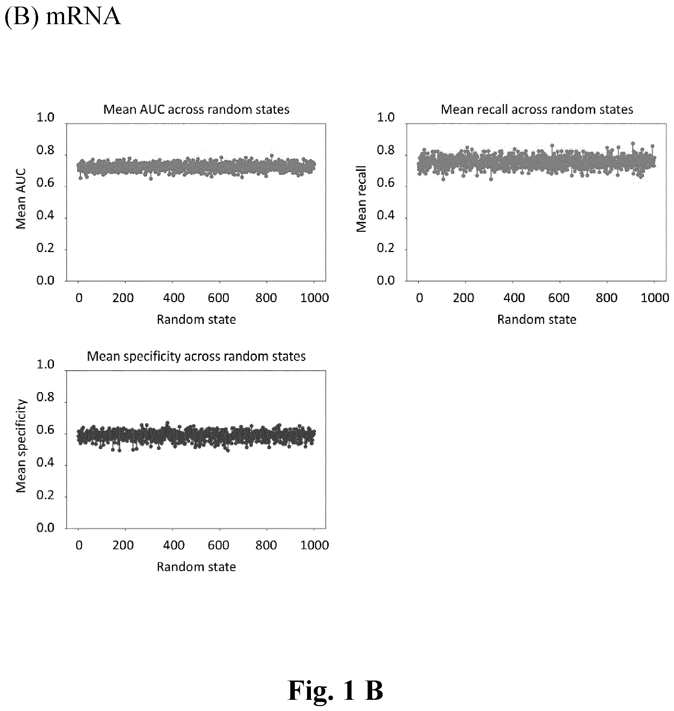

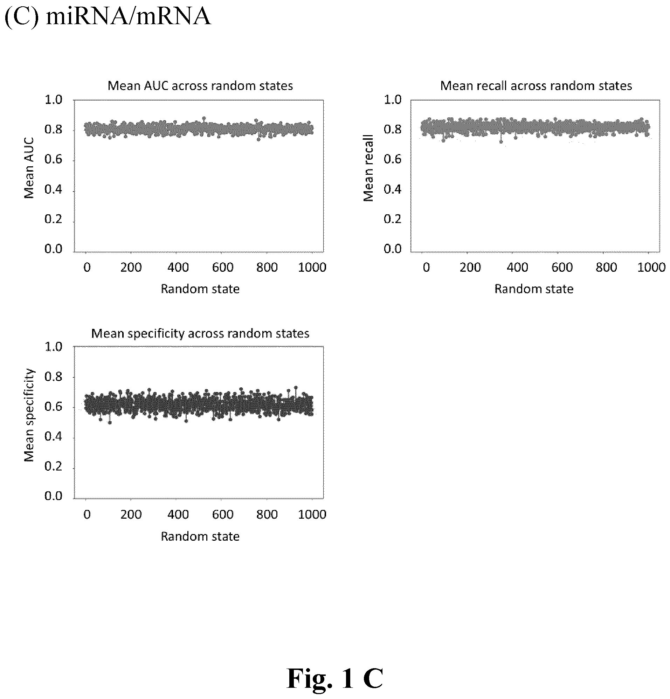

The model's effectiveness was evaluated by calculating and plotting the mean Area Under the Curve (AUC), mean recall (sensitivity), and mean specificity. **Figure 1** illustrates these metrics for the *initial model*, which was trained using all 798 features (miRNA), 730 features (mRNA), and 1528 features (miRNA/mRNA). The model yielded the following metrics in **Table 1:**

| Panel | Mean AUC | Mean recall | Mean specificity |
|---|---|---|---|
| miRNA | 0.72 | 0.73 | 0.56 |
| mRNA | 0.72 | 0.76 | 0.59 |
| miRNA/mRNA | 0.81 | 0.82 | 0.62 |

For miRNA-only and mRNA-only data, after scaling the data to ensure comparability across features and prevent the model from mistakenly identifying features with larger numerical ranges as more important, which could introduce noise into the feature selection process, the features were ranked based on their importance as determined by the trained model. For combined miRNA/mRNA data, no scaling was applied to avoid bias against one of the omics layers yet using raw values, the features were ranked based on their importance as determined by the trained model.

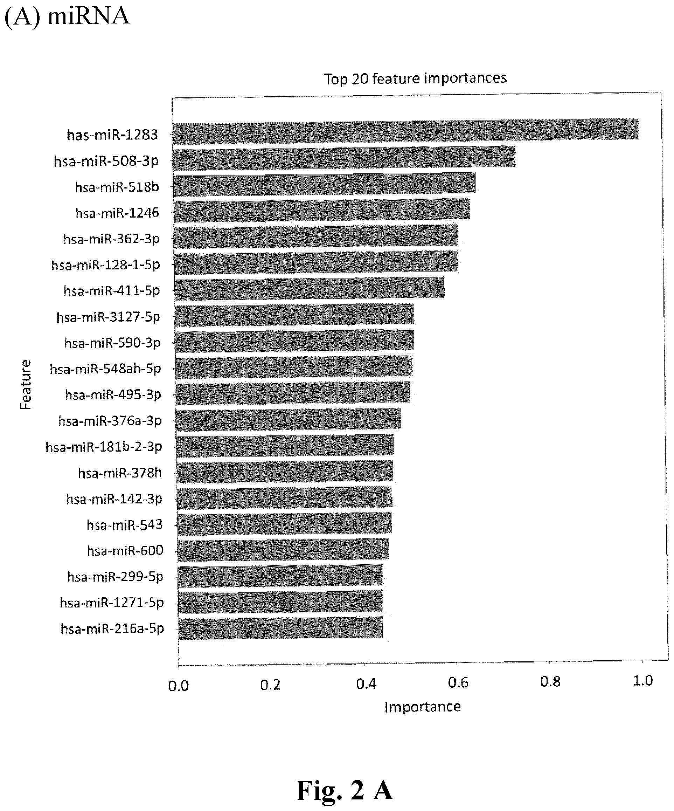

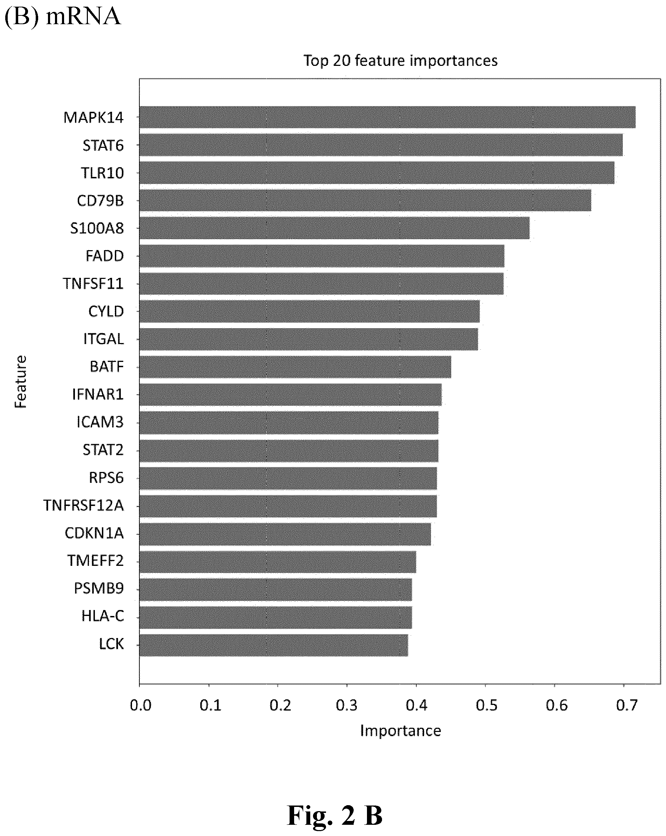

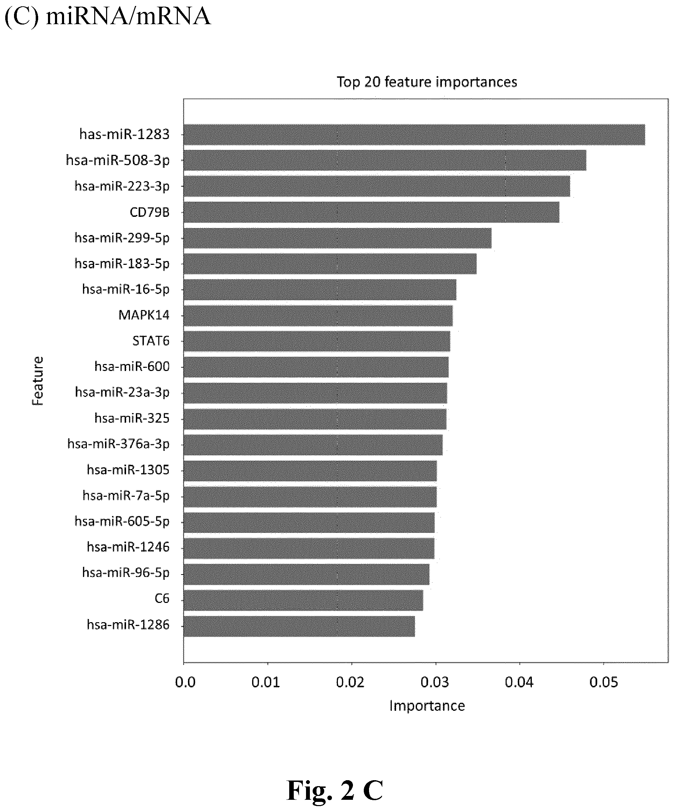

The top 20 features were selected for further analysis as shown in **Figure 2****,** as they were deemed to be the most influential in predicting the outcome (had the highest importance coefficient). The top 20 features hence are called the **main panel** or **Panel A:**

### Panel A (miRNA):

hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p.

### Panel A (mRNA):

MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK.

### Panel A (miRNA/mRNA combined):

hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let7a-5p, hsa-miR-605-5p, hsa-miR-1246, hsa-miR-96-5p, C6, hsa-miR-1286.

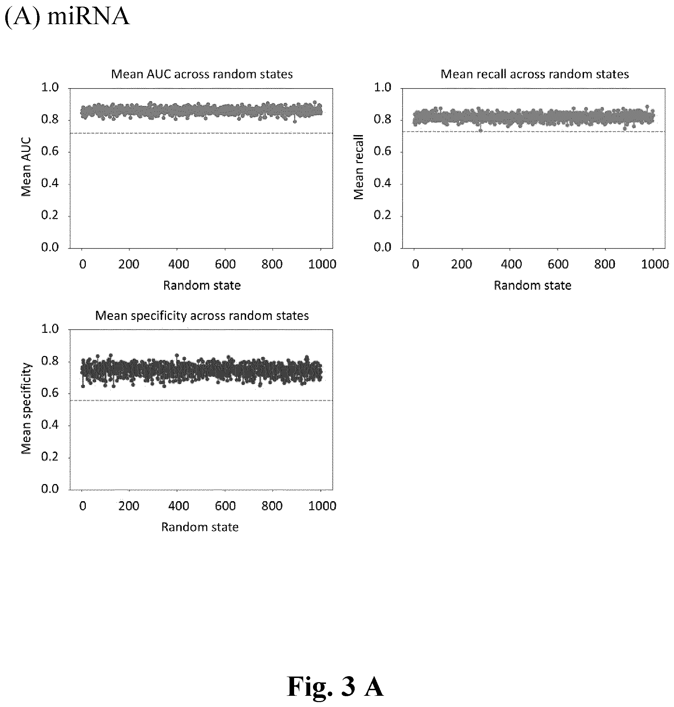

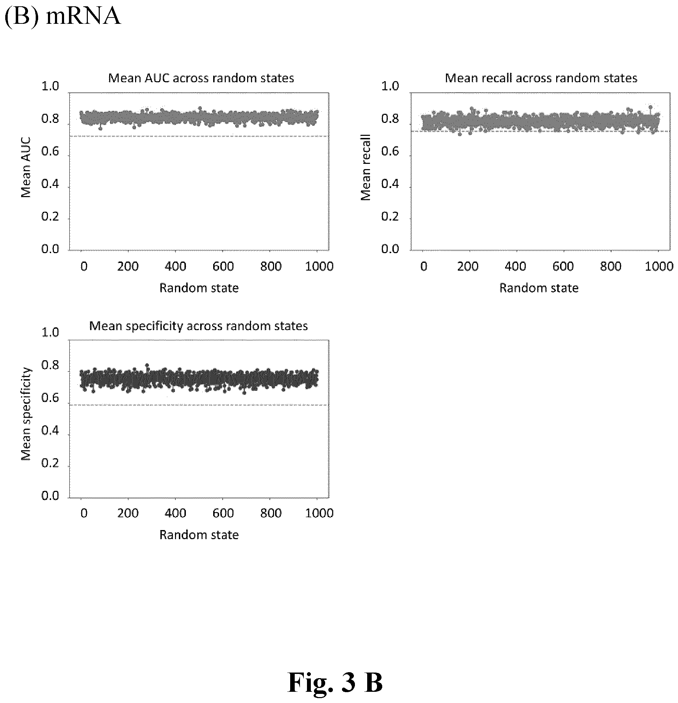

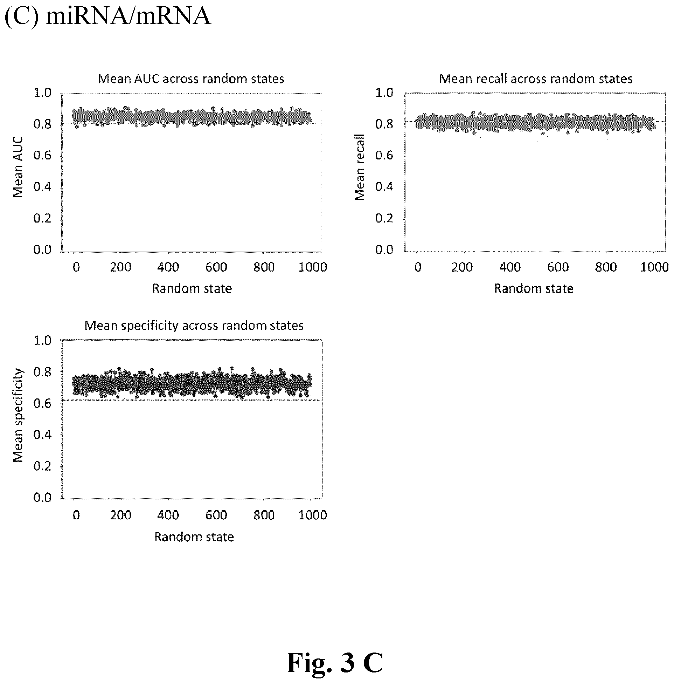

After identifying and outlining the top 20 features that were used to form **Panel A,** the model was reevaluated using the same method applied to the full set of features. If the selected features possess strong diagnostic power, the model's performance metrics are expected to improve. Indeed, this was observed, as shown in **Figure 3** (dotted gray line displays means of specs of the initial model). **Panel A evaluation** yielded the following metrics in **Table 2:**

| Panel | Mean AUC | Mean recall | Mean specificity |
|---|---|---|---|
| miRNA | 0.87 | 0.82 | 0.74 |
| mRNA | 0.84 | 0.82 | 0.75 |
| miRNA/mRNA | 0.85 | 0.82 | 0.72 |

After establishing the main panel, odds ratios (OR) were calculated to identify a **core panel,** referred to as **Panel B.** Initially, the odds ratios of the features were calculated under the assumption that each feature was independent of the others. Subsequently, to account for multicollinearity and redundancy, odds ratios were recalculated using the variance-covariance matrix. This adjustment revealed that not all features from Panel A displayed statistically significant p-values. However, it is important to note that features with p-values between 0.05 and 0.1 might also demonstrate statistical significance with an increased sample size. Therefore, it is recommended that the **core panel (Panel B)** includes these additional features, resulting in the following panels:

### Panel B (miRNA) (12 features):

hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, hsa-miR-216a-5p.

### Panel B (mRNA) (15 features):

STAT6, TLR10, S100A8, TNFSF11, CYLD, ITGAL, BATF, ICAM3, STAT2, RPS6, CDKN1A, TMEFF2, HLA-C, LCK, PSMB9.

### Panel B (miRNA/mRNA combined) (10 features):

hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, hsa-miR-1286.

Finally, **both Panels A and B** were further divided into two **Sub-Panels:** a **positive Panel,** consisting of features with positive coefficients, and a **negative Panel,** consisting of features with negative coefficients. This division was made to separately assess the contribution of features that are positively associated with HNSCC and those that are negatively associated. A positive coefficient indicates that as the expression level of the feature increases, the likelihood of the outcome (*i.e*., the presence of HNSCC) also increases. A negative coefficient suggests that as the expression level of the feature increases, the likelihood of the outcome decreases. For each sub-panel, odds ratios were calculated along with their corresponding confidence intervals (CI) and p-values. This analysis provides a clearer picture of how these features contribute to the diagnostic power of the panels outlined.

### Panel A (miRNA) (mean AUC: 0.87, mean recall: 0.82, mean specificity: 0.74)

Positive Panel - Combined OR: 14.80, CI: (0.84, 260.77), p-value: 0.0656
Negative Panel - Combined OR: 0.51, CI: (0.38, 0.69), p-value: 1.5019e-05

### Panel B (miRNA) (mean AUC: 0.89, mean recall: 0.83, mean specificity: 0.72)

Positive Panel - Combined OR: 1.59, CI: (1.25, 2.02), p-value: 0.0001
Negative Panel - Combined OR: 0.58, CI: (0.45, 0.74), p-value: 1.8449e-05

### Panel A (mRNA) (mean AUC: 0.84, mean recall: 0.82, mean specificity: 0.75)

Positive Panel - Combined OR: 29.34, CI: (0.89, 962.21), p-value: 0.0578
Negative Panel - Combined OR: 0.39, CI: (0.26, 0.60), p-value: 1.1380e-05

### Panel B (mRNA) (mean AUC: 0.87, mean recall: 0.84, mean specificity: 0.70)

Positive Panel - Combined OR: 25.29, CI: (1.13, 567.69), p-value: 0.0418
Negative Panel - Combined OR: 0.46, CI: (0.33, 0.65), p-value: 1.1545e-05

### Panel A (miRNA/mRNA) (mean AUC: 0.85, mean recall: 0.82, mean specificity: 0.72)

Positive Panel - Combined OR: 1.51, CI: (1.23, 1.85), p-value: 7.0806e-05
Negative Panel - Combined OR: 0.0451, CI: (0.0026, 0.77), p-value: 0.0326

### Panel B (miRNA/mRNA) (mean AUC: 0.89, mean recall: 0.83, mean specificity: 0.70)

Positive Panel - Combined OR: 1.38, CI: (1.20, 1.59), p-value: 5.6906e-06
Negative Panel - Combined OR: 0.0754, CI: (0.0093, 0.61), p-value: 0.0157.

### Example 4: Usage of the Panels

The panels can be applied on sEV-RNA from biofluids of patients using single-target or multiplexed RT-PCR assays, and odds ratios (OR) can be calculated. Results will be interpreted as follows: The higher the expression of positive features, the higher the chance of cancer with OR >1. The higher the expression of negative features, the lower the chance of cancer with OR <1. The combination of features will determine if a test person is at a higher risk of cancer. Further, upon validation with RT-PCR, RT-PCR data can be binarized, and a threshold can be built, upon which a feature is positively associated with cancer, to further facilitate data interpretability.

sEV isolation and sEV RNA isolation are not common methods performed in clinical laboratories" daily routine. The effort of establishing these methods, including characterization and verification of the presence of sEVs, is very high. Therefore, applicants plan to develop a ready-to-use lab kit ("lab on a chip") for potential easy usage of this method in every clinical laboratory, implementing sEV-RNA isolation and target RNA identification. Additionally, a self-testing saliva kit will be developed for simple laboratory-independent application of the liquid biomarker test without the involvement of trained staff.

### References

Aboy M et al. (2019). How does emerging patent case law in the US and Europe affect precision medicine? Nature Biotechnology Vol. 37: 1118-1126.
BEST Resource (Biomarkers, Endpoints, and other Tools) (2018). FDA-NIH Biomarker Working Group. Food and Drug Administration (US); co-published by National Institutes of Health (US), Bethesda (MD).
Liu Y et al. (2024). Head and neck cancer: pathogenesis and targeted therapy. MedComm. 2024; 5:e702.
Vazquez-Levin M.H. et al. (2023). Artificial intelligence: A step forward in biomarker discovery and integration towards improved cancer diagnosis and treatment. Lausanne, Frontiers Media SA. doi: 10.3389/978-2-83252-180-9.

### SEQUENCES

The following sequences form part of the disclosure of the present application. A WIPO ST 26 compatible electronic sequence listing is provided with this application, too. For the avoidance of doubt, if discrepancies exist between the sequences in the following table and the electronic sequence listing, the sequences in this table shall be deemed to be the correct ones.

| **SEQ ID No.** | **Name** | **(Accession)** | **Sequence** |
|---|---|---|---|
| | **miRNA** | | |
| 1 | hsa-miR-1283 | (MIMAT0005799) | UCUACAAAGGAAAGCGCUUUCU |
| 2 | hsa-miR-508-3p | (MIMAT0002880) | UGAUUGUAGCCUUUUGGAGUAGA |
| 3 | hsa-miR-518b | (MIMAT0002844) | CAAAGCGCUCCCCUUUAGAGGU |
| 4 | hsa-miR-1246 | (MIMAT0005898) | AAUGGAUUUUUGGAGCAGG |
| 5 | hsa-miR-362-3p | (MIMAT0004683) | AACACACCUAUUCAAGGAUUCA |
| 6 | hsa-miR-128-1-5p | (MIMAT0026477) | CGGGGCCGUAGCACUGUCUGAGA |
| 7 | hsa-miR-411-5p | (MIMAT0003329) | UAGUAGACCGUAUAGCGUACG |
| 8 | hsa-miR-3127-5p | (MIMAT0014990) | AUCAGGGCUUGUGGAAUGGGAAG |
| 9 | hsa-miR-590-3p | (MIMAT0004801) | UAAUUUUAUGUAUAAGCUAGU |
| 10 | hsa-miR-548ah-5p | (MIMAT0018972) | AAAAGUGAUUGCAGUGUUUG |
| 11 | hsa-miR-495-3p | (MIMAT0002817) | AAACAAACAUGGUGCACUUCUU |
| 12 | hsa-miR-376a-3p | (MIMAT0000729) | AUCAUAGAGGAAAAUCCACGU |
| 13 | hsa-miR-181b-2-3p | (MIMAT0031893) | CUCACUGAUCAAUGAAUGCA |
| 14 | hsa-miR-378h | (MIMAT0018984) | ACUGGACUUGGUGUCAGAUGG |
| 15 | hsa-miR-142-3p | (MIMAT0000434) | UGUAGUGUUUCCUACUUUAUGGA |
| 16 | hsa-miR-543 | (MIMAT0004954) | AAACAUUCGCGGUGCACUUCUU |
| 17 | hsa-miR-600 | (MIMAT0003268) | ACUUACAGACAAGAGCCUUGCUC |
| 18 | hsa-miR-299-5p | (MIMAT0002890) | UGGUUUACCGUCCCACAUACAU |
| 19 | hsa-miR-1271-5p | (MIMAT0005796) | CUUGGCACCUAGCAAGCACUCA |
| 20 | hsa-miR-216a-5p | (MIMAT0000273) | UAAUCUCAGCUGGCAACUGUGA |
| 21 | hsa-miR-223-3p | (MIMAT0000280) | UGUCAGUUUGUCAAAUACCCCA |
| 22 | hsa-miR-183-5p | (MIMAT0000261) | UAUGGCACUGGUAGAAUUCACU |
| 23 | hsa-miR-16-5p | (MIMAT0000069) | UAGCAGCACGUAAAUAUUGGCG |
| 24 | hsa-miR-23a-3p | (MIMAT0000078) | AUCACAUUGCCAGGGAUUUCC |
| 25 | hsa-miR-325 | (MIMAT0000771) | CCUAGUAGGUGUCCAGUAAGUGU |
| 26 | hsa-miR-1305 | (MIMAT0005893) | UUUUCAACUCUAAUGGGAGAGA |
| 27 | hsa-let-7a-5p | (MIMAT0000062) | UGAGGUAGUAGGUUGUAUAGUU |
| 28 | hsa-miR-605-5p | (MIMAT0003273) | UAAAUCCCAUGGUGCCUUCUCCU |
| 29 | hsa-miR-96-5p | (MIMAT0000095) | UUUGGCACUAGCACAUUUUUGCU |
| 30 | hsa-miR-1286 | (MIMAT0005877) | UGCAGGACCAAGAUGAGCCCU |
| 31 | hsa-miR-1204 | (MIMAT0005868) | UCGUGGCCUGGUCUCCAUUAU |
| 32 | hsa-miR-33a-5p | (MIMAT0000091) | GUGCAUUGUAGUUGCAUUGCA |
| 33 | hsa-miR-590-5p | (MIMAT0003258) | GAGCUUAUUCAUAAAAGUGCAG |
| 34 | hsa-miR-574-5p | (MIMAT0004795) | UGAGUGUGUGUGUGUGAGUGUGU |

| **SEQ ID No.** | **Name (Accession) Sequence** |
|---|---|
| | **mRNA** |
| | **MAPK14 (NM_001315.3)** |
| 35 | ACTGGAGCCTTAGCGGGCGCAGCAGCTGGAACGGGAGTACTGCGACGCAGCCCGGAGTCGGCCTTGTAG GGGCGAAGGTGCAGGGAGATCGCGGCGGGCGCAGTCTTGAGCGCCGGAGCGCGTCCCTGCCCTTAGCG GGGCTTGCCCCAGTCGCAGGGGCACATCCAGCCGCTGCGGCTGACAGCAGCCGCGCGCGCGGGAGTCT GCGGGGTCGCGGCAGCCGCACCTGCGCGGGCGACCAGCGCAAGGTCCCCGCCCGGCTGGGCGGGCA GCAAGGGCCGGGGAGAGGGTGCGGGTGCAGGCGGGGGCCCCACAGGGCCACCTTCTTGCCCGGCGGC TGCCGCTGGAAAATGTCTCAGGAGAGGCCCACGTTCTACCGGCAGGAGCTGAACAAGACAATCTGGGAGGT GCCCGAGCGTTACCAGAACCTGTCTCCAGTGGGCTCTGGCGCCTATGGCTCTGTGTGTGCTGCTTTTGACAC AAAAACGGGGTTACGTGTGGCAGTGAAGAAGCTCTCCAGACCATTTCAGTCCATCATTCATGCGAAAAGAACC TACAGAGAACTGCGGTTACTTAAACATATGAAACATGAAAATGTGATTGGTCTGTTGGACGTTTTTACACCTGCA AGGTCTCTGGAGGAATTCAATGATGTGTATCTGGTGACCCATCTCATGGGGGCAGATCTGAACAACATTGT GAAATGTCAGAAGCTTACAGATGACCATGTTCAGTTCCTTATCTACCAAATTCTCCGAGGTCTAAAGTAT ATACATTCAGCTGACATAATTCACAGGGACCTAAAACCTAGTAATCTAGCTGTGAATGAAGACTGTGAGC TGAAGATTCTGGATTTTGGACTGGCTCGGCACACAGATGATGAAATGACAGGCTACGTGGCCACTAGGTG GTACAGGGCTCCTGAGATCATGCTGAACTGGATGCATTACAACCAGACAGTTGATATTTGGTCAGTGGGA TGCATAATGGCCGAGCTGTTGACTGGAAGAACATTGTTTCCTGGTACAGACCATATTAACCAGCTTCAGC AGATTATGCGTCTGACAGGAACACCCCCCGCTTATCTCATTAACAGGATGCCAAGCCATGAGGCAAGAAA CTATATTCAGTCTTTGACTCAGATGCCGAAGATGAACTTTGCGAATGTATTTATTGGTGCCAATCCCCTG GCTGTCGACTTGCTGGAGAAGATGCTTGTATTGGACTCAGATAAGAGAATTACAGCGGCCCAAGCCCTTG CACATGCCTACTTTGCTCAGTACCACGATCCTGATGATGAACCAGTGGCCGATCCTTATGATCAGTCCTT TGAAAGCAGGGACCTCCTTATAGATGAGTGGAAAAGCCTGACCTATGATGAAGTCATCAGCTTTGTGCCA CCACCCCTTGACCAAGAAGAGATGGAGTCCTGAGCACCTGGTTTCTGTTCTGTTGATCCCACTTCACTGT GAGGGGAAGGCCTTTTCACGGGAACTCTCCAAATATTATTCAAGTGCCTCTTGTTGCAGAGATTTCCTCC ATGGTGGAAGGGGGTGTGCGTGCGTGTGCGTGCGTGTTAGTGTGTGTGCATGTGTGTGTCTGTCTTTGTG GGAGGGTAAGACAATATGAACAAACTATGATCACAGTGACTTTACAGGAGGTTGTGGATGCTCCAGGGCA GCCTCCACCTTGCTCTTCTTTCTGAGAGTTGGCTCAGGCAGACAAGAGCTGCTGTCCTTTTAGGAATATG TTCAATGCAAAGTAAAAAAATATGAATTGTCCCCAATCCCGGTCATGCTTTTGCCACTTTGGCTTCTCCT GTGACCCCACCTTGACGGTGGGGCGTAGACTTGACAACATCCCACAGTGGCACGGAGAGAAGGCCCATAC CTTCTGGTTGCTTCAGACCTGACACCGTCCCTCAGTGATACGTACAGCCAAAAAGGACCAACTGGCTTCT GTGCACTAGCCTGTGATTAACTTGCTTAGTATGGTTCTCAGATCTTGACAGTATATTTGAAACTGTAAAT ATGTTTGTGCCTTAAAAGGAGAGAAGAAAGTGTAGATAGTTAAAAGACTGCAGCTGCTGAAGTTCTGAGC CGGGCAAGTCGAGAGGGCTGTTGGACAGCTGCTTGTGGGCCCGGAGTAATCAGGCAGCCTTCATAGGCGG TCATGTGTGCATGTGAGCACATGCGTATATGTGCGTCTCTCTTTCTCCCTCACCCCCAGGTGTTGCCATT TCTCTGCTTACCCTTCACCTTTGGTGCAGAGGTTTCTTGAATATCTGCCCCAGTAGTCAGAAGCAGGTTC TTGATGTCATGTACTTCCTGTGTACTCTTTATTTCTAGCAGAGTGAGGATGTGTTTTGCACGTCTTGCTA TTTGAGCATGCACAGCTGCTTGTCCTGCTCTCTTCAGGAGGCCCTGGTGTCAGGCAGGTTTGCCAGTGAA GACTTCTTGGGTAGTTTAGATCCCATGTCACCTCAGCTGATATTATGGCAAGTGATATCACCTCTCTTCA GCCCCTAGTGCTATTCTGTGTTGAACACAATTGATACTTCAGGTGCTTTTGATGTGAAAATCATGAAAAG AGGAACAGGTGGATGTATAGCATTTTTATTCATGCCATCTGTTTTCAACCAACTATTTTTGAGGAATTAT CATGGGAAAAGACCAGGGCTTTTCCCAGGAATATCCCAAACTTCGGAAACAAGTTATTCTCTTCACTCCC AATAACTAATGCTAAGAAATGCTGAAAATCAAAGTAAAAAATTAAAGCCCATAAGGCCAGAAACTCCTTT TGCTGTCTTTCTCTAAATATGATTACTTTAAAATAAAAAAGTAACAAGGTGTCTTTTCCACTCCTATGGA AAAGGGTCTTCTTGGCAGCTTAACATTGACTTCTTGGTTTGGGGAGAAATAAATTTTGTTTCAGAATTTT GTATATTGTAGGAATCCTTTGAGAATGTGATTCCTTTTGATGGGGAGAAAGGGCAAATTATTTTAATATT TTGTATTTTCAACTTTATAAAGATAAAATATCCTCAGGGGTGGAGAAGTGTCGTTTTCATAACTTGCTGA ATTTCAGGCATTTTGTTCTACATGAGGACTCATATATTTAAGCCTTTTGTGTAATAAGAAAGTATAAAGT CACTTCCAGTGTTGGCTGTGTGACAGAATCTTGTATTTGGGCCAAGGTGTTTCCATTTCTCAATCAGTGC AGTGATACATGTACTCCAGAGGGACAGGGTGGACCCCCTGAGTCAACTGGAGCAAGAAGGAAGGAGGCAG ACTGATGGCGATTCCCTCTCACCCGGGACTCTCCCCCTTTCAAGGAAAGTGAACCTTTAAAGTAAAGGCC TCATCTCCTTTATTGCAGTTCAAATCCTCACCATCCACAGCAAGATGAATTTTATCAGCCATGTTTGGTT GTAAATGCTCGTGTGATTTCCTACAGAAATACTGCTCTGAATATTTTGTAATAAAGGTCTTTGCACATGT |
| | GACCACATACGTGTTAGGAGGCTGCATGCTCTGGAAGCCTGGACTCTAAGCTGGAGCTCTTGGAAGAGCT CTTCGGTTTCTGAGCATAATGCTCCCATCTCCTGATTTCTCTGAACAGAAAACAAAAGAGAGAATGAGGG AAATTGCTATTTTATTTGTATTCATGAACTTGGCTGTAATCAGTTATGCCGTATAGGATGTCAGACAATA CCACTGGTTAAAATAAAGCCTATTTTTCAAATTTAGTGAGTTTCTCAAGTTTATTATATTTTTCTCTTGT TTTTATTTAATGCACAATATGGCATTATATCAATATCCTTTAAACTGTGACCTGGCATACTTGTCTGACA GATCTTAATACTACTCCTAACATTTAGAAAATGTTGATAAAGCTTCTTAGTTGTACATTTTTTGGTGAAG AGTATCCAGGTCTTTGCTGTGGATGGGTAAAGCAAAGAGCAAATGAACGAAGTATTAAGCATTGGGGCCT GTCTTATCTACACTCGAGTGTAAGAGTGGCCGAAATGACAGGGCTCAGCAGACTGTGGCCTGAGGGCCAA ATCTGGCCCACCACCTGTTTGGTGTAGCCTGCTAAGAATGGCTTTTACATTTTTAAATGGTTGGGAAAGA AAAAAAAAGAAGTAGTAGATTTTGTAGCATGTGATGTAAGTAATGTAAAACTTAAATTCCAGTATCCATA AATAAAGTTTTATGAGAACAGA |

| | **STAT6 (NM_003153.5)** |
|---|---|
| 36 | GGGGCAGCCACTGCTTACACTGAAGAGGGAGGACGGGAGAGGAGTGTGTGTGTGTGTGTGTGTGTGTGTG TGTATGTATGTGTGTGCTTTATCTTATTTTTCTTTTTGGTGGTGGTGGTGGAAGGGGGGAGGTGCTAGCA GGGCCAGCCTTGAACTCGCTGGACAGAGCTACAGACCTATGGGGCCTGGAAGTGCCCGCTGAGAAAGGGA GAAGACAGCAGAGGGGTTGCCGAGGCAACCTCCAAGTCCCAGATCATGTCTCTGTGGGGTCTGGTCTCCA AGATGCCCCCAGAAAAAGTGCAGCGGCTCTATGTCGACTTTCCCCAACACCTGCGGCATCTTCTGGGTGA CTGGCTGGAGAGCCAGCCCTGGGAGTTCCTGGTCGGCTCCGACGCCTTCTGCTGCAACTTGGCTAGTGCC CTACTTTCAGACACTGTCCAGCACCTTCAGGCCTCGGTGGGAGAGCAGGGGGAGGGGAGCACCATCTTGC AACACATCAGCACCCTTGAGAGCATATATCAGAGGGACCCCCTGAAGCTGGTGGCCACTTTCAGACAAAT ACTTCAAGGAGAGAAAAAAGCTGTTATGGAACAGTTCCGCCACTTGCCAATGCCTTTCCACTGGAAGCAG GAAGAACTCAAGTTTAAGACAGGCTTGCGGAGGCTGCAGCACCGAGTAGGGGAGATCCACCTTCTCCGAG AAGCCCTGCAGAAGGGGGCTGAGGCTGGCCAAGTGTCTCTGCACAGCTTGATAGAAACTCCTGCTAATGG GACTGGGCCAAGTGAGGCCCTGGCCATGCTACTGCAGGAGACCACTGGAGAGCTAGAGGCAGCCAAAGC CCTAGTGCTGAAGAGGATCCAGATTTGGAAACGGCAGCAGCAGCTGGCAGGGAATGGCGCACCGTTTGAGG AGAGCCTGGCCCCACTCCAGGAGAGGTGTGAAAGCCTGGTGGACATTTATTCCCAGCTACAGCAGGAGGT AGGGGCGGCTGGTGGGGAGCTTGAGCCCAAGACCCGGGCATCGCTGACTGGCCGGCTGGATGAAGTCCT GAGAACCCTCGTCACCAGTTGCTTCCTGGTGGAGAAGCAGCCCCCCCAGGTACTGAAGACTCAGACCAAGT TCCAGGCTGGAGTTCGATTCCTGTTGGGCTTGAGGTTCCTGGGGGCCCCAGCCAAGCCTCCGCTGGTCAG GGCCGACATGGTGACAGAGAAGCAGGCGCGGGAGCTGAGTGTGCCTCAGGGTCCTGGGGCTGGAGCAGA AAGCACTGGAGAAATCATCAACAACACTGTGCCCTTGGAGAACAGCATTCCTGGGAACTGCTGCTCTGCCC TGTTCAAGAACCTGCTTCTCAAGAAGATCAAGCGGTGTGAGCGGAAGGGCACTGAGTCTGTCACAGAGGA GAAGTGCGCTGTGCTCTTCTCTGCCAGCTTCACACTTGGCCCCGGCAAACTCCCCATCCAGCTCCAGGCC CTGTCTCTGCCCCTGGTGGTCATCGTCCATGGCAACCAAGACAACAATGCCAAAGCCACTATCCTGTGGG ACAATGCCTTCTCTGAGATGGACCGCGTGCCCTTTGTGGTGGCTGAGCGGGTGCCCTGGGAGAAGATGTG TGAAACTCTGAACCTGAAGTTCATGGCTGAGGTGGGGACCAACCGGGGGCTGCTCCCAGAGCACTTCCTC TTCCTGGCCCAGAAGATCTTCAATGACAACAGCCTCAGTATGGAGGCCTTCCAGCACCGTTCTGTGTCCT GGTCGCAGTTCAACAAGGAGATCCTGCTGGGCCGTGGCTTCACCTTTTGGCAGTGGTTTGATGGTGTCCT GGACCTCACCAAACGCTGTCTCCGGAGCTACTGGTCTGACCGGCTGATCATTGGCTTCATCAGCAAACAG TACGTTACTAGCCTTCTTCTCAATGAGCCCGACGGAACCTTTCTCCTCCGCTTCAGCGACTCAGAGATTG GGGGCATCACCATTGCCCATGTCATCCGGGGCCAGGATGGCTCTCCACAGATAGAGAACATCCAGCCATT CTCTGCCAAAGACCTGTCCATTCGCTCACTGGGGGACCGAATCCGGGATCTTGCTCAGCTCAAAAATCTC TATCCCAAGAAGCCCAAGGATGAGGCTTTCCGGAGCCACTACAAGCCTGAACAGATGGGTAAGGATGGCA GGGGTTATGTCCCAGCTACCATCAAGATGACCGTGGAAAGGGACCAACCACTTCCTACCCCAGAGCTCCA GATGCCTACCATGGTGCCTTCTTATGACCTTGGAATGGCCCCTGATTCCTCCATGAGCATGCAGCTTGGC CCAGATATGGTGCCCCAGGTGTACCCACCACACTCTCACTCCATCCCCCCGTATCAAGGCCTCTCCCCAG AAGAATCAGTCAACGTGTTGTCAGCCTTCCAGGAGCCTCACCTGCAGATGCCCCCCAGCCTGGGCCAGAT GAGCCTGCCCTTTGACCAGCCTCACCCCCAGGGCCTGCTGCCGTGCCAGCCTCAGGAGCATGCTGTGTCC AGCCCTGACCCCCTGCTCTGCTCAGATGTGACCATGGTGGAAGACAGCTGCCTGAGCCAGCCAGTGACAG CGTTTCCTCAGGGCACTTGGATTGGTGAAGACATATTCCCTCCTCTGCTGCCTCCCACTGAACAGGACCT CACTAAGCTTCTCCTGGAGGGGCAAGGGGAGTCGGGGGGAGGGTCCTTGGGGGCACAGCCCCTCCTGCA GCCCTCCCACTATGGGCAATCTGGGATCTCAATGTCCCACATGGACCTAAGGGCCAACCCCAGTTGGTGAT CCCAGCTGGAGGGAGAACCCAAAGAGACAGCTCTTCTACTACCCCCACAGACCTGCTCTGGACACTTGCT CATGCCCTGCCAAGCAGCAGATGGGGAGGGTGCCCTCCTATCCCCACCTACTCCTGGGTCAGGAGGAAAA GACTAACAGGAGAATGCACAGTGGGTGGAGCCAATCCACTCCTTCCTTTCTATCATTCCCCTGCCCACCT CCTTCCAGCACTGACTGGAAGGGAAGTTCAGGCTCTGAGACACACCCCAACATGCCTGCACCTGCAGCGC GCACACGCACGCACACACACATACAGAGCTCTCTGAGGGTGATGGGGCTGAGCAGGAGGGGGGCTGGGT AAGAGCACAGGTTAGGGCATGGAAGGCTTCTCCGCCCATTCTGACCCAGGGCCTAGGACGGATAGGCAGG AACATACAGACACATTTACACTAGAGGCCAGGGATAGAGGATATTGGGTCTCAGCCCTAGGGGAATGGGAA GCAGCTCAAGGGACCCTGGGTGGGAGCATAGGAGGGGTCTGGACATGTGGTTACTAGTACAGGTTTTGCC CTGATTAAAAAATCTCCCAAAGCCCCAAATTCCTGTTAGCCAGGTGGAGGCTTCTGATACGTGTATGAGA CTATGCAAAAGTACAAGGGCTGAGATTCTTCGTGTATAGCTGTGTGAACGTGTATGTACCTAGGATATGT TAAATGTATAGCTGGCACCTTAGTTGCATGACCACATAGAACATGTGTCTATCTGCTTTTGCCTACGTGA CAACACAAATTTGGGAGGGTGAGACACTGCACAGAAGACAGCAGCAAGTGTGCTGGCCTCTCTGACATAT |
| | GCTAACCCCCAAATACTCTGAATTTGGAGTCTGACTGTGCCCAAGTGGGTCCAAGTGGCTGTGACATCTA CGTATGGCTCCACACCTCCAATGCTGCCTGGGAGCCAGGGTGAGAGTCTGGGTCCAGGCCTGGCCATGTG GCCCTCCAGTGTATGAGAGGGCCCTGCCTGCTGCATCTTTTCTGTTGCCCCATCCACCGCCAGCTTCCCT TCACTCCCCTATCCCATTCTCCCTCTCAAGGCAGGGGTCATAGATCCTAAGCCATAAAATAAATTTTATT CCAAAATAACAAAATAAATAATCTACTGTACACAATCTGAAAA |

| | **TLR10 (NM_030956.4)** |
|---|---|
| 37 | AGCCAATTCTGACCGTGTCAACGAATCATCCACGCACCTGCAGCTCTGCTGAGAGAGTGCAAGCCGTGGGA ATTCAGCAGCTGAATATCAAGACCTTTGAATTCAACAAGAAGTTAAGACATTTATAGTTGTCTAACAAC AGACTGAAGATTGTGGCTTGGTATTCACTGGCAGGTTTCAGACATTTAGATCTTTCTTTTAATGACTAAC ACCATGCCTATCTGTGGAGAAGCTGGCAACATGTCACACCTGGAAATTGTTTTTCAACATTAATACTATT ATTTGGCAGTAATCCAGATTGCTTTTGCCACCAACCTGAAGACATATAGAGGCAGAAGGACAGGAATAAT TCTATTTGTTTCCTGTTTTGAAACTTCCATCTGTAAGGCTATCAAAAGGAGATGTGAGAGAGGGTATTGA GTCTGGCCTGACAATGCAGTTCTTAAACCAAAGGTCCATTATGCTTCTCCTCTCTGAGAATCCTGACTTA CCTCAACAACGGAGACATGGCACAGTAGCCAGCTTGGAGACTTCTCAGCCAATGCTCTGAGATCAAGTCG AAGACCCAATATACAGGGTTTTGAGCTCATCTTCATCATTCATATGAGGAAATAAGTGGTAAAATCCTTG GAAATACAATGAGACTCATCAGAAACATTTACATATTTTGTAGTATTGTTATGACAGCAGAGGGTGATGC TCCAGAGCTGCCAGAAGAAAGGGAACTGATGACCAACTGCTCCAACATGTCTCTAAGAAAGGTTCCCGCA GACTTGACCCCAGCCACAACGACACTGGATTTATCCTATAACCTCCTTTTTCAACTCCAGAGTTCAGATT TTCATTCTGTCTCCAAACTGAGAGTTTTGATTCTATGCCATAACAGAATTCAACAGCTGGATCTCAAAAC CTTTGAATTCAACAAGGAGTTAAGATATTTAGATTTGTCTAATAACAGACTGAAGAGTGTAACTTGGTAT TTACTGGCAGGTCTCAGGTATTTAGATCTTTCTTTTAATGACTTTGACACCATGCCTATCTGTGAGGAAG CTGGCAACATGTCACACCTGGAAATCCTAGGTTTGAGTGGGGCAAAAATACAAAAATCAGATTTCCAGAA AATTGCTCATCTGCATCTAAATACTGTCTTCTTAGGATTCAGAACTCTTCCTCATTATGAAGAAGGTAGC CTGCCCATCTTAAACACAACAAAACTGCACATTGTTTTACCAATGGACACAAATTTCTGGGTTCTTTTGC GTGATGGAATCAAGACTTCAAAAATATTAGAAATGACAAATATAGATGGCAAAAGCCAATTTGTAAGTTA TGAAATGCAACGAAATCTTAGTTTAGAAAATGCTAAGACATCGGTTCTATTGCTTAATAAAGTTGATTTA CTCTGGGACGACCTTTTCCTTATCTTACAATTTGTTTGGCATACATCAGTGGAACACTTTCAGATCCGAA ATGTGACTTTTGGTGGTAAGGCTTATCTTGACCACAATTCATTTGACTACTCAAATACTGTAATGAGAAC TATAAAATTGGAGCATGTACATTTCAGAGTGTTTTACATTCAACAGGATAAAATCTATTTGCTTTTGACC AAAATGGACATAGAAAACCTGACAATATCAAATGCACAAATGCCACACATGCTTTTCCCGAATTATCCTA CGAAATTCCAATATTTAAATTTTGCCAATAATATCTTAACAGACGAGTTGTTTAAAAGAACTATCCAACT GCCTCACTTGAAAACTCTCATTTTGAATGGCAATAAACTGGAGACACTTTCTTTAGTAAGTTGCTTTGCT AACAACACACCCTTGGAACACTTGGATCTGAGTCAAAATCTATTACAACATAAAAATGATGAAAATTGCT CATGGCCAGAAACTGTGGTCAATATGAATCTGTCATACAATAAATTGTCTGATTCTGTCTTCAGGTGCTT GCCCAAAAGTATTCAAATACTTGACCTAAATAATAACCAAATCCAAACTGTACCTAAAGAGACTATTCAT CTGATGGCCTTACGAGAACTAAATATTGCATTTAATTTTCTAACTGATCTCCCTGGATGCAGTCATTTCA GTAGACTTTCAGTTCTGAACATTGAAATGAACTTCATTCTCAGCCCATCTCTGGATTTTGTTCAGAGCTG CCAGGAAGTTAAAACTCTAAATGCGGGAAGAAATCCATTCCGGTGTACCTGTGAATTAAAAAATTTCATT CAGCTTGAAACATATTCAGAGGTCATGATGGTTGGATGGTCAGATTCATACACCTGTGAATACCCTTTAA ACCTAAGGGGAACTAGGTTAAAAGACGTTCATCTCCACGAATTATCTTGCAACACAGCTCTGTTGATTGT CACCATTGTGGTTATTATGCTAGTTCTGGGGTTGGCTGTGGCCTTCTGCTGTCTCCACTTTGATCTGCCC TGGTATCTCAGGATGCTAGGTCAATGCACACAAACATGGCACAGGGTTAGGAAAACAACCCAAGAACAAC TCAAGAGAAATGTCCGATTCCACGCATTTATTTCATACAGTGAACATGATTCTCTGTGGGTGAAGAATGA ATTGATCCCCAATCTAGAGAAGGAAGATGGTTCTATCTTGATTTGCCTTTATGAAAGCTACTTTGACCCT GGCAAAAGCATTAGTGAAAATATTGTAAGCTTCATTGAGAAAAGCTATAAGTCCATCTTTGTTTTGTCTC CCAACTTTGTCCAGAATGAGTGGTGCCATTATGAATTCTACTTTGCCCACCACAATCTCTTCCATGAAAA TTCTGATCATATAATTCTTATCTTACTGGAACCCATTCCATTCTATTGCATTCCCACCAGGTATCATAAA CTGAAAGCTCTCCTGGAAAAAAAAGCATACTTGGAATGGCCCAAGGATAGGCGTAAATGTGGGCTTTTCT GGGCAAACCTTCGAGCTGCTATTAATGTTAATGTATTAGCCACCAGAGAAATGTATGAACTGCAGACATT CACAGAGTTAAATGAAGAGTCTCGAGGTTCTACAATCTCTCTGATGAGAACAGATTGTCTATAAAATCCC ACAGTCCTTGGGAAGTTGGGGACCACATACACTGTTGGGATGTACATTGATACAACCTTTATGATGGCAA TTTGACAATATTTATTAAAATAAAAAATGGTTATTCCCTTCATATCAGTTTCTAGAAGGATTTCTAAGAA TGTATCCTATAGAAACACCTTCACAAGTTTATAAGGGCTTATGGAAAAAGGTGTTCATCCCAGGATTGTT TATAATCATGAAAAATGTGGCCAGGTGCAGTGGCTCACTCTTGTAATCCCAGCACTATGGGAGGCCAAGG TGGGTGACCCACGAGGTCAAGAGATGGAGACCATCCTGGCCAACATGGTGAAACCCTGTCTCTACTAAAA ATACAAAAATTAGCTGGGCGTGATGGTGCACGCCTGTAGTCCCAGCTACTTGGGAGGCTGAGGCAGGAGA ATCGCTTGAACCCGGGAGGTGGCAGTTGCAGTGAGCTGAGATCGAGCCACTGCACTCCAGCCTGGTGACA GAGCGAGACTCCATCTCAAAAAAAAGAAAAAAAAAAAAGAAAAAAATGGAAAACATCCTCATGGCCACAA AATAAGGTCTAATTCAATAAATTATAGTACATTAATGTAATATAATATTACATGCCACTAAAAAGAATAA GGTAGCTGTATATTTCCTGGTATGGAAAAAACATATTAATATGTTATAAACTATTAGGTTGGTGCAAAAC TAATTGTGGTTTTTGCCATTGAAATGGCATTGAAATAAAAGTGTAAAGAAATCTATACCAGATGTAGTAA CAGTGGTTTGGGTCTGGGAGGTTGGATTACAGGGAGCATTTGATTTCTATGTTGTGTATTTCTATAATGT TTGAATTGTTTAGAATGAATCTGTATTTCTTTTATAAGTAGAAAAAAAATAAAGATAGTTTTTACAGCCTA |

| | **CD79B (NM_021602.4)** |
|---|---|
| 38 | AGTTACACGTTTTCCTCCAAGGAGCCTCGGACGTTGTCACGGGTTTGGGGTCGGGGACAGAGCGGTGACC ATGGCCAGGCTGGCGTTGTCTCCTGTGCCCAGCCACTGGATGGTGGCGTTGCTGCTGCTGCTCTCAGCTG AGCCAGTACCAGCAGCCAGATCGGAGGACCGGTACCGGAATCCCAAAGGATTCAGCACCTTGGCACAGCT GAAGCAGAGGAACACGCTGAAGGATGGTATCATCATGATCCAGACGCTGCTGATCATCCTCTTCATCATC GTGCCTATCTTCCTGCTGCTGGACAAGGATGACAGCAAGGCTGGCATGGAGGAAGATCACACCTACGAGG GCCTGGACATTGACCAGACAGCCACCTATGAGGACATAGTGACGCTGCGGACAGGGGAAGTGAAGTGGTC TGTAGGTGAGCACCCAGGCCAGGAGTGAGAGCCAGGTCGCCCCATGACCTGGGTGCAGGCTCCCTGGCC TCAGTGACTGCTTCGGAGCTGCCTGGCTCATGGCCCAACCCCTTTCCTGGACCCCCCAGCTGGCCTCTGAA GCTGGCCCACCAGAGCTGCCATTTGTCTCCAGCCCCTGGTCCCCAGCTCTTGCCAAAGGGCCTGGAGTAG AAGGACAACAGGGCAGCAACTTGGAGGGAGTTCTCTGGGGATGGACGGGACCCAGCCTTCTGGGGGTGCT ATGAGGTGATCCGTCCCCACACATGGGATGGGGGAGGCAGAGACTGGTCCAGAGCCCGCAAATGGACTCG GAGCCGAGGGCCTCCCAGCAGAGCTTGGGAAGGGCCATGGACCCAACTGGGCCCCAGAAGAGCCACAG GAACATCATTCCTCTCCCGCAACCACTCCCACCCCAGGGAGGCCCTGGCCTCCAGTGCCTTCCCCCGTGG AATAAACGGTGTGTCCTGAGAAACCA |

| | **S100A8 (NM_002964.5)** |
|---|---|
| 39 | ATGTCTCTTGTCAGCTGTCTTTCAGAAGACCTGGTGGGGCAAGTCCGTGGGCATCATGTTGACCGAGCTG GAGAAAGCCTTGAACTCTATCATCGACGTCTACCACAAGTACTCCCTGATAAAGGGGAATTTCCATGCCG TCTACAGGGATGACCTGAAGAAATTGCTAGAGACCGAGTGTCCTCAGTATATCAGGAAAAAGGGTGCAGA CGTCTGGTTCAAAGAGTTGGATATCAACACTGATGGTGCAGTTAACTTCCAGGAGTTCCTCATTCTGGTG ATAAAGATGGGCGTGGCAGCCCACAAAAAAAGCCATGAAGAAAGCCACAAAGAGTAGCTGAGTTACTGGG CCCAGAGGCTGGGCCCCTGGACATGTACCTGCAGAATAATAAAGTCATCAATACCTCA |

| | **FADD (NM_003824.4)** |
|---|---|
| 40 | GCTCTTGTCGATTTCCTGTAGTGAATCAGGCACCGGAGTGCAGGTTCGGGGGTGGAATCCTTGGGCCGCT GGGCAAGCGGCGAGACCTGGCCAGGGCCAGCGAGCCGAGGACAGAGGGCGCACGGAGGGCCGGGCC GCAGCCCCGGCCGCTTGCAGACCCCGCCATGGACCCGTTCCTGGTGCTGCTGCACTCGGTGTCGTCCAG CCTGTCGAGCAGCGAGCTGACCGAGCTCAAGTTCCTATGCCTCGGGCGCGTGGGCAAGCGCAAGCTGGAC CGCGTGCAGAGCGGCCTAGACCTCTTCTCCATGCTGCTGGAGCAGAACGACCTGGAGCCCGGGCACACC GAGCTCCTGCGCGAGCTGCTCGCCTCCCTGCGGCGCCACGACCTGCTGCGGCGCGTCGACGACTTCGAG GCGGGGGCGGCGGCCGGGGCCGCGCCTGGGGAAGAAGACCTGTGTGCAGCATTTAACGTCATATGTGATA ATGTGGGGAAAGATTGGAGAAGGCTGGCTCGTCAGCTCAAAGTCTCAGACACCAAGATCGACAGCATCGAG GACAGATACCCCCGCAACCTGACAGAGCGTGTGCGGGAGTCACTGAGAATCTGGAAGAACACAGAGAAGG AGAACGCAACAGTGGCCCACCTGGTGGGGGCTCTCAGGTCCTGCCAGATGAACCTGGTGGCTGACCTGGT ACAAGAGGTTCAGCAGGCCCGTGACCTCCAGAACAGGAGTGGGGCCATGTCCCCGATGTCATGGAACTCA GACGCATCTACCTCCGAAGCGTCCTGATGGGCCGCTGCTTTGCGCTGGTGGACCACAGGCATCTACACAGC CTGGACTTTGGTTCTCTCCAGGAAGGTAGCCCAGCACTGTGAAGACCCAGCAGGAAGCCAGGCTGAGTGAG CCACAGACCACCTGCTTCTGAACTCAAGCTGCGTTTATTAATGCCTCTCCCGCACCAGGCCGGGCTTGGGC CCTGCACAGATATTTCCATTTCTTCCTCACTATGACACTGAGCAAGATCTTGTCTCCACTAAATGAGCTCCTG CGGGAGTAGTTGGAAAGTTGGAACCGTGTCCAGCACAGAAGGAATCTGTGCAGATGAGCAGTCACACTGT TACTCCACAGCGGAGGAGACCAGCTCAGAGGCCCAGGAATCGGAGCGAAGCAGAGAGGTGGAGAACTGG GATTTGAACCCCCGCCATCCTTCACCAGAGCCCATGCTCAACCACTGTGGCGTTCTGCTGCCCCTGCAGTT GGCAGAAAGGATGTTTTGTCCCATTTCCTTGGAGGCCACCGGGACAGACCTGGACACTAGGGTCAGGCGG GGTGCTGTGGTGGGGAGAGGCATGGCTGGGGTGGGGGTGGGGAGACCTGGTTGGCCGTGGTCCAGCTCTT GGCCCCTGTGTGAGTTGAGTCTCCTCTCTGAGACTGCTAAGTAGGGGCAGTGATGGTTGCCAGGACGAAT TGAGATAATATCTGTGAGGTGCTGATGAGTGATTGACACACAGCACTCTCTAAATCTTCCTTGTGAGGAT TATGGGTCCTGCAATTCTACAGTTTCTTACTGTTTTGTATCAAAATCACTATCTTTCTGATAACAGAATT GCCAAGGCAGCGGGATCTCGTATCTTTAAAAAGCAGTCCTCTTATTCCTAAGGTAATCCTATTAAAACAC AGCTTTACAACTTCCATACTACAAAAAA |

| | **TNFSF11 (NM_003701.4)** |
|---|---|
| 41 | GCCCGCTCGCCCGCGCGCCCCAGGACCCAAAGCCGGGCTCCAAGTCGGCGCCCCACGTCGAGGCTCC GCCGCAGCCTCCGGAGTTGGCCGCAGACAAGAAGGGGAGGGAGCGGGAGAGGGAGGAGAGCTCCGAAG CGAGAGGGCCGAGCGCCATGCGCCGCGCCAGCAGAGACTACACCAAGTACCTGCGTGGCTCGGAGGAG ATGGGCGGCGGCCCCGGAGCCCCGCACGAGGGCCCCCTGCACGCCCCGCCGCCGCCTGCGCCGCAC CAGCCCCCTGCCGCCTCCCGCTCCATGTTCGTGGCCCTCCTGGGGCTGGGGCTGGGCCAGGTTGTCTGCA GCGTCGCCCTGTTCTTCTATTTCAGAGCGCAGATGGATCCTAATAGAATATCAGAAGATGGCACTCACTGCATT TATAGAATTTTGAGACTCCATGAAAATGCAGATTTTCAAGACACAACTCTGGAGAGTCAAGATACAAAATTAATA CCTGATTCATGTAGGAGAATTAAACAGGCCTTTCAAGGAGCTGTGCAAAAGGAATTACAACATATCGTTGGA TCACAGCACATCAGAGCAGAGAAAGCGATGGTGGATGGCTCATGGTTAGATCTGGCCAAGAGGAGCAAGC TTGAAGCTCAGCCTTTTGCTCATCTCACTATTAATGCCACCGACATCCCATCTGGTTCCCATAAAGTGAG TCTGTCCTCTTGGTACCATGATCGGGGTTGGGCCAAGATCTCCAACATGACTTTTAGCAATGGAAAACTA ATAGTTAATCAGGATGGCTTTTATTACCTGTATGCCAACATTTGCTTTCGACATCATGAAACTTCAGGAG ACCTAGCTACAGAGTATCTTCAACTAATGGTGTACGTCACTAAAACCAGCATCAAAATCCCAAGTTCTCA TACCCTGATGAAAGGAGGAAGCACCAAGTATTGGTCAGGGAATTCTGAATTCCATTTTTATTCCATAAAC |
| | GTTGGTGGATTTTTTAAGTTACGGTCTGGAGAGGAAATCAGCATCGAGGTCTCCAACCCCTCCTTACTGG ATCCGGATCAGGATGCAACATACTTTGGGGCTTTTAAAGTTCGAGATATAGATTGAGCCCCAGTTTTTGG AGTGTTATGTATTTCCTGGATGTTTGGAAACATTTTTTAAAACAAGCCAAGAAAGATGTATATAGGTGTG TGAGACTACTAAGAGGCATGGCCCCAACGGTACACGACTCAGTATCCATGCTCTTGACCTTGTAGAGAAC ACGCGTATTTACAGCCAGTGGGAGATGTTAGACTCATGGTGTGTTACACAATGGTTTTTAAATTTTGTAA TGAATTCCTAGAATTAAACCAGATTGGAGCAATTACGGGGTGACCTTATGAGAAACTGCATGTGGGCTAT GGGAGGGGTTGGTCCCTGGTCATGTGCCCCTTCGCAGCTGAAGTGGAGAGGGTGTCATCTAGCGCAATTG AAGGATCATCTGAAGGGGCAAATTCTTTTGAATTGTTACATCATGCTGGAACCTGCAAAAAATACTTTTT CTAATGAGGAGAGAAAATATATGTATTTTTATATAATATCTAAAGTTATATTTCAGATGTAATGTTTTCT TTGCAAAGTATTGTAAATTATATTTGTGCTATAGTATTTGATTCAAAATATTTAAAAATGTCTTGCTGTT GACATATTTAATGTTTTAAATGTACAGACATATTTAACTGGTGCACTTTGTAAATTCCCTGGGGAAAACT TGCAGCTAAGGAGGGGAAAAAAATGTTGTTTCCTAATATCAAATGCAGTATATTTCTTCGTTCTTTTTAA GTTAATAGATTTTTTCAGACTTGTCAAGCCTGTGCAAAAAAATTAAAATGGATGCCTTGAATAATAAGCA GGATGTTGGCCACCAGGTGCCTTTCAAATTTAGAAACTAATTGACTTTAGAAAGCTGACATTGCCAAAAA GGATACATAATGGGCCACTGAAATCTGTCAAGAGTAGTTATATAATTGTTGAACAGGTGTTTTTCCACAA GTGCCGCAAATTGTACCTTTTTTGTTTTTTCAAAATAGAAAAGTTATTAGTGGTTTATCAGCAAAAAAGT CCAATTTTAATTTAGTAAATGTTATCTTATACTGTACAATAAAAACATTGCCTTTGAATGTTAATTTTTT GGTACAAAAATAAATTTATATGAAAACCTGC |

| | **CYLD (NM_015247.3)** |
|---|---|
| 42 | AGGTAGCAGGTTTGGCTGCGCGGGGGCCGCGCGTCGGAGTTTCCCCCTTTCTAGGGTGAGGATGGTTCTA CACAGCCACCCGGAGTTCCTTAGTTGAAAGGTGCGCCCTGCTGTGACAGAATGTGGTAATTGTAATCTTT AACATTTTCATGTAAAACATATTTCCTGATCATCTTTCCATTGTCTTCATGGAAAATTGATAAATATTTG TGCCTTCCAACTCTCGTCTTGGTTGAATGACTTCATCTTAATACAACATGGACACCACGTTGCTGAAAAC ATGCTTTGGGACTGCCACTGAATTTATCTTTTGCGGTTTTATGACAAAGTTATTAGTAGTTTCCCTTTTT TGAATTAGTATTTTGAAGTTAATATCACAATGAGTTCAGGCTTATGGAGCCAAGAAAAAGTCACTTCACC CTACTGGGAAGAGCGGATTTTTTACTTGCTTCTTCAAGAATGCAGCGTTACAGACAAACAAACACAAAAG CTCCTTAAAGTACCGAAGGGAAGTATAGGACAGTATATTCAAGATCGTTCTGTGGGGCATTCAAGGATTC CTTCTGCAAAAGGCAAGAAAAATCAGATTGGATTAAAAATTCTAGAGCAACCTCATGCAGTTCTCTTTGT TGATGAAAAGGATGTTGTAGAGATAAATGAAAAGTTCACAGAGTTACTTTTGGCAATTACCAATTGTGAG GAGAGGTTCAGCCTGTTTAAAAACAGAAACAGACTAAGTAAAGGCCTCCAAATAGACGTGGGCTGTCCTG TGAAAGTACAGCTGAGATCTGGGGAAGAAAAATTTCCTGGAGTTGTACGCTTCAGAGGACCCCTGTTAGC AGAGAGGACAGTCTCCGGAATATTCTTTGGAGTTGAATTGCTGGAAGAAGGTCGTGGTCAAGGTTTCACT GACGGGGTGTACCAAGGGAAACAGCTTTTTCAGTGTGATGAAGATTGTGGCGTGTTTGTTGCATTGGACA AGCTAGAACTCATAGAAGATGATGACACTGCATTGGAAAGTGATTACGCAGGTCCTGGGGACACAATGCA GGTCGAACTTCCTCCTTTGGAAATAAACTCCAGAGTTTCTTTGAAGGTTGGAGAAACAATAGAATCTGGA ACAGTTATATTCTGTGATGTTTTGCCAGGAAAAGAAAGCTTAGGATATTTTGTTGGTGTGGACATGGATA ACCCTATTGGCAACTGGGATGGAAGATTTGATGGAGTGCAGCTTTGTAGTTTTGCGTGTGTTGAAAGTAC AATTCTATTGCACATCAATGATATCATCCCAGCTTTATCAGAGAGTGTGACGCAGGAAAGGAGGCCTCCC AAACTTGCCTTTATGTCAAGAGGTGTTGGGGACAAAGGTTCATCCAGTCATAATAAACCAAAGGCTACAG GATCTACCTCAGACCCTGGAAATAGAAACAGATCTGAATTATTTTATACCTTAAATGGGTCTTCTGTTGA CTCACAACCACAATCCAAATCAAAAAATACATGGTACATTGATGAAGTTGCAGAAGACCCTGCAAAATCT CTTACAGAGATATCTACAGACTTTGACCGTTCTTCACCACCACTCCAGCCTCCTCCTGTGAACTCACTGA CCACCGAGAACAGATTCCACTCTTTACCATTCAGTCTCACCAAGATGCCCAATACCAATGGAAGTATTGG CCACAGTCCACTTTCTCTGTCAGCCCAGTCTGTAATGGAAGAGCTAAACACTGCACCCGTCCAAGAGAGT CCACCCTTGGCCATGCCTCCTGGGAACTCACATGGTCTAGAAGTGGGCTCATTGGCTGAAGTTAAGGAGA ACCCTCCTTTCTATGGGGTAATCCGTTGGATCGGTCAGCCACCAGGACTGAATGAAGTGCTCGCTGGACT GGAACTGGAAGATGAGTGTGCAGGCTGTACGGATGGAACCTTCAGAGGCACTCGGTATTTCACCTGTGCC CTGAAGAAGGCGCTGTTTGTGAAACTGAAGAGCTGCAGGCCTGACTCTAGGTTTGCATCATTGCAGCCGG TTTCCAATCAGATTGAGCGCTGTAACTCTTTAGCATTTGGAGGCTACTTAAGTGAAGTAGTAGAAGAAAA TACTCCACCAAAAATGGAAAAAGAAGGCTTGGAGATAATGATTGGGAAGAAGAAAGGCATCCAGGGTCAT TACAATTCTTGTTACTTAGACTCAACCTTATTCTGCTTATTTGCTTTTAGTTCTGTTCTGGACACTGTGT TACTTAGACCCAAAGAAAAGAACGATGTAGAATATTATAGTGAAACCCAAGAGCTACTGAGGACAGAAAT TGTTAATCCTCTGAGAATATATGGATATGTGTGTGCCACAAAAATTATGAAACTGAGGAAAATACTTGAA AAGGTGGAGGCTGCATCAGGATTTACCTCTGAAGAAAAAGATCCTGAGGAATTCTTGAATATTCTGTTTC ATCATATTTTAAGGGTAGAACCTTTGCTAAAAATAAGATCAGCAGGTCAAAAGGTACAAGATTGTTACTT CTATCAAATTTTTATGGAAAAAAATGAGAAAGTTGGCGTTCCCACAATTCAGCAGTTGTTAGAATGGTCT TTTATCAACAGTAACCTGAAATTTGCAGAGGCACCATCATGTCTGATTATTCAGATGCCTCGATTTGGAA AAGACTTTAAACTATTTAAAAAAATTTTTCCTTCTCTGGAATTAAATATAACAGATTTACTTGAAGACAC TCCCAGACAGTGCCGGATATGTGGAGGGCTTGCAATGTATGAGTGTAGAGAATGCTACGACGATCCGGAC ATCTCAGCTGGAAAAATCAAGCAGTTTTGTAAAACCTGCAACACTCAAGTCCACCTTCATCCGAAGAGGC TGAATCATAAATATAACCCAGTGTCACTTCCCAAAGACTTACCCGACTGGGACTGGAGACACGGCTGCAT CCCTTGCCAGAATATGGAGTTATTTGCTGTTCTCTGCATAGAAACAAGCCACTATGTTGCTTTTGTGAAG TATGGGAAGGACGATTCTGCCTGGCTCTTCTTTGACAGCATGGCCGATCGGGATGGTGGTCAGAATGGCT TCAACATTCCTCAAGTCACCCCATGCCCAGAAGTAGGAGAGTACTTGAAGATGTCTCTGGAAGACCTGCA |
| | TTCCTTGGACTCCAGGAGAATCCAAGGCTGTGCACGAAGACTGCTTTGTGATGCATATATGTGCATGTAC CAGAGTCCAACAATGAGTTTGTACAAATAACTGGGGTCATCGGGAAAGGCAAAGAAACTGAAGGCAGAGT CCTAACGTTGCATCTTATTCGAGCTGGCAGTTCTGTTCACGTCCATTGCCGGCAATGGATGTCTTTGTGG TGATGATCCTTCAGAAAAGGATGCCTCTGTTTAAAAACAAATTGCTTTTGTGTCCCTGAAGTATTTAATA AGAAGCATTTTGCACTCTAGAAAGTATGTTTGTGTTGGTTTTTTAAGAAGTCTAAATGAAGTTATTAATA CCTGAAGCTTTAAGTTAAGTGCATTGATCATATGATATTTTTGGAAGCATACAATTTTAATTGTGGAAGT TTAAAGCCTCTTTTAGTCCATTGAGAATGTAAATAAATGTGTCTTCTTTATGGACCAAGGATATGAAATC ATTTTTCTTTTGTAGCTAACGGTTGCCTTGAGGAAGAAATAATTTGGTTTTATTAAGAGTCTACTCTCAA TCCAGTTATTAGAGATGTACTGAGTTTGATTTGTTAATCCTTTCTATATACTGCTGATCTTGCATGTCTA CAATCTGCTCAGTTTTTCTGTGTTTCTGCAATAGTGGTCAGAAAAATACTTAAATTCCCTTAATGGTGTT GTTTTCTATTTGTTCTGGTTTTGAGATAAATGAGTGATTCTGTCCCCAAATGTCCATTTTTGAAGTGATT TTCCTGGAGGATTAGGGTATTTAGCAGTTGAAGCTCTTCATTCATAGTAGTTACTGTCAGCTAACAGGTT TTTTAAGGCTTTTAACTATTAATATTTTATGGAATGGGGCAAAGTAAATTGATGAAAGAATTGGAGTGAT AATAGTCCTTTACAAACATACAGTCCATAAGAAAATGAATTTGGCATATAGAATTATTACAATTTCCTGG GAGAGATGGATATTTAAACCTCTATTATTTTAGACAAGACTGTCTAGAACTTAAGTTTGATCTGTCAGCC AGTACTCCCATTAAATTCAGTGTAGTTTCACTTGATAGAATCAGATATGTTATCGAAATGTTAGCAGCAG CTTCATCCTCCTTCTGATTAAAGTAAGTAGAAATGGGATGTTTTGTTTAATAACAGCCATAGTGTGTGTT TAGACCACAGCGGATGTTGTAGACCAGGACCATAGATGATACATGTCAGTGCTGTGGAATGTGCATTCTC TGAGTGTTGTTTTGTGGTATCATTGTCTTTCCTGAATGACTTTCTAACTGTGCAGAAAGGCAGAAAAGTC ATCATATGTATATGTCATATGACTTTATAAAATATTTAATGTGACAAAAAGTGGAAAGAATCTTTACAAA CCCTGCAATTACTTTTTTAAAGGCACTTTTACTCTTTGGTTTTATCATTCCATTTTGCTAATATTTACTA GCTTTATAAATTACAGTAAGGTACAAAAACTCATCTTGTAATATTTTCATTTTTGAAGTGAAAAAGTACA TATATTTTGCACAAGGTTTTATACTGCTAAGTGCTTGGTTGGGGTGGTGAGATGATGATTAGATCAGGGG TGAGGCTGAGAGACTCTGGGTTTAGGGCTAGCCCTGCCTCCATCTCCCTTGGGTAAAATGAAGGGTGTGG GGTAAAAGATGCATAAGGCCTTTTCTAGCTCTGACAGCCTAGAAGTCCAATCACCCTGTAATAAATATGT GTTGAATGAAGAAATGGGTGAATGAGCTTGTCAATGTGATTTTAAAAAATTGACTACCTGGAGGAATGAT TAGGAATCTAAATGAAGCCAGCCCTCGGTATCTGCAGGTTTCTCATCCATGGATTCAACCAACTGCAAAT GGAAAATACGATTTTTTTTAAAAAAAGGATGGTTACATCCGTATTGAACATGTACAGACTTTTTTCTTGT CATTATTCTCTGAACAATACAAGAACTCTTTATGTAGCATTTACATTTATTAGGTATTATAAGTAATCTA GAGATTATTTAATTAAAATATACAGGAGGATGTGTGTCAGTTATATGCAAATTCTGTACCATTTTGTATC AGGGAATTGAGCATCTTCAGATGTTGGTATCTGCAGGGATCCTGGAACCAAACCCCTGCAGATACTAAGG GCTGACGATCTAGGTAAGACTGGATTTAACAGTTGGAAAAAAAAAAAAAAAAGGAGAGAGAAGACAGTTC CTTTCCTGTAGAAATTAAAACAAAATACAAATTGAGGAAGCTCTGCTACCCAGGCTGTCATGGTAGAGAA CTTGAAGAAGACCTGTTTGGATGGACACCTGGTTTCAAAAGTCAGGTGTGGAGACTGTTAAATGGGAGGG CCTCATCCATAAATGATTTCTGGCAACGTCTTCTTCAGGTGGAGCTTGACGTCTTTTTAATGTTACTTGG GGAGGGAGTGCTCATTAAGGGATGCCAGGGCCAGCTCTGGTGGTTCCTGGGGAGGCTGCGTCCTTCCCTG CTTCTGCATGTCATGAGGCAGCAGGAAGGTTTCCCCTGCACCTGTCTGTCCTGGCTCCCTCTGGGTAGCC CCCTACTGTTCTGTGCTTCAGCACAGCCTGGTTTGTCAAGAGGCACATAGTTGGGGCTGGGCTGCATGGC ACAGGGGCTTATGTGCCTGCTGGTTATTTAATTTTCAGCCTTAAGTTTTCTTTAATATTTTCCTGTTGGC TATTTAAAGGTTTTGGTTATCTTTTATTCCTTATCTACAATCAAGATGACAATGTAATTGAATTATCTTA TTTATAACACGGTTCGTGATTCATGATTCATGATTACAAGTAGAAAATATGTCATGTTCCTCACCTCCAA ATAAATATGTGTGTGTCTGTGTGTGTGTATATATGTATGTGGCGGAGAGGGAGAGAGTGGGGAAGGAGAG CAGTGTTATCATACATAGAGAGGCTAAATGTGTCCCATCCCTCACTGTCAGCTTTATAAAGGAGTTTGAC TCCATCCACAGAAGAATGTTTTATAAGACTAGGAAAACACGTTGAAAACTAGGATAAACAGCAACAAAAA TCAACTAAATATGTTGTTACTGTTGCTAAGGATTTTCTCCTTAGAATAATTTAGGATTTTTAAAAATTTC TGTTTGCCAAATGCTGTAGATAAATGGCCAGATTCTTCCTATCCCTAGGATTCCTTTATTATTTTTTTTC ACAGATTTTGAGAACAAGGGGGAGAGATAGTATGGAAGATTAAGATTCCATTAATCTTATAGAACTGTGT TGTCACCCAAATTCCTGCTTGTTTGAACATGGCATCTTCATAGATTCAGGATTCACTACCCTCTATAGCT GGATCTTGAAAATTATCTGGCCAGATAATTTTGCATCTGCTTGGATGATTGTAGACTGAGATGTGAGTGG AGGATAAAGTATTAGACTTTTGCTGAGTAACTGCCAACCAAGAAGTATTTATCGGACACTTACTAGGTGC CTAGGATTGTATCAGAGGGAATATGAAATGTGTCCCTGCCCTACCTAGTTTTAACGACAGAATATCTATT AAAGGCTACTTAGCTGAAGGGTAAGGGTGACAGGTCTAGGGGAAGCTTTGGGAGGTGGTGTGCTGTGACA GAAAAAGTGGCAGAGTAGGGACGAGAGACCTGCATTCTAGCCCTGTTTCTGTCACTTGCTCTGTACACTT AGACAACAGCTTGACCTCTTGAGCTTTAGTTTCCTCCTCTGCATAATGAGAGGGTTAGACTACTGAATTG TATGGGAAAAAAATACAAATTCCTGGGTCCTAGGCCATGCCTGCTGAATCCGACTGTTCAGGAAGAGGCC TAGGAAATCTGTGAGGGAATCCCCAGGGGAATCTCGTGACCAGCCAGGTGTGAAATCTGCTAACTGGAAG ATCTCAAAGCTTCCTTCACTTTTTGTGATTTTGTGGTCATGTAACGTTACTGTATTATTCTACGTAAATG TGGGTACTTGGATGTTTATCATACTGTTTCTCTGTGTTTACATACTAATTTGTGTAAGAAATGCATTTTA GTCTGTGTACCTCAACCTGCTGTTTGTTTCCTAGAGGTGTTAGTAGTCTTTAAATACAAGTAAGACTTAA GAGGATATTTGATGTTATTTACCTGGATATTTTCTTCCCCTTTTATTTATTTAGAGGAAATTGAGATTCT AGGAGCCAAAAAAATGAAAACAAAATTCTAAGGCAAAGTTAAAGAAAAAAATTACATTATTTCTTACCAT TTGCTACTTTATAATGAAAATTTAAAAATTATATGGGAAGATTTTTCTCTGGGATAACAAATCCTTGTCA TAAAGTAAGAGGTCTTTTTAAAGTAGGTAGGCTATAAGGCCTGTAATTTAAAATAATACTCCTTTCTCTA GGGTTTGGTGCAATTCTCCATTAATGAAGATAACATTTGAATTCCCCAAAGCAGGTGAGGAGTCGGGGAG |
| | GAGAAAGCGATGTTAAAATGAAAACTCACTGCAAAAGAGGAGGCAGAGGAAGAAGGAATGTAAACCCCTT AAAGCAGATGTGTGTGGGGCCTTATGAAGACCAGGATTCTGCGGGTGTCAGGGGATTGCCCCTCTTGACA GAGACTAGGGTTTTAGACTGAGGCTTCCTGCAGGGTGTTCGCATTGCCCTTCTCCGTTCCCCTTCAGACC TTTCTGGGGAGAAGAGGTGGGAGGAGGGGAGAAAGACTGTTCATCTTATTCTGAATCCTGGAGCAGCTGA AGGTTTTCTCTTGAGTCAGGATGCAGTGGTAATGCATTAACCAGCAAGTGTGGCCAAGGATAATGAAAAA GTGGGAAAGGAAGGTCCTCCTCCTCCCTGATTGTAGCATCCAGCAGTCTCTGTAGCCAGGTTACTCAAGA ACCACATTTGATTTCCTGGCCCTTTGCCTTGGCAGTGATGGCATTTTTATTTCACTGTGTTTTAAAGTCT TCATTTATTTTTATAACATGGGTTAGGGAGAAGGGCCACAAATGGAGGGATTGTCCTTTCAAGCACCACA GCTTCAGATAAAATTAGTACTTTCAAATATTGTCCACTTTAACTTAAAAAATTCTAGAGGGATTATATTG GAGACTCAACTGCCCTTGGTTTTAGTTTATAAAATGGCCTAGTACTGTGGAATTTTAATTTTAGAAAGTC TTAGCATCAGATCATAAACATTCATTAAAAGAACTCACATCCCATCTGAAACTTCCCAGGGGAGTTGGGA TTCTTAGTAGATTGGTAGAAAGGGGCTCATTTTCTACTGCATTTCCCATTTTTGGTATCTTGTTCAGCAT GTTTTATTTTTATTTCTTGTCTGCAGAACATCCTATATTTATGAGAACATTCTTTAAGAAGACCACCACA TAGAATACCCCTTCCTATCAGCTCGCTCTGATTTAGCCTTAATTTTGTTAAATTTTTTAGAGATGAATGA AGTGCTGCTGTGGAAAGAAATGTACATATACTATTTCTGTATCATTAAAATTACATTTTTATGGTTC |

| | **ITGAL (NM_002209.3)** |
|---|---|
| 43 | ATCATTTTCCTCTTTCACCCTGTCTAGGTTGCCAGCAAATCCCACGGGCCTCCTGACGCTGCCCCTGGGG CCACAGGTCCCTCGAGTGCTGGAAGGATGAAGGATTCCTGCATCACTGTGATGGCCATGGCGCTGCTGTC TGGGTTCTTTTTCTTCGCGCCGGCCTCGAGCTACAACCTGGACGTGCGGGGCGCGCGGAGCTTCTCCCCA CCGCGCGCCGGGAGGCACTTTGGATACCGCGTCCTGCAGGTCGGAAACGGGGTCATCGTGGGAGCTCCA GGGGAGGGGAACAGCACAGGAAGCCTCTATCAGTGCCAGTCGGGCACAGGACACTGCCTGCCAGTCACC CTGAGAGGTTCCAACTATACCTCCAAGTACTTGGGAATGACCTTGGCAACAGACCCCACAGATGGAAGCATT TTGGCCTGTGACCCTGGGCTGTCTCGAACGTGTGACCAGAACACCTATCTGAGTGGCCTGTGTTACCTCT TCCGCCAGAATCTGCAGGGTCCCATGCTGCAGGGGCGCCCTGGTTTTCAGGAATGTATCAAGGGCAACGT AGACCTGGTATTTCTGTTTGATGGTTCGATGAGCTTGCAGCCAGATGAATTTCAGAAAATTCTGGACTTC ATGAAGGATGTGATGAAGAAACTCAGCAACACTTCGTACCAGTTTGCTGCTGTTCAGTTTTCCACAAGCT ACAAAACAGAATTTGATTTCTCAGATTATGTTAAACGGAAGGACCCTGATGCTCTGCTGAAGCATGTAAA GCACATGTTGCTGTTGACCAATACCTTTGGTGCCATCAATTATGTCGCGACAGAGGTGTTCCGGGAGGAG CTGGGGGCCCGGCCAGATGCCACCAAAGTGCTTATCATCATCACGGATGGGGAGGCCACTGACAGTGGCA ACATCGATGCGGCCAAAGACATCATCCGCTACATCATCGGGATTGGAAAGCATTTTCAGACCAAGGAGAG TCAGGAGACCCTCCACAAATTTGCATCAAAACCCGCGAGCGAGTTTGTGAAAATTCTGGACACATTTGAG AAGCTGAAAGATCTATTCACTGAGCTGCAGAAGAAGATCTATGTCATTGAGGGCACAAGCAAACAGGACC TGACTTCCTTCAACATGGAGCTGTCCTCCAGCGGCATCAGTGCTGACCTCAGCAGGGGCCATGCAGTCGT GGGGGCAGTAGGAGCCAAGGACTGGGCTGGGGGCTTTCTTGACCTGAAGGCAGACCTGCAGGATGACACA TTTATTGGGAATGAACCATTGACACCAGAAGTGAGAGCAGGCTATTTGGGTTACACCGTGACCTGGCTGC CCTCCCGGCAAAAGACTTCGTTGCTGGCCTCGGGAGCCCCTCGATACCAGCACATGGGCCGAGTGCTGCT GTTCCAAGAGCCACAGGGCGGAGGACACTGGAGCCAGGTCCAGACAATCCATGGGACCCAGATTGGCTCT TATTTCGGTGGGGAGCTGTGTGGCGTCGACGTGGACCAAGATGGGGAGACAGAGCTGCTGCTGATTGGTG CCCCACTGTTCTATGGGGAGCAGAGAGGAGGCCGGGTGTTTATCTACCAGAGAAGACAGTTGGGGTTTGA AGAAGTCTCAGAGCTGCAGGGGGACCCCGGCTACCCACTCGGGCGGTTTGGAGAAGCCATCACTGCTCTG ACAGACATCAACGGCGATGGGCTGGTAGACGTGGCTGTGGGGGCCCCTCTGGAGGAGCAGGGGGCTGTGT ACATCTTCAATGGGAGGCACGGGGGGCTTAGTCCCCAGCCAAGTCAGCGGATAGAAGGGACCCAAGTGCT CTCAGGAATTCAGTGGTTTGGACGCTCCATCCATGGGGTGAAGGACCTTGAAGGGGATGGCTTGGCAGAT GTGGCTGTGGGGGCTGAGAGCCAGATGATCGTGCTGAGCTCCCGGCCCGTGGTGGATATGGTCACCCTGA TGTCCTTCTCTCCAGCTGAGATCCCAGTGCATGAAGTGGAGTGCTCCTATTCAACCAGTAACAAGATGAA AGAAGGAGTTAATATCACAATCTGTTTCCAGATCAAGTCTCTCATCCCCCAGTTCCAAGGCCGCCTGGTT GCCAATCTCACTTACACTCTGCAGCTGGATGGCCACCGGACCAGAAGACGGGGGTTGTTCCCAGGAGGGA GACATGAACTCAGAAGGAATATAGCTGTCACCACCAGCATGTCATGCACTGACTTCTCATTTCATTTCCC GGTATGTGTTCAAGACCTCATCTCCCCCATCAATGTTTCCCTGAATTTCTCTCTTTGGGAGGAGGAAGGG ACACCGAGGGACCAAAGGGCGCAGGGCAAGGACATACCGCCCATCCTGAGACCCTCCCTGCACTCGGAA ACCTGGGAGATCCCTTTTGAGAAGAACTGTGGGGAGGACAAGAAGTGTGAGGCAAACTTGAGAGTGTCCTT CTCTCCTGCAAGATCCAGAGCCCTGCGTCTAACTGCTTTTGCCAGCCTCTCTGTGGAGCTGAGCCTGAGT AACTTGGAAGAAGATGCTTACTGGGTCCAGCTGGACCTGCACTTCCCCCCGGGACTCTCCTTCCGCAAGG TGGAGATGCTGAAGCCCCATAGCCAGATACCTGTGAGCTGCGAGGAGCTTCCTGAAGAGTCCAGGCTTCT GTCCAGGGCATTATCTTGCAATGTGAGCTCTCCCATCTTCAAAGCAGGCCACTCGGTTGCTCTGCAGATG ATGTTTAATACACTGGTAAACAGCTCCTGGGGGGACTCGGTTGAATTGCACGCCAATGTGACCTGTAACA ATGAGGACTCAGACCTCCTGGAGGACAACTCAGCCACTACCATCATCCCCATCCTGTACCCCATCAACAT CCTCATCCAGGACCAAGAAGACTCCACACTCTATGTCAGTTTCACCCCCAAAGGCCCCAAGATCCACCAA GTCAAGCACATGTACCAGGTGAGGATCCAGCCTTCCATCCACGACCACAACATACCCACCCTGGAGGCTG TGGTTGGGGTGCCACAGCCTCCCAGCGAGGGGCCCATCACACACCAGTGGAGCGTGCAGATGGAGCCTC CCGTGCCCTGCCACTATGAGGATCTGGAGAGGCTCCCGGATGCAGCTGAGCCTTGTCTCCCCGGAGCCCT GTTCCGCTGCCCTGTTGTCTTCAGGCAGGAGATCCTCGTCCAAGTGATCGGGACTCTGGAGCTGGTGGGAG AGATCGAGGCCTCTTCCATGTTCAGCCTCTGCAGCTCCCTCTCCATCTCCTTCAACAGCAGCAAGCATTT CCACCTCTATGGCAGCAACGCCTCCCTGGCCCAGGTTGTCATGAAGGTTGACGTGGTGTATGAGAAGCAG |
| | ATGCTCTACCTCTACGTGCTGAGCGGCATCGGGGGGCTGCTGCTGCTGCTCATTTTCATAGTGCTGT ACAAGGTTGGTTTCTTCAAACGGAACCTGAAGGAGAAGATGGAGGCTGGCAGAGGTGTCCCGAATGGAAT CCCTGCAGAAGACTCTGAGCAGCTGGCATCTGGGCAAGAGGCTGGGGATCCCGGCTGCCTGAAGCCCCTC CATGAGAAGGACTCTGAGAGTGGTGGTGGCAAGGACTGAGTCCAGGCCTGTGAGGTGCAGAGTGCCCAGA ACTGGACTCAGGATGCCCAGGGCCACTCTGCCTCTGCCTGCATTCTGCCGTGTGCCCTCGGGCGAGTCAC TGCCTCTCCCTGGCCCTCAGTTTCCCTATCTCGAACATGGAACTCATTCCTGCCTGTCTCCTTTGCAGGC TCATAGGGAAGACCTGCTGAGGGACCAGCCAAGAGGGCTGCAAAAGTGAGGGCTTGTCATTACCAGACGG TTCACCAGCCTCTCTTGGTTTCCTTCCTTGGAAGAGAATGTCTGATCTAAATGTGGAGAAACTGTAGTCT CAGGACCTAGGGATGTTCTGGCCCTCACCCCTGCCCTGGGATGTCCACAGATGCCTCCACCCCCCAGAAC CTGTCCTTGCACACTCCCCTGCACTGGAGTCCAGTCTCTTCTGCTGGCAGAAAGCAAATGTGACCTGTGT CACTACGTGACTGTGGCACACGCCTTGTTCTTGGCCAAAGACCAAATTCCTTGGCATGCCTTCCAGCACC CTGCAAAATGAGACCCTCGTGGCCTTCCCCAGCCTCTTCTAGAGCCGTGATGCCTCCCTGTTGAAGCTCT GGTGACACCAGCCTTTCTCCCAGGCCAGGCTCCTTCCTGTCTTCCTGCATTCACCCAGACAGCTCCCTCT GCCTGAACCTTCCATCTCGCCACCCCTCCTTCCTTGACCAGCAGATCCCAGCTCACGTCACACTTGGTTG GGTCCTCACATCTTTCACACTTCCACCAGCCTGCACTACTCCCTCAAAGCACACGTCATGTTTCTTCATC CGGCAGCCTGGATGTTTTTTCCCTGTTTAATGATTGACGTACTTAGCAGCTATCTCTCAGTGAACTGTGA GGGTAAAGGCTATACTTGTCTTGTTCACCTTGGGATGATGCCTCATGATATGTCAGGGCGTGGGACATCT AGTAGGTGCTTGACATAATTTCACTGAATTAATGACAGAGCCAGTGGGAAGATACAGAAAAAGAGGGGCT GGGCTGGGCGCGGTGGTTCACGCCTGTAATCCCAGCACTTTGGGAGGCCAAGGAGGGTGGATCACCTGAG GTCAGGAGTTAGAGGCCAGCCTGGCGAAACCCCATCTCTACTAAAAATACAAAATCCAGGCGTGGTGGCA CACACCTGTAGTCCCAGCTACTCAGGAGGTTGAGGTAGGAGAATTGCTTGAACCTGGGAGGTGGAGGTTG CAGTGAGCCAAGATTGCGCCATTGCACTCCAGCCTGGGCAACACAGCGAGACTCCGTCTCAAGGAAAAAA TAAAAATAAAAAGCGGGCACGGGCCCGTGACATCCCCACCCTTGGAGGCTGTCTTCTCAGGCTCTGCCCT GCCCTAGCTCCACACCCTCTCCCAGGACCCATCACGCCTGTGCAGTGGCCCCCACAGAAAGACTGAGCTC AAGGTGGGAACCACGTCTGCTAACTTGGAGCCCCAGTGCCAAGCACAGTGCCTGCATGTATTTATCCAAT AAATGTGAAATTCTGTCCA |

| | **BATF (NM_006399.5)** |
|---|---|
| 44 | AAAGCGAGCGACATGTCCCTTTGGGGAGCAGTCCCTCTGCACCCCAGAGTGAGGAGGACGCAGGGGTCAG AGGTGGCTACAGGGCAGGCAGAGGAGGCACCTGTAGGGGGTGGTGGGCTGGTGGCCCAGGAGAAGTCAG GAAGGGAGCCCAGCTGGTGACAAGAGAGCCCAGAGGTGCCTGGGGCTGAGTGTGAGAGCCCGGAAGATTT CAGCCATGCCTCACAGCTCCGACAGCAGTGACTCCAGCTTCAGCCGCTCTCCTCCCCCTGGCAAACAGGA CTCATCTGATGATGTGAGAAGAGTTCAGAGGAGGGAGAAAAATCGTATTGCCGCCCAGAAGAGCCGACAGA GGCAGACACAGAAGGCCGACACCCTGCACCTGGAGAGCGAAGACCTGGAGAAACAGAACGCGGCTCTAC GCAAGGAGATCAAGCAGCTCACAGAGGAACTGAAGTACTTCACGTCGGTGCTGAACAGCCACGAGCCCCTG TGCTCGGTGCTGGCCGCCAGCACGCCCTCGCCCCCCGAGGTGGTGTACAGCGCCCACGCATTCCACCAA CCTCATGTCAGCTCCCCGCGCTTCCAGCCCTGAGCTTCCGATGCGGGGAGAGCAGAGCCTCGGGAGGGG CACACAGACTGTGGCAGAGCTGCGCCCATCCCGCAGAGGCCCCTGTCCACCTGGAGACCCGGAGACAGA GGCCTGGACAAGGAGTGAACACGGGAACTGTCACGACTGGAAGGGCGTGAGGCCTCCCAGCAGTGCCGC AGCGTTTCGAGGGGCGTGTGCTGGACCCCACCACTGTGGGTTGCAGGCCCAATGCAGAAGAGTATTAAGAA AGATGCTCAAGTCCCATGGCACAGAGCAAGGCGGGCAGGGAACGGTTATTTTTCTAAATAAATGCTTTAAAAG AAA |

| | **IFNAR1 (NM_000629.3)** |
|---|---|
| 45 | GGTGTGACTTAGGACGGGGCGATGGCGGCTGAGAGGAGCTGCGCGTGCGCGAACATGTAACTGGTGGGAT CTGCGGCGGCTCCCAGATGATGGTCGTCCTCCTGGGCGCGACGACCCTAGTGCTCGTCGCCGTGGCGCC ATGGGTGTTGTCCGCAGCCGCAGGTGGAAAAAATCTAAAATCTCCTCAAAAAGTAGAGGTCGACATCATAG ATGACAACTTTATCCTGAGGTGGAACAGGAGCGATGAGTCTGTCGGGAATGTGACTTTTTCATTCGATTA TCAAAAAACTGGGATGGATAATTGGATAAAATTGTCTGGGTGTCAGAATATTACTAGTACCAAATGCAAC TTTTCTTCACTCAAGCTGAATGTTTATGAAGAAATTAAATTGCGTATAAGAGCAGAAAAAGAAAACACTT CTTCATGGTATGAGGTTGACTCATTTACACCATTTCGCAAAGCTCAGATTGGTCCTCCAGAAGTACATTT AGAAGCTGAAGATAAGGCAATAGTGATACACATCTCTCCTGGAACAAAAGATAGTGTTATGTGGGCTTTG GATGGTTTAAGCTTTACATATAGCTTAGTTATCTGGAAAAACTCTTCAGGTGTAGAAGAAAGGATTGAAA ATATTTATTCCAGACATAAAATTTATAAACTCTCACCAGAGACTACTTATTGTCTAAAAGTTAAAGCAGC ACTACTTACGTCATGGAAAATTGGTGTCTATAGTCCAGTACATTGTATAAAGACCACAGTTGAAAATGAA CTACCTCCACCAGAAAATATAGAAGTCAGTGTCCAAAATCAGAACTATGTTCTTAAATGGGATTATACAT ATGCAAACATGACCTTTCAAGTTCAGTGGCTCCACGCCTTTTTAAAAAGGAATCCTGGAAACCATTTGTA TAAATGGAAACAAATACCTGACTGTGAAAATGTCAAAACTACCCAGTGTGTCTTTCCTCAAAACGTTTTC CAAAAAGGAATTTACCTTCTCCGCGTACAAGCATCTGATGGAAATAACACATCTTTTTGGTCTGAAGAGA TAAAGTTTGATACTGAAATACAAGCTTTCCTACTTCCTCCAGTCTTTAACATTAGATCCCTTAGTGATTC ATTCCATATCTATATCGGTGCTCCAAAACAGTCTGGAAACACGCCTGTGATCCAGGATTATCCACTGATT TATGAAATTATTTTTTGGGAAAACACTTCAAATGCTGAGAGAAAAATTATCGAGAAAAAAACTGATGTTA CAGTTCCTAATTTGAAACCACTGACTGTATATTGTGTGAAAGCCAGAGCACACACCATGGATGAAAAGCT GAATAAAAGCAGTGTTTTTAGTGACGCTGTATGTGAGAAAACAAAACCAGGAAATACCTCTAAAATTTGG CTTATAGTTGGAATTTGTATTGCATTATTTGCTCTCCCGTTTGTCATTTATGCTGCGAAAGTCTTCTTGA |
| | GATGCATCAATTATGTCTTCTTTCCATCACTTAAACCTTCTTCCAGTATAGATGAGTATTTCTCTGAACA GCCATTGAAGAATCTTCTGCTTTCAACTTCTGAGGAACAAATCGAAAAATGTTTCATAATTGAAAATATA AGCACAATTGCTACAGTAGAAGAAACTAATCAAACTGATGAAGATCATAAAAAATACAGTTCCCAAACTA GCCAAGATTCAGGAAATTATTCTAATGAAGATGAAAGCGAAAGTAAAACAAGTGAAGAACTACAGCAGGA CTTTGTATGACCAGAAATGAACTGTGTCAAGTATAAGGTTTTTCAGCAGGAGTTACACTGGGAGCCTGAG GTCCTCACCTTCCTCTCAGTAACTACAGAGAGGACGTTTCCCTGTTTAGGGAAAGAAAAAACATCTTCAG ATCATAGGTCCTAAAAATACGGGCAAGCTCTTAACTATTTAAAAATGAAATTACAGGCCCGGGCACGGTG GCTCACACCTGTAATCCCAGCACTTTGGGAGGCTGAGGCAGGCAGATCATGAGGTCAAGAGATCGAGACC AGCCTGGCCAACGTGGTGAAACCCCATCTCTACTAAAAATACAAAAATTAGCCGGGTGTGGTGGCGCGCG CCTGTTGTCTTAGCTACTCAGGAGGCTGAGGCAGGAGAATCGCTTGAAAACAGGAGGTGGAGGTTGCAGT GAGCCGAGATCACGCCACTGCACTCCAGCCTGGTGACAGCGTGAGACTCTTTAAAAAAAGAAATTAAAAG AGTTGAGACAAACGTTTCCTACATTCTTTTCCATGTGTAAAATCATGAAAAAGCCTGTCACCGGACTTGC ATTGGATGAGATGAGTCAGACCAAAACAGTGGCCACCCGTCTTCCTCCTGTGAGCCTAAGTGCAGCCGTG CTAGCTGCGCACCGTGGCTAAGGATGACGTCTGTGTTCCTGTCCATCACTGATGCTGCTGGCTACTGCAT GTGCCACACCTGTCTGTTCGCCATTCCTAACATTCTGTTTCATTCTTCCTCGGGAGATATTTCAAACATT TGGTCTTTTCTTTTAACACTGAGGGTAGGCCCTTAGGAAATTTATTTAGGAAAGTCTGAACACGTTATCA CTTGGTTTTCTGGAAAGTAGCTTACCCTAGAAAACAGCTGCAAATGCCAGAAAGATGATCCCTAAAAATG TTGAGGGACTTCTGTTCATTCATCCCGAGAACATTGGCTTCCACATCACAGTATCTACCCTTACATGGTT TAGGATTAAAGCCAGGCAATCTTTTACTATGCATTAAGACCTCTGATTCAAAACTTATTAGAACAGTAGC TTCTGCTGGAATTTGCAATCACTGAAGTCATAGAAAATAGGTAACTATCTAATTAGAGAAATAATTGTTG TATTTTAAGATCTGAGAGTGTGTACAAGTTTTAGTATACATGCCATGCCAGAAGATAGTGTATGCAAGAA GTCTTGGGACCAGAAAATGGCAATGATAGGAGACTGACATAGAAGAAGAATGCTTCCCTAGGAAAAAGGT CGCTGGCTTTGGTGCAAGAGGAAGAAGAATGTTCCACTGGAAGCCTGAGCACCTAATCAGCTCTCAGTGA TCAACCCACTCTTGTTATGGGTGGTCTCTGTCACTTTGAATGCCAGGCTGGCTTCTCGTCTAGCAGTATT CAGATACCCCTTCTGCTCAGCCTGCTTGGCGTTAAAATACAAATCATTGAACTGAGGGGGAAAAATGTAA CTAGGAAGAAAAACCCAATTTAAGAAATTACATAATGCTTTCCAAAGGCACCTACAACTTAGTTTTAAAT TACTTGCTACTGGGGATTACCCATGGATATCCTTAATAGGCAGGAAGTCTGGGAATTCTGGTGGCCTCTA GGGCAGTGTTCTCACAGCACCGTTCCGACAGGGACCAGTGAAAGAAAAGAGACAAAGTTAGAACGTGCTG GGGAGCGGCCATTTCTAAGGCCAGTCTGGTTTAAGTAGTCATTTCTGCTGAAAAAACAGATGATCCTGGT GGAAGAAAAGGTTGAAGGCAGCTGCCCTCGGGAGGGCTGTGATGCTCGGCACATCCTGCCTGGCACATAC ACGTGTCTGCAGGCCACACCGTGCATGTCCCCAGACCTGCCGCCTGGCTTCTGGAGTGCTTCAAGCAGAG CATGGTGGGTCATTGAGGAGACCCAGGAATCTCATCTGAGAACCCACTCTCTGCCGGAGAACCCCATGGT GACACATTTTCATCTTTCTGACCAGAGGCTGTTTTTTTTTTTTTTTGAGACAGTCTCATTCTGTTGCCCA GGCTGGAGTGCAGTGGCTTGATCTCGGCTCACTGCAACCTCGCCTCCCGGGTTCAAGCAATTCTCTGCCG CAGCCTCCAGAGTAGCTGGGATAACAGGTGCCCACCACCACACCCCACTAATTTTTGTATTTGTATTTTT AGTAGAGATGGGGTTTCACCATGTTGGTCAGGCTGGTCTTGGACTCCTGACCTCATGCTCCACCCGCTTC GGCCTCCCAAAGTTCTGGGATTACAGGTGTGAGCCACCGTGCACGGCCGGCCTGACCTTTGGAAAAGCCT TGTCACTTTGGACGTTTGCGTCTTTGAAGAGGCGATGGGAGCATATCATGACTGCCTGCCACCATTGCTT TTCAGACTACCACAACTCAATCATGCTGTCCAGGACTTCTGGCCCTGTGTTCACCACTGGGAAAACGTAC TTCAGACTGGATAGCCTAAAAAGGAGCAATGCCCTTGTAGGATGTGGAGAAGGGAAAATACGGACATTAA CATTAAAAGACACCAGTGAAATTGTTAGGTCTCTAGGAAGTTGGAGCACAAGGCTTCACGCTTTAAGACC ATCTGTGGTTTTCAGTGAACAAGCGCTGAGCACCAGCAGCAGAAAACAACAACAAAAAAACACCTCGTTT TTACCTTGTCTTCTAGACATGAAAAGGCAGTTGCATTCCACTCTGCATTATGTTCTACATGTTGCTTTAT CAGTATATGCTTAGCTGTAAGTGACAAGTATTTTTTCTGAACAGAAGTTTACTTAGAAATACCATGCACT TGGGGGTACCAATTAACCGCCTGAAAATTAGCATATTGATAGTTCTTAGAGAGACCAGATATAATCTAAG AATTTATATGAAAGATTTGTATCATTAGAGCCAGAAATAATTTTATATTAATATATAATACAGATTAACA TTATATATAATATGTACCTGTGTCACTTCTGACATGAGCCTGTAAACATATATTCATATATGTACCTGCA CATGTACCCACCTGATGTAGGTCTTATTCCTTTAGTATGGACTTAAAGTACTTATTCATATACCTTGTAA CTAAAAATTAGAACAGCTCCCTAGAATTGTGAACTTTTAAGAGTCTGACTAGAAATTTGCAACTTATAAA AAAGTTACTTTTAAAAATATAAGTTAGGGCTAGGCACAGTGGCTCATGCCTATAATCTCAGCACTTTTGG GAGGCCAAGACAGGAGGATCACTTCAGGCCAGGAGTTCAAGATCAACCAACCTGGGTAACATGGCCAGAC CCCATCTCTATTTATATATATATATATAAAACTTAGAGTTTTTATCTTCCCCTAAAAGAGGCCGTGATAT TTGCAGCAGCCTCAAATTGCTCTTAAGGGGTTTAGGTGTGCAGAAGCTTTCCTTTCCCTACCCAGTAACC ATGTGACTACTAACGTGGTATATTGATTTATTTTGTTTGCTGTCTGTCTCCCCTGCCCCACTGCTGGAAC AGAGGCTCCAAGAAAACAGGGACCTTATTATTCATTACTGCATCCCCAGTAATGAAAGTACTTAGAAAAT AATTATTGAATGAATGAAATCTAAACTGTGAACCTGAGGGTGTTTGTGGCAGTGTTTGTTTTACTGAATT GTAGAAGGACATAACCGTGTTTTCAGTGTTTCTATGGAACAAACTTGTACATTTTATTTCACTTGTGTTT TGTCTTAAACCCTACTGCTGGAAACAATTTTATGTAATAAGCAATGGGCCCAAAAGTCTAGGAGTTTTTT TGTACTTAGTGAATTTGTATGCAACAGAGATGCTGCAGCTGATGCCTTTAAAAGGTATTCATCATGGAAG AGCTGAGGCCTGTGCTTGGTGTTCCAGAGCCCAGGGTTGAGCATCCTGAAGGAGCCACTGCAGCCGTCAC TGTCCCCAGAGCCTGTGGAGATAGAGCCTGTTTGCTGCTTTTTCTTCCCGCTCTTAAGACATGGCTGGAG CTCAGTCTTCATTGAATGAAGTTTGCTGTGGTATTGCATAGCCTTGCTTTCTTGAACTAAACTGTTTGCC CTTCACAAGTAGTTCTTCTTTCAGGATTAGTTCGTTCCAAGGAGGCTCTTCAGTCTCACAGATAAGTAGA TCTCTCCTGCTGTCTGGACACATTTCACTCGGAAATTGAATACAATTTGTATTCAGGCTGGGAACCTGAA |
| | CACACACTTGTGTTTTTAAGCTTCCCTTTTTTACAGTGGACAAGGACACAAATAATAAATAAATCATCCC TAATGCCCAAGAAATGCCCTGGTACTTAGTAATAACAAAATACCAGTAACTTCCA |

| | **ICAM3 (NM_002162.5)** |
|---|---|
| 46 | CTCTGTCAGAATGGCCACCATGGTACCATCCGTGTTGTGGCCCAGGGCCTGCTGGACTCTGCTGGTCTGC TGTCTGCTGACCCCAGGTGTCCAGGGGCAGGAGTTCCTTTTGCGGGTGGAGCCCCAGAACCCTGTGCTCT CTGCTGGAGGGTCCCTGTTTGTGAACTGCAGTACTGATTGTCCCAGCTCTGAGAAAATCGCCTTGGAGAC GTCCCTATCAAAGGAGCTGGTGGCCAGTGGCATGGGCTGGGCAGCCTTCAATCTCAGCAACGTGACTGGC AACAGTCGGATCCTCTGCTCAGTGTACTGCAATGGCTCTCAGATAACAGGCTCCTCTAACATCACCGTGT ACAGGCTCCCGGAGCGTGTGGAGCTGGCACCCCTGCCTCCTTGGCAGCCGGTGGGCCAGAACTTCACCC TGCGCTGCCAAGTGGAGGATGGGTCGCCCCGGACCAGCCTCACGGTGGTGCTGCTTCGCTGGGAGGAGG AGCTGAGCCGGCAGCCCGCAGTGGAGGAGCCAGCGGAGGTCACTGCCACTGTGCTGGCCAGCAGAGAC GACCACGGAGCCCCTTTCTCATGCCGCACAGAACTGGACATGCAGCCCCAGGGGCTGGGACTGTTCGTGA ACACCTCAGCCCCCCGCCAGCTCCGAACCTTTGTCCTGCCCGTGACCCCCCCGCGCCTCGTGGCCCCCC GGTTCTTGGAGGTGGAAACGTCGTGGCCGGTGGACTGCACCCTAGACGGGCTTTTTCCAGCCTCAGAGGCC CAGGTCTACCTGGCGCTGGGGGACCAGATGCTGAATGCGACAGTCATGAACCACGGGGACACGCTAACGG CCACAGCCACAGCCACGGCGCGCGCGGATCAGGAGGGTGCCCGGGAGATCGTCTGCAACGTGACCCTAG GGGGCGAGAGACGGGAGGCCCGGGAGAACTTGACGGTCTTTAGCTTCCTAGGACCCATTGTGAACCTCAG CGAGCCCACCGCCCATGAGGGGTCCACAGTGACCGTGAGTTGCATGGCTGGGGCTCGAGTCCAGGTCAC GCTGGACGGAGTTCCGGCCGCGGCCCCGGGGCAGCCAGCTCAACTTCAGCTAAATGCTACCGAGAGTGA CGACGGACGCAGCTTCTTCTGCAGTGCCACTCTCGAGGTGGACGGCGAGTTCTTGCACAGGAACAGTAGCG TCCAGCTGCGAGTCCTGTATGGTCCCAAAATTGACCGAGCCACATGCCCCCAGCACTTGAAATGGAAAGATA AAACGAGACACGTCCTGCAGTGCCAAGCCAGGGGCAACCCGTACCCCGAGCTGCGGTGTTTGAAGGAAGG CTCCAGCCGGGAGGTGCCGGTGGGGATCCCGTTCTTCGTCAACGTAACACATAATGGTACTTATCAGTGCCA AGCGTCCAGCTCACGAGGCAAATACACCCTGGTCGTGGTGATGGACATTGAGGCTGGGAGCTCCCACTTTG TCCCCGTCTTCGTGGCGGTGTTACTGACCCTGGGCGTGGTGACTATCGTACTGGCCTTAATGTACGTCTTC AGGGAGCACCAACGGAGCGGCAGTTACCATGTTAGGGAGGAGAGCACCTATCTGCCCCTCACGTCTATGC AGCCGACAGAAGCAATGGGGGAAGAACCGTCCAGAGCTGAGTGACGCTGGGATCCGGGATCAAAGTTGGC GGGGGCTTGGCTGTGCCCTCAGATTCCGCACCAATAAAGCCTTCAAACTCCCTAA |

| | **STAT2 (NM_005419.4)** |
|---|---|
| 47 | AAGTCGCGACCAGAGCCATTGGAGGGCGCGGGGACTGCAACCCTAATCAGAGCCCAAATGGCGCAGTGG GAAATGCTGCAGAATCTTGACAGCCCCTTTCAGGATCAGCTGCACCAGCTTTACTCGCACAGCCTCCTGCC TGTGGACATTCGACAGTACTTGGCTGTCTGGATTGAAGACCAGAACTGGCAGGAAGCTGCACTTGGGAGT GATGATTCCAAGGCTACCATGCTATTCTTCCACTTCTTGGATCAGCTGAACTATGAGTGTGGCCGTTGCA GCCAGGACCCAGAGTCCTTGTTGCTGCAGCACAATTTGCGGAAATTCTGCCGGGACATTCAGCCCTTTTC CCAGGATCCTACCCAGTTGGCTGAGATGATCTTTAACCTCCTTCTGGAAGAAAAAAGAATTTTGATCCAG GCTCAGAGGGCCCAATTGGAACAAGGAGAGCCAGTTCTCGAAACACCTGTGGAGAGCCAGCAACATGAGA TTGAATCCCGGATCCTGGATTTAAGGGCTATGATGGAGAAGCTGGTAAAATCCATCAGCCAACTGAAAGA CCAGCAGGATGTCTTCTGCTTCCGATATAAGATCCAGGCCAAAGGGAAGACACCCTCTCTGGACCCCCAT CAGACCAAAGAGCAGAAGATTCTGCAGGAAACTCTCAATGAACTGGACAAAAGGAGAAAGGAGGTGCTGG ATGCCTCCAAAGCACTGCTAGGCCGATTAACTACCCTAATCGAGCTACTGCTGCCAAAGTTGGAGGAGTG GAAGGCCCAGCAGCAAAAAGCCTGCATCAGAGCTCCCATTGACCACGGGTTGGAACAGCTGGAGACATGG TTCACAGCTGGAGCAAAGCTGTTGTTTCACCTGAGGCAGCTGCTGAAGGAGCTGAAGGGACTGAGTTGCC TGGTTAGCTATCAGGATGACCCTCTGACCAAAGGGGTGGACCTACGCAACGCCCAGGTCACAGAGTTGCT ACAGCGTCTGCTCCACAGAGCCTTTGTGGTAGAAACCCAGCCCTGCATGCCCCAAACTCCCCATCGACCC CTCATCCTCAAGACTGGCAGCAAGTTCACCGTCCGAACAAGGCTGCTGGTGAGACTCCAGGAAGGCAATG AGTCACTGACTGTGGAAGTCTCCATTGACAGGAATCCTCCTCAATTACAAGGCTTCCGGAAGTTCAACAT TCTGACTTCAAACCAGAAAACTTTGACCCCCGAGAAGGGGCAGAGTCAGGGTTTGATTTGGGACTTTGGT TACCTGACTCTGGTGGAGCAACGTTCAGGTGGTTCAGGAAAGGGCAGCAATAAGGGGCCACTAGGTGTGA CAGAGGAACTGCACATCATCAGCTTCACGGTCAAATATACCTACCAGGGTCTGAAGCAGGAGCTGAAAAC GGACACCCTCCCTGTGGTGATTATTTCCAACATGAACCAGCTCTCAATTGCCTGGGCTTCAGTTCTCTGG TTCAATTTGCTCAGCCCAAACCTTCAGAACCAGCAGTTCTTCTCCAACCCCCCCAAGGCCCCCTGGAGCT TGCTGGGCCCTGCTCTCAGTTGGCAGTTCTCCTCCTATGTTGGCCGAGGCCTCAACTCAGACCAGCTGAG CATGCTGAGAAACAAGCTGTTCGGGCAGAACTGTAGGACTGAGGATCCATTATTGTCCTGGGCTGACTTC ACTAAGCGAGAGAGCCCTCCTGGCAAGTTACCATTCTGGACATGGCTGGACAAAATTCTGGAGTTGGTAC ATGACCACCTGAAGGATCTCTGGAATGATGGACGCATCATGGGCTTTGTGAGTCGGAGCCAGGAGCGCCG GCTGCTGAAGAAGACCATGTCTGGCACCTTTCTACTGCGCTTCAGTGAATCGTCAGAAGGGGGCATTACC TGCTCCTGGGTGGAGCACCAGGATGATGACAAGGTGCTCATCTACTCTGTGCAACCGTACACGAAGGAGG TGCTGCAGTCACTCCCGCTGACTGAAATCATCCGCCATTACCAGTTGCTCACTGAGGAGAATATACCTGA AAACCCACTGCGCTTCCTCTATCCCCGAATCCCCCGGGATGAAGCTTTTGGGTGCTACTACCAGGAGAAA GTTAATCTCCAGGAACGGAGGAAATACCTGAAACACAGGCTCATTGTGGTCTCTAATAGACAGGTGGATG AACTGCAACAACCGCTGGAGCTTAAGCCAGAGCCAGAGCTGGAGTCATTAGAGCTGGAACTAGGGCTGGT GCCAGAGCCAGAGCTCAGCCTGGACTTAGAGCCACTGCTGAAGGCAGGGCTGGATCTGGGGCCAGAGCTA GAGTCTGTGCTGGAGTCCACTCTGGAGCCTGTGATAGAGCCCACACTATGCATGGTATCACAAACAGTGC |
| | CAGAGCCAGACCAAGGACCTGTATCACAGCCAGTGCCAGAGCCAGATTTGCCCTGTGATCTGAGACATTT GAACACTGAGCCAATGGAAATCTTCAGAAACTGTGTAAAGATTGAAGAAATCATGCCGAATGGTGACCCA CTGTTGGCTGGCCAGAACACCGTGGATGAGGTTTACGTCTCCCGCCCCAGCCACTTCTACACTGATGGAC CCTTGATGCCTTCTGACTTCTAGGAACCACATTTCCTCTGTTCTTTTCATATCTCTTGCCCTTCCTACTC CTCATAGCATGATATTGTTCTCCAAGGATGGGAATCAGGCATGTGTCCCTTCCAAGCTGTGTTAACTGTT CAAACTCAGGCCTGTGTGACTCCATTGGGGTGAGAGGTGAAAGCATAACATGGGTACAGAGGGGACAACA ATGAATCAGAACAGATGCTGAGCCATAGGTCTAAATAGGATCCTGGAGGCTGCCTGCTGTGCTGGGAGGT ATAGGGGTCCTGGGGGCAGGCCAGGGCAGTTGACAGGTACTTGGAGGGCTCAGGGCAGTGGCTTCTTTCC AGTATGGAAGGATTTCAACATTTTAATAGTTGGTTAGGCTAAACTGGTGCATACTGGCATTGGCCCTTGG TGGGGAGCACAGACACAGGATAGGACTCCATTTCTTTCTTCCATTCCTTCATGTCTAGGATAACTTGCTT TCTTCTTTCCTTTACTCCTGGCTCAAGCCCTGAATTTCTTCTTTTCCTGCAGGGGTTGAGAGCTTTCTGC CTTAGCCTACCATGTGAAACTCTACCCTGAAGAAAGGGATGGATAGGAAGTAGACCTCTTTTTCTTACCA GTCTCCTCCCCTACTCTGCCCCTAAGCTGGCTGTACCTGTTCCTCCCCCATAAAATGATCCTGCCAATCT AATGTGAGTGTGAAGCTTTGCACACTAGTTTATGCTACCTAGTCTCCACTTTCTCAATGCTTAGGAGACA GATCACTCCTGGAGGCTGGGGATGGTAGGATTGCTGGGGATTTTTTTTTTTTTAAACAGGGTCTCACTCT GTTGCCCAGGCTAGAGTGCAATGGTGCAATCACAGCTCACTGCAGCCTCAACCTCCTGGGTTCAAGCAAT CCTCCTACCTCAGCCTCCTGGGTAGCTAGCACCATGGCATGCGCCACCATGCCCTATTTTTTTTTTTTAA AGACAGGGTCTTGCTATATTGCCCAGGCTGGTCTTGAACTGGGCTCAAGTGATCCTCACGCCTTGGCCTC CCAAAGTGCTGGGATTATAGGCATGAGCCACTGTGCTTGGCCAGGATTTTTTTTTTTTTTTTTTTGAGAT GGAGTTTCTCTCTTGTTGTCCAGGCTGGAGTGCAATGGTGTGATCTCGGCTCACTGCAACCTCCGCCTTC CGGGTTCAAGTGACTCTCCTGCCTCAGCCTCCCCAGTAGCTGGGATTACAGATCTGCACCACCATGCCCA GCTAATTTTGTATTTTTAGTAGAGACGGGGTTTCTCCATGTTGGTCAGGCTGGTCTCGAACTCCTGACCT CAAGTGATCTGTCCACCTCGGCCTCCCAGAGTGCTGGGATTACAGGCGTGAGCCACTGTTCCCAGCAGGA ATTTCTTTTTTATAGTATTGGATAAAGTTTGGTGTTTTTACAGAGGAGAAGCAATGGGTCTTAGCTCTTT CTCTATTATGTTATCATCCTCCCTTTTTTGTACAATATGTTGTTTACCTGAAAGGAAGGTTTCTATTCGT TGGTTGTGGACCTGGACAAAGTCCAAGTCTGTGGAACTTAAAACCTTGAAGGTCTGTCATAGGACTCTGG ACAATCTCACACCTTAGCTATTCCCAGGGAACCCCAGGGGGCAACTGACATTGCTCCAAGATGTTCTCCT GATGTAGCTTGAGATATAAAGGAAAGGCCCTGCACAGGTGGCTGTTTCTTGTCTGTTATGTCAGAGGAAC AGTCCTGTTCAGAAAGGGGCTCTTCTGAGCAGAAATGGCTAATAAACTTTGTGCTGATCTGGAAA |

| | **RPS6 (NM_001010.3)** |
|---|---|
| 48 | CTCTTTTCCGTGGCGCCTCGGAGGCGTTCAGCTGCTTCAAGATGAAGCTGAACATCTCCTTCCCAGCCAC TGGCTGCCAGAAACTCATTGAAGTGGACGATGAACGCAAACTTCGTACTTTCTATGAGAAGCGTATGGCC ACAGAAGTTGCTGCTGACGCTCTGGGTGAAGAATGGAAGGGTTATGTGGTCCGAATCAGTGGTGGGAACG ACAAACAAGGTTTCCCCATGAAGCAGGGTGTCTTGACCCATGGCCGTGTCCGCCTGCTACTGAGTAAGGG GCATTCCTGTTACAGACCAAGGAGAACTGGAGAAAGAAAGAGAAAATCAGTTCGTGGTTGCATTGTGGAT GCAAATCTGAGCGTTCTCAACTTGGTTATTGTAAAAAAAGGAGAGAAGGATATTCCTGGACTGACTGATA CTACAGTGCCTCGCCGCCTGGGCCCCAAAAGAGCTAGCAGAATCCGCAAACTTTTCAATCTCTCTAAAGA AGATGATGTCCGCCAGTATGTTGTAAGAAAGCCCTTAAATAAAGAAGGTAAGAAACCTAGGACCAAAGCA CCCAAGATTCAGCGTCTTGTTACTCCACGTGTCCTGCAGCACAAACGGCGGCGTATTGCTCTGAAGAAGC AGCGTACCAAGAAAAATAAAGAAGAGGCTGCAGAATATGCTAAACTTTTGGCCAAGAGAATGAAGGAGGC TAAGGAGAAGCGCCAGGAACAAATTGCGAAGAGACGCAGACTTTCCTCTCTGCGAGCTTCTACTTCTAAG TCTGAATCCAGTCAGAAATAAGATTTTTTGAGTAACAAATAAATAAGATCAGACTCTGATCTCTGTTGAC TTTTCATTGCTGATAGAAAACTACATAAATGAAGAGAGGGAAAGTTAGGTCATAGAAAATGGGGATATGT ATATATGGTATTCCAATGGTGGATAAAGTCAGACCTATTGTGTGTGGTAGTGGAAGACAAGGTCAGTGTA CAGGGGTTCCTTTCAGGGGATGGGTGGCACAGGAAGGGATTCTGAGCATAAGACACCTGCCTCTTCAGAA TGGCATGGAACTTAGGGGAAATGATGAGGACTATTAATCTTTTTTATATAGTTTATTTTTGAGGAATATA GCTCTTTAAAGCACTATACACAATTTAATACATGAAAATTGACCTTGTGAAGATGAACATGTCTTTTTTA AAAGGTAACTTTTAAGGCATAGTTTTAGTGGATACCTAGTTTTAGTGGATACCATCCACTAAATGGTTTT TAAGCCATTTCAAATTTGTTTTAACAAAGCTTTTCAACTCCAGTGAATTGTTAGGCTGTTCACACACTGA AAAGGAAATGACTAAAAACATTCTTGGATCAGTGAAAAA |

| | **TNFRSF12A (NM_016639.3)** |
|---|---|
| 49 | GCAGACAGCGGCGGGCGCAGGACGTGCACTATGGCTCGGGGCTCGCTGCGCCGGTTGCTGCGGCTCCTC GTGCTGGGGCTCTGGCTGGCGTTGCTGCGCTCCGTGGCCGGGGAGCAAGCGCCAGGCACCGCCCCCTG CTCCCGCGGCAGCTCCTGGAGCGCGGACCTGGACAAGTGCATGGACTGCGCGTCTTGCAGGGCGCGACC GCACAGCGACTTCTGCCTGGGCTGCGCTGCAGCACCTCCTGCCCCCTTCCGGCTGCTTTGGCCCATCCTTG GGGGCGCTCTGAGCCTGACCTTCGTGCTGGGGCTGCTTTCTGGCTTTTTGGTCTGGAGACGATGCCGCAGG AGAGAGAAGTTCACCACCCCCATAGAGGAGACCGGCGGAGAGGGCTGCCCAGCTGTGGCGCTGATCCAGT GACAATGTGCCCCCTGCCAGCCGGGGCTCGCCCACTCATCATTCATTCATCCATTCTAGAGCCAGTCTCTGC CTCCCAGACGCGGCGGGAGCCAAGCTCCTCCAACCACAAGGGGGGTGGGGGGCGGTGAATCACCTCTGA GGCCTGGGCCCAGGGTTCAGGGGAACCTTCCAAGGTGTCTGGTTGCCCTGCCTCTGGCTCCAGAACAGAAA GGGAGCCTCACGCTGGCTCACACAAAACAGCTGACACTGACTAAGGAACTGCAGCATTTGCACAGGGGAG GGGGGTGCCCTCCTTCCTAGAGGCCCTGGGGGCCAGGCTGACTTGGGGGGCAGACTTGACACTAGGCCC CACTCACTCAGATGTCCTGAAATTCCACCACGGGGGTCACCCTGGGGGGTTAGGGACCTATTTTTAACACT |
| | AGGGGGCTGGCCCACTAGGAGGGCTGGCCCTAAGATACAGACCCCCCCAACTCCCCAAAGCGGGGAGGA GATATTTATTTTGGGGAGAGTTTGGAGGGGAGGGAGAATTTATTAATAAAAGAATCTTTAACTTTAAA |

| | **CDKN1A (NM_000389.5)** |
|---|---|
| 50 | GAGGTGTGAGCAGCTGCCGAAGTCAGTTCCTTGTGGAGCCGGAGCTGGGCGCGGATTCGCCGAGGCACC GAGGCACTCAGAGGAGGCGCCATGTCAGAACCGGCTGGGGATGTCCGTCAGAACCCATGCGGCAGCAAG GCCTGCCGCCGCCTCTTCGGCCCAGTGGACAGCGAGCAGCTGAGCCGCGACTGTGATGCGCTAATGGCG GGCTGCATCCAGGAGGCCCGTGAGCGATGGAACTTCGACTTTGTCACCGAGACACCACTGGAGGGTGACTT CGCCTGGGAGCGTGTGCGGGGCCTTGGCCTGCCCAAGCTCTACCTTCCCACGGGGCCCCGGCGAGGCC GGGATGAGTTGGGAGGAGGCAGGCGGCCTGGCACCTCACCTGCTCTGCTGCAGGGGACAGCAGAGGAAG ACCATGTGGACCTGTCACTGTCTTGTACCCTTGTGCCTCGCTCAGGGGAGCAGGCTGAAGGGTCCCCAGGT GGACCTGGAGACTCTCAGGGTCGAAAACGGCGGCAGACCAGCATGACAGATTTCTACCACTCCAAACGCC GGCTGATCTTCTCCAAGAGGAAGCCCTAATCCGCCCACAGGAAGCCTGCAGTCCTGGAAGCGCGAGGGCC TCAAAGGCCCGCTCTACATCTTCTGCCTTAGTCTCAGTTTGTGTGTCTTAATTATTATTTGTGTTTTAATTTAA ACACCTCCTCATGTACATACCCTGGCCGCCCCCTGCCCCCCAGCCTCTGGCATTAGAATTATTTAAACAA AAACTAGGCGGTTGAATGAGAGGTTCCTAAGAGTGCTGGGCATTTTTATTTTATGAAATACTATTTAAAG CCTCCTCATCCCGTGTTCTCCTTTTCCTCTCTCCCGGAGGTTGGGTGGGCCGGCTTCATGCCAGCTACTT CCTCCTCCCCACTTGTCCGCTGGGTGGTACCCTCTGGAGGGGTGTGGCTCCTTCCCATCGCTGTCACAGG CGGTTATGAAATTCACCCCCTTTCCTGGACACTCAGACCTGAATTCTTTTTCATTTGAGAAGTAAACAGA TGGCACTTTGAAGGGGCCTCACCGAGTGGGGGCATCATCAAAAACTTTGGAGTCCCCTCACCTCCTCTAA GGTTGGGCAGGGTGACCCTGAAGTGAGCACAGCCTAGGGCTGAGCTGGGGACCTGGTACCCTCCTGGCTC TTGATACCCCCCTCTGTCTTGTGAAGGCAGGGGGAAGGTGGGGTCCTGGAGCAGACCACCCCGCCTGCCC TCATGGCCCCTCTGACCTGCACTGGGGAGCCCGTCTCAGTGTTGAGCCTTTTCCCTCTTTGGCTCCCCTG TACCTTTTGAGGAGCCCCAGCTACCCTTTTTCTCCAGCTGGGCTCTGCAATTCCCCTCTGCTGCTGTCCC TCCCCCTTGTCCTTTCCCTTCAGTACCCTCTCAGCTCCAGGTGGCTCTGAGGTGCCTGTCCCACCCCCAC CCCCAGCTCAATGGACTGGAAGGGGAAGGGACACACAAGAAGAAGGGCACCCTAGTTCTACCTCAGGCAG CTCAAGCAGCGACCGCCCCCTCCTCTAGCTGTGGGGGTGAGGGTCCCATGTGGTGGCACAGGCCCCCTTG AGTGGGGTTATCTCTGTGTTAGGGGTATATGATGGGGGAGTAGATCTTTCTAGGAGGGAGACACTGGCCC CTCAAATCGTCCAGCGACCTTCCTCATCCACCCCATCCCTCCCCAGTTCATTGCACTTTGATTAGCAGCG GAACAAGGAGTCAGACATTTTAAGATGGTGGCAGTAGAGGCTATGGACAGGGCATGCCACGTGGGCTCAT ATGGGGCTGGGAGTAGTTGTCTTTCCTGGCACTAACGTTGAGCCCCTGGAGGCACTGAAGTGCTTAGTGT ACTTGGAGTATTGGGGTCTGACCCCAAACACCTTCCAGCTCCTGTAACATACTGGCCTGGACTGTTTTCT CTCGGCTCCCCATGTGTCCTGGTTCCCGTTTCTCCACCTAGACTGTAAACCTCTCGAGGGCAGGGACCAC ACCCTGTACTGTTCTGTGTCTTTCACAGCTCCTCCCACAATGCTGAATATACAGCAGGTGCTCAATAAAT GATTCTTAGTGACTTTA |

| | **TMEFF2 (NM_016192.4)** |
|---|---|
| 51 | AGAGGGATGCGGGCGGCAGAGCTCGAGAGGCGGCTGCCGGGCTGCGGGGCGCCTTGACTCTCCCTCCA CCCTGCCTCCTCGGGCTCCACTCGTCTGCCCCTGGACTCCCGTCTCCTCCTGTCCTCCGGCTTCCCAGAG CTCCCTCCTTATGGCAGCAGCTTCCCGCGTCTCCGGCGCAGCTTCTCAGCGGACGACCCTCTCGCTCCGG GGCTGAGCCCAGTCCCTGGATGTTGCTGAAACTCTCGAGATCATGCGCGGGTTTGGCTGCTGCTTCCCCGC CGGGTGCCACTGCCACCGCCGCCGCCTCTGCTGCCGCCGTCCGCGGGATGCTCAGTAGCCCGCTGCCC GGCCCCCGCGATCCTGTGTTCCTCGGAAGCCGTTTGCTGCTGCAGAGTTGCACGAACTAGTCATGGTGCTGT GGGAGTCCCCGCGGCAGTGCAGCAGCTGGACACTTTGCGAGGGCTTTTGCTGGCTGCTGCTGCTGCCCGT CATGCTACTCATCGTAGCCCGCCCGGTGAAGCTCGCTGCTTTCCCTACCTCCTTAAGTGACTGCCAAACGC CCACCGGCTGGAATTGCTCTGGTTATGATGACAGAGAAAATGATCTCTTCCTCTGTGACACCAACACCTG TAAATTTGATGGGGAATGTTTAAGAATTGGAGACACTGTGACTTGCGTCTGTCAGTTCAAGTGCAACAAT GACTATGTGCCTGTGTGTGGCTCCAATGGGGAGAGCTACCAGAATGAGTGTTACCTGCGACAGGCTGCAT GCAAACAGCAGAGTGAGATACTTGTGGTGTCAGAAGGATCATGTGCCACAGATGCAGGATCAGGATCTGG AGATGGAGTCCATGAAGGCTCTGGAGAAACTAGTCAAAAGGAGACATCCACCTGTGATATTTGCCAGTTT GGTGCAGAATGTGACGAAGATGCCGAGGATGTCTGGTGTGTGTGTAATATTGACTGTTCTCAAACCAACT TCAATCCCCTCTGCGCTTCTGATGGGAAATCTTATGATAATGCATGCCAAATCAAAGAAGCATCGTGTCA GAAACAGGAGAAAATTGAAGTCATGTCTTTGGGTCGATGTCAAGATAACACAACTACAACTACTAAGTCT GAAGATGGGCATTATGCAAGAACAGATTATGCAGAGAATGCTAACAAATTAGAAGAAAGTGCCAGAGAAC ACCACATACCTTGTCCGGAACATTACAATGGCTTCTGCATGCATGGGAAGTGTGAGCATTCTATCAATAT GCAGGAGCCATCTTGCAGGTGTGATGCTGGTTATACTGGACAACACTGTGAAAAAAAGGACTACAGTGTT CTATACGTTGTTCCCGGTCCTGTACGATTTCAGTATGTCTTAATCGCAGCTGTGATTGGAACAATTCAGA TTGCTGTCATCTGTGTGGTGGTCCTCTGCATCACAAGGAAATGCCCCAGAAGCAACAGAATTCACAGACA GAAGCAAAATACAGGGCACTACAGTTCAGACAATACAACAAGAGCGTCCACGAGGTTAATCTAAAGGGAG CATGTTTCACAGTGGCTGGACTACCGAGAGCTTGGACTACACAATACAGTATTATAGACAAAAGAATAAG ACAAGAGATCTACACATGTTGCCTTGCATTTGTGGTAATCTACACCAATGAAAACATGTACTACAGCTAT ATTTGATTATGTATGGATATATTTGAAATAGTATACATTGTCTTGATGTTTTTTCTGTAATGTAAATAAA CTATTTATATCACACAATATAGTTTTTTCTTTCCCATGTATTTGTTATATATAATAAATACTCAGTGATG AGAAAAAATTGGCATTCTTAAATTTGCGGTATCTCATAACTGTAAATATAATCAGACTAGTACAATCTGT ACAGCTACCAATATTTCATGTTTCTTCTCATCTTGAGACAGCACATTAGTTCGTACAGGACTCAGTGGCT |
| | AGGTTTTGAATGATTCCAAGATCAAGGGAAATGATGGTTATTGGAAAAGAGAAAAAATAATTTACTTTAT ATCGAGTGAGGATAAAATATTTCCGATCTTTGAATCATCTCTATTTCATCAACTTTCTTCCCTGGTCTTC AGGTTTTGAATGATTCCAAGATCAAGGGAAATGATGGTTATTGGAAAAGAGAAAAAATAATTTACTTTAT ATCGAGTGAGGATAAAATATTTCCGATCTTTGAATCATCTCTATTTCATCAACTTTCTTCCCTGGTCTTC CATTTTCATCCCTAGAGCAGAAAAATCTCTGGCATATAAACTAAATAAAAGAAGAAGGGGAGGGAAAGTG TTTTATAACTCATAAAGGAGAGGGAAAGAAAATATTGGTTTTTATTGGGGAAGTAGCTTAGAATCCCCCA GTTAAGTGCATATATCTGAACTTACTGAACAAGTTACATACTAGGTATACACAGAGTGGCAAAATATATT CCATTTAGGTGGGTGGAATTACCAGGGGAAAAATGTAATAACACCACTAGATGTGAAACACCAAAATCGT GAATTCTCAAAAGCACCATACAATATGTATAGTATATAGTTCTTTGAAAAGAAGTTAGAATCACAACCAA TACCCCATGAATAGCTTTGTGGCTAATGCAGCACCATAATTTGTAATGGAACTAAGATGATGATGACGAT ATTTCATGAAAACAGAGAGATGTTTTGAGCATATTTATGTGGTGAGGTAAGAAAGAAAATTAATCCTATA GCATCTGAAAGACCTCACTGGGAAGTTGGTATGGATTTTTGTTTGATTTGTGCATACAAATAGGTATCAC AACTTGATCTGGAAAAAATAAGCTGTGAAAATTCTCAAGGAATAAGATGAAAATAAATCAATTATTATCA TTTAGCTCTGCAAAGCTTTCCATGGCTAACACAGTAAATTTAAATAAAGCTCTCTTTGTCTCCTTCAAA |

| | **PSMB9 (NM_002800.5)** |
|---|---|
| 52 | AGTGCCCCAGGCGGCGAGGAGAGCGGTGCCTTGCAGGGATGCTGCGGGCGGGAGCACCAACCGGGGAC TTACCCCGGGCGGGAGAAGTCCACACCGGGACCACCATCATGGCAGTGGAGTTTGACGGGGGCGTTGTGA TGGGTTCTGATTCCCGAGTGTCTGCAGGCGAGGCGGTGGTGAACCGAGTGTTTGACAAGCTGTCCCCGCTG CACGAGCGCATCTACTGTGCACTCTCTGGTTCAGCTGCTGATGCCCAAGCCGTGGCCGACATGGCCGCCTA CCAGCTGGAGCTCCATGGGATAGAACTGGAGGAACCTCCACTTGTTTTGGCTGCTGCAAATGTGGTGAGA AATATCAGCTATAAATATCGAGAGGACTTGTCTGCACATCTCATGGTAGCTGGCTGGGACCAACGTGAAG GAGGTCAGGTATATGGAACCCTGGGAGGAATGCTGACTCGACAGCCTTTTGCCATTGGTGGCTCCGGCAG CACCTTTATCTATGGTTATGTGGATGCAGCATATAAGCCAGGCATGTCTCCCGAGGAGTGCAGGCGCTTC ACCACAGACGCTATTGCTCTGGCCATGAGCCGGGATGGCTCAAGCGGGGGTGTCATCTACCTGGTCACTA TTACAGCTGCCGGTGTGGACCATCGAGTCATCTTGGGCAATGAACTGCCAAAATTCTATGATGAGTGAAC CTTCCCCAGACTTCTCTTTCTTATTTTGTAATAAACTCTCTAGGGCCAAAACCTGGTATGGTCATTGGGA AATGAGTGCTCAGGGAGATGGAGCTTAGGGGAGGTGGGTGCTTCCCTCCTAGATGTCAGCATACACTCTT TCTTCTTTTGTCCCAGGTCTAAAACATCTTTCCTAGAGAAAACAAAAGGGACTAAACTAGAAATATAAAG AGCCCTATACATGACAGGTGATCACGTACTGAATGATTTTGAAGTAGTACAAACAATAAAAATTCTCATT CCGCATCATCATGCGGTCCATGATGATGAGGCCGCAA |

| | **HLA-C (NM_002117.6)** |
|---|---|
| 53 | ACATTCTCCCCAGAGGCCGAGATGCGGGTCATGGCGCCCCGAGCCCTCCTCCTGCTGCTCTCGGGAGGC CTGGCCCTGACCGAGACCTGGGCCTGCTCCCACTCCATGAGGTATTTCGACACCGCCGTGTCCCGGCCCG GCCGCGGAGAGCCCCGCTTCATCTCAGTGGGCTACGTGGACGACACGCAGTTCGTGCGGTTCGACAGCGA CGCCGCGAGTCCGAGAGGGGAGCCGCGGGCGCCGTGGGTGGAGCAGGAGGGGCCGGAGTATTGGGAC CGGGAGACACAGAAGTACAAGCGCCAGGCACAGGCTGACCGAGTGAGCCTGCGGAACCTGCGCGGCTAC TACAACCAGAGCGAGGACGGGTCTCACACCCTCCAGAGGATGTCTGGCTGCGACCTGGGGCCCGACGGG CGCCTCCTCCGCGGGTATGACCAGTCCGCCTACGACGGCAAGGATTACATCGCCCTGAACGAGGACCTGC GCTCCTGGACCGCCGCGGACACCGCGGCTCAGATCACCCAGCGCAAGTTGGAGGCGGCCCGTGCGGCG GAGCAGCTGAGAGCCTACCTGGAGGGCACGTGCGTGGAGTGGCTCCGCAGATACCTGGAGAACGGGAAG GAGACGCTGCAGCGCGCAGAACCCCCAAAGACACACGTGACCCACCACCCCCTCTCTGACCATGAGGCC ACCCTGAGGTGCTGGGCCCTGGGCTTCTACCCTGCGGAGATCACACTGACCTGGCAGCGGGATGGGGAGG ACCAGACCCAGGACACCGAGCTTGTGGAGACCAGGCCAGCAGGAGATGGAACCTTCCAGAAGTGGGCAG CTGTGGTGGTGCCTTCTGGACAAGAGCAGAGATACACGTGCCATATGCAGCACGAGGGGCTGCAAGAGCC CCTCACCCTGAGCTGGGAGCCATCTTCCCAGCCCACCATCCCCATCATGGGCATCGTTGCTGGCCTGGCTG TCCTGGTTGTCCTAGCTGTCCTTGGAGCTGTGGTCACCGCTATGATGTGTAGGAGGAAGAGCTCAGGTGGAA AAGGAGGGAGCTGCTCTCAGGCTGCGTGCAGCAACAGTGCCCAGGGCTCTGATGAGTCTCTCATCACTTGT AAAGCCTGAGACAGCTGCCTGTGTGGGACTGAGATGCAGGATTTCTTCACACCTCTCCTTTGTGACTTCAAGA GCCTCTGGCATCTCTTTCTGCAAAGGCACCTGAATGTGTCTGCGTTCCTGTTAGCATAATGTGAGGAGGTGGA GAGACAGCCCACCCCCGTGTCCACCGTGACCCCTGTCCCCACACTGACCTGTGTTCCCTCCCCGATCATC TTTCCTGTTCCAGAGAGGTGGGGCTGGATGTCTCCATCTCTGTCTCAAATTCATGGTGCACTGAGCTGCAA CTTCTTACTTCCCTAATGAAGTTAAGAACCTGAATATAAATTTGTGTTCTCAAATATTTGCTATGAAGCG TTGATGGATTAATTAAATAAGTCAATTCCTAGAAGTTGAGAGAGCAAATAAAGACCTGAGAACCTTCCAGAA |

| | **LCK (NM_005356.5)** |
|---|---|
| 54 | GAGACAGGTGGTGGCTACGACGGCGAAGGGAGCTGAGACTGTCCAGGCAGCCAGGTTAGGCCAGGAGGA CCATGTGAATGGGGCCAGAGGGCTCCCGGGCTGGGCAGGGACCATGGGCTGTGGCTGCAGCTCACACCC GGAAGATGACTGGATGGAAAACATCGATGTGTGTGAGAACTGCCATTATCCCATAGTCCCACTGGATGGCAA GGGCACGCTGCTCATCCGAAATGGCTCTGAGGTGCGGGACCCACTGGTTACCTACGAAGGCTCCAATCCG CCGGCTTCCCCACTGCAAGACAACCTGGTTATCGCTCTGCACAGCTATGAGCCCTCTCACGACGGAGATC TGGGCTTTGAGAAGGGGGAACAGCTCCGCATCCTGGAGCAGAGCGGCGAGTGGTGGAAGGCGCAGTCCCT GACCACGGGCCAGGAAGGCTTCATCCCCTTCAATTTTGTGGCCAAAGCGAACAGCCTGGAGCCCGAACCC TGGTTCTTCAAGAACCTGAGCCGCAAGGACGCGGAGCGGCAGCTCCTGGCGCCCGGGAACACTCACGGC TCCTTCCTCATCCGGGAGAGCGAGAGCACCGCGGGATCGTTTTCACTGTCGGTCCGGGACTTCGACCAGAA CCAGGGAGAGGTGGTGAAACATTACAAGATCCGTAATCTGGACAACGGTGGCTTCTACATCTCCCCTCGA ATCACTTTTCCCGGCCTGCATGAACTGGTCCGCCATTACACCAATGCTTCAGATGGGCTGTGCACACGGT |
| | TGAGCCGCCCCTGCCAGACCCAGAAGCCCCAGAAGCCGTGGTGGGAGGACGAGTGGGAGGTTCCCAGG GAGACGCTGAAGCTGGTGGAGCGGCTGGGGGCTGGACAGTTCGGGGAGGTGTGGATGGGGTACTACAACG GGCACACGAAGGTGGCGGTGAAGAGCCTGAAGCAGGGCAGCATGTCCCCGGACGCCTTCCTGGCCGAGG CCAACCTCATGAAGCAGCTGCAACACCAGCGGCTGGTTCGGCTCTACGCTGTGGTCACCCAGGAGCCCAT CTACATCATCACTGAATACATGGAGAATGGGAGTCTAGTGGATTTTCTCAAGACCCCTTCAGGCATCAAGTTG ACCATCAACAAACTCCTGGACATGGCAGCCCAAATTGCAGAAGGCATGGCATTCATTGAAGAGCGGAATT ATATTCATCGTGACCTTCGGGCTGCCAACATTCTGGTGTCTGACACCCTGAGCTGCAAGATTGCAGACTT TGGCCTAGCACGCCTCATTGAGGACAACGAGTACACAGCCAGGGAGGGGGCCAAGTTTCCCATTAAGTGG ACAGCGCCAGAAGCCATTAACTACGGGACATTCACCATCAAGTCAGATGTGTGGTCTTTTGGGATCCTGC TGACGGAAATTGTCACCCACGGCCGCATCCCTTACCCAGGGATGACCAACCCGGAGGTGATTCAGAACCT GGAGCGAGGCTACCGCATGGTGCGCCCTGACAACTGTCCAGAGGAGCTGTACCAACTCATGAGGCTGTGC TGGAAGGAGCGCCCAGAGGACCGGCCCACCTTTGACTACCTGCGCAGTGTGCTGGAGGACTTCTTCACGG CCACAGAGGGCCAGTACCAGCCTCAGCCTTGAGAGGCCTTGAGAGGCCCTGGGGTTCTCCCCCTTTCTCT CCAGCCTGACTTGGGGAGATGGAGTTCTTGTGCCATAGTCACATGGCCTATGCACATATGGACTCTGCAC ATGAATCCCACCCACATGTGACACATATGCACCTTGTGTCTGTACACGTGTCCTGTAGTTGCGTGGACTC TGCACATGTCTTGTACATGTGTAGCCTGTGCATGTATGTCTTGGACACTGTACAAGGTACCCCTTTCTGG CTCTCCCATTTCCTGAGACCACAGAGAGAGGGGAGAAGCCTGGGATTGACAGAAGCTTCTGCCCACCTAC TTTTCTTTCCTCAGATCATCCAGAAGTTCCTCAAGGGCCAGGACTTTATCTAATACCTCTGTGTGCTCCT CCTTGGTGCCTGGCCTGGCACACATCAGGAGTTCAATAAATGTCTGTTGATGACTGTTGTA |

| | **C6 (NM_000065.5)** |
|---|---|
| 55 | AGGCTTCTGGGATATGACAGCATTGCCTTGTGTTAGCTAGCAATAAGAAAAGAAGCTTTGTTTGGATTAA CATATATACCCTCTTCATTCTGCATACCTATTTTTTCCCCAATAATTTGCAGCTTAGGTCCGAGGACACC ACAAACTCTGCTTAAAGGGCCTGGAGGCTCTCAAGGCATGGCCAGACGCTCTGTCTTGTACTTCATCCTG CTGAATGCTCTGATCAACAAGGGCCAAGCCTGCTTCTGTGATCACTATGCATGGACTCAGTGGACCAGCT GCTCAAAAACTTGCAATTCTGGAACCCAGAGCAGACACAGACAAATAGTAGTAGATAAGTACTACCAGGA AAACTTTTGTGAACAGATTTGCAGCAAGCAGGAGACTAGAGAATGTAACTGGCAAAGATGCCCCATCAAC TGCCTCCTGGGAGATTTTGGACCATGGTCAGACTGTGACCCTTGTATTGAAAAACAGTCTAAAGTTAGAT CTGTCTTGCGTCCCAGTCAGTTTGGGGGACAGCCATGCACTGCGCCTCTGGTAGCCTTTCAACCATGCAT TCCATCTAAGCTCTGCAAAATTGAAGAGGCTGACTGCAAGAATAAATTTCGCTGTGACAGTGGCCGCTGC ATTGCCAGAAAGTTAGAATGCAATGGAGAAAATGACTGTGGAGACAATTCAGATGAAAGGGACTGTGGGA GGACAAAGGCAGTATGCACACGGAAGTATAATCCCATCCCTAGTGTACAGTTGATGGGCAATGGGTTTCA TTTTCTGGCAGGAGAGCCCAGAGGAGAAGTCCTTGATAACTCTTTCACTGGAGGAATATGTAAAACTGTC AAAAGCAGTAGGACAAGTAATCCATACCGTGTTCCGGCCAATCTGGAAAATGTCGGCTTTGAGGTACAAA CTGCAGAAGATGACTTGAAAACAGATTTCTACAAGGATTTAACTTCTCTTGGACACAATGAAAATCAACA AGGCTCATTCTCAAGTCAGGGGGGGAGCTCTTTCAGTGTACCAATTTTTTATTCCTCAAAGAGAAGTGAA AATATCAACCATAATTCTGCCTTCAAACAAGCCATTCAAGCCTCTCACAAAAAGGATTCTAGTTTTATTA GGATCCATAAAGTGATGAAAGTCTTAAACTTCACAACGAAAGCTAAAGATCTGCACCTTTCTGATGTCTT TTTGAAAGCACTTAACCATCTGCCTCTAGAATACAACTCTGCTTTGTACAGCCGAATATTCGATGACTTT GGGACTCATTACTTCACCTCTGGCTCCCTGGGAGGCGTGTATGACCTTCTCTATCAGTTTAGCAGTGAGG AACTAAAGAACTCAGGTTTAACCGAGGAAGAAGCCAAACACTGTGTCAGGATTGAAACAAAGAAACGCGT TTTATTTGCTAAGAAAACAAAAGTGGAACATAGGTGCACCACCAACAAGCTGTCAGAGAAACATGAAGGT TCATTTATACAGGGAGCAGAGAAATCCATATCCCTGATTCGAGGTGGAAGGAGTGAATATGGAGCAGCTT TGGCATGGGAGAAAGGGAGCTCTGGTCTGGAGGAGAAGACATTTTCTGAGTGGTTAGAATCAGTGAAGGA AAATCCTGCTGTGATTGACTTTGAGCTTGCCCCCATCGTGGACTTGGTAAGAAACATCCCCTGTGCAGTG ACAAAACGGAACAACCTCAGGAAAGCTTTGCAAGAGTATGCAGCCAAGTTCGATCCTTGCCAGTGTGCTC CATGCCCTAATAATGGCCGACCCACCCTCTCAGGGACTGAATGTCTGTGTGTGTGTCAGAGTGGCACCTA TGGTGAGAACTGTGAGAAACAGTCTCCAGATTATAAATCCAATGCAGTAGACGGACAGTGGGGTTGTTGG TCTTCCTGGAGTACCTGTGATGCTACTTATAAGAGATCGAGAACCCGAGAATGCAATAATCCTGCCCCCC AACGAGGAGGGAAACGCTGTGAGGGGGAGAAGCGACAAGAGGAAGACTGCACATTTTCAATCATGGAAAA CAATGGACAACCATGTATCAATGATGACGAAGAAATGAAAGAGGTCGATCTTCCTGAGATAGAAGCAGAT TCCGGGTGTCCTCAGCCAGTTCCTCCAGAAAATGGATTTATCCGGAATGAAAAGCAACTATACTTGGTTG GAGAAGATGTTGAAATTTCATGCCTTACTGGCTTTGAAACTGTTGGATACCAGTACTTCAGATGCTTACC AGACGGGACCTGGAGACAAGGGGATGTGGAATGCCAACGGACGGAGTGCATCAAGCCAGTTGTGCAGGAA GTCCTGACAATTACACCATTTCAGAGATTGTATAGAATTGGTGAATCCATTGAGCTAACTTGCCCCAAAG GCTTTGTTGTTGCTGGGCCATCAAGGTACACATGCCAGGGGAATTCCTGGACACCACCCATTTCAAACTC TCTCACCTGTGAAAAAGATACTCTAACAAAATTAAAAGGCCATTGTCAGCTGGGACAGAAACAATCAGGA TCTGAATGCATTTGTATGTCTCCAGAAGAAGACTGTAGCCATCATTCAGAAGATCTCTGTGTGTTTGACA CAGACTCCAACGATTACTTTACTTCACCCGCTTGTAAGTTTTTGGCTGAGAAATGTTTAAATAATCAGCA ACTCCATTTTCTACATATTGGTTCCTGCCAAGACGGCCGCCAGTTAGAATGGGGTCTTGAAAGGACAAGA CTTTCATCCAACAGCACAAAGAAAGAATCCTGTGGCTATGACACCTGCTATGACTGGGAAAAATGTTCAG CCTCCACTTCCAAATGTGTCTGCCTATTGCCCCCACAGTGCTTCAAGGGTGGAAACCAACTCTACTGTGT CAAAATGGGATCATCAACAAGTGAGAAAACATTGAACATCTGTGAAGTGGGAACTATAAGATGTGCAAAC AGGAAGATGGAAATACTGCATCCTGGAAAGTGTTTGGCCTAGCACAATTACTGCTAGGCCCAGCACAATG AACAGATTTACCATCCCGAAGAACCAACTCCTACAAATGAGAATTCTTGCACAAACAGCAGACTGGCATG |
| | CTCAAAGTTACTGACAAAAATTATTTTCTGTTAGTTTGAGATCATTATTCTCCCCTGACTCTCCTGTTTG GGCATGTCTTATTCAGTTCCAGCTCATGACGCCCTGTAGCATACCCCTAGGTACCAACTTCCACAGCAGT CTCGTAAATTCTCCTGTTCACATTGTACAAAAATAATGTGACTTCTGAGGCCCTTATGTAGCCTGTGACA TTAAGCATTCTCGCAATTAGAAATAAGAATAAAACCCATAATTTTCTTCAATGAGTTAATAAACAGAAAT CTCCAGAACCTCTGAAACACATTCTTGAAGCCCAGCTTTCATATCTTCATTCAACAAATAATTTCTGAGT GTGTATACAGGATGTCAAGTACTGACCAAAGTCCTGAGAACTCGGCAGATAATAAAACAGACAAAAGCCT TTGCCTTCATGAAGCATACATTCATTCAGGGGTAGACACACAAAAAATGAAATAAACAGGTAAAATATGT AGCATGTTTGATGGTGATAATTGTTTTGGAGAAAATACAACACGGAAGGGTGAGTCCCATCCTTAATTAT CATCTTATGCAAGGAAATATTCCATAACAATAAACATCGCTGTAAGGATGA |

| | **CLEC4A (NM_194448.3)** |
|---|---|
| 56 | GCATTTGTTTCAAGGCTGTGATTCTCACTATACTGGTCCTGAGGAAAGGGCTTCTGTGAACTGCGGTTTTTAGTT TTTATTGTGGTTCTTAGTTCTCATGAGACCCCTCTTGAGGATATGTGCCTATCTGGTGCCTCTGCTCTCCACTAG TTGAGTGAAAGGAAGGAGGTAATTTACCACCATGTTTGGTTCCTGTTTATAAGATGTTTTAAGAAAGATCTGAAAC AGATTTTCTGAAGAAAGCAGAAGCTCTCTTCCCATTATGACTTCGGAAATCACTTATGCTGAAGTGAGGTTCAAA AATGAATTCAAGTCCTCAGGCATCAACACAGCCTCTTCTGCAGAGACAGCCTGGAGCTGTTGCCCAAAGAATT GGAAGTCATTTAGTTCCAACTGCTACTTTATTTCTACTGAATCAGCATCTTGGCAAGACAGTGAGAAGGACTGTG CTAGAATGGAGGCTCACCTGCTGGTGATAAACACTCAAGAAGAGCAGGATTTCATCTTCCAGAATCTGCAAGA AGAATCTGCTTATTTTGTGGGGCTCTCAGATCCAGAAGGTCAGCGACATTGGCAATGGGTTGATCAGACACCA TACAATGAAAGTTCCACATTCTGGCATCCACGTGAGCCCAGTGATCCCAATGAGCGCTGCGTTGTGCTAAATT TTCGTAAATCACCCAAAAGATGGGGCTGGAATGATGTTAATTGTCTTGGTCCTCAAAGGTCAGTTTGTGAGATGA TGAAGATCCACTTATGAACTGAACATTCTCCATGAACAGGTGGTTGGATTGGTATCTGTCATTGTAGGGATAGAT AATAAGCTCTTCTTATTCATGTGTAAGGGAGGTCCATAGAATTTAGGTGGTCTGTCAACTATTCTACTTATGAGAG AATTGGTCTGTACATTGACTGATTCACTTTTTCATAAAGTGAGCATTTATTGAGCATTTTTTCATGTGCCAGAGCCT GTACTGGAGGCCCCCATTGTGCACACATGGAGAGAACATGAGTCTCTCTTAATTTTTATCTGGTTGCTAAAGAA TTATTTACCAATAAAATTATATGATGTGGTGTCTC |

| | **IL13RA1 (NM_001560.3)** |
|---|---|
| 57 | CCAGCCCGGCCGGGCTCCGAGGCGAGAGGCTGCATGGAGTGGCCGGCGCGGCTCTGCGGGCTGTGGG CGCTGCTGCTCTGCGCCGGCGGCGGGGGCGGGGGCGGGGGCGCCGCGCCTACGGAAACTCAGCCACC TGTGACAAATTTGAGTGTCTCTGTTGAAAACCTCTGCACAGTAATATGGACATGGAATCCACCCGAGGGAGCC AGCTCAAATTGTAGTCTATGGTATTTTAGTCATTTTGGCGACAAACAAGATAAGAAAATAGCTCCGGAAACTCGT CGTTCAATAGAAGTACCCCTGAATGAGAGGATTTGTCTGCAAGTGGGGTCCCAGTGTAGCACCAATGAGAGTG AGAAGCCTAGCATTTTGGTTGAAAAATGCATCTCACCCCCAGAAGGTGATCCTGAGTCTGCTGTGACTGAGCT TCAATGCATTTGGCACAACCTGAGCTACATGAAGTGTTCTTGGCTCCCTGGAAGGAATACCAGTCCCGACACT AACTATACTCTCTACTATTGGCACAGAAGCCTGGAAAAAATTCATCAATGTGAAAACATCTTTAGAGAAGGCCAA TACTTTGGTTGTTCCTTTGATCTGACCAAAGTGAAGGATTCCAGTTTTGAACAACACAGTGTCCAAATAATGGTC AAGGATAATGCAGGAAAAATTAAACCATCCTTCAATATAGTGCCTTTAACTTCCCGTGTGAAACCTGATCCTCCA CATATTAAAAACCTCTCCTTCCACAATGATGACCTATATGTGCAATGGGAGAATCCACAGAATTTTATTAGCAGA TGCCTATTTTATGAAGTAGAAGTCAATAACAGCCAAACTGAGACACATAATGTTTTCTACGTCCAAGAGGCTAAA TGTGAGAATCCAGAATTTGAGAGAAATGTGGAGAATACATCTTGTTTCATGGTCCCTGGTGTTCTTCCTGATACTT TGAACACAGTCAGAATAAGAGTCAAAACAAATAAGTTATGCTATGAGGATGACAAACTCTGGAGTAATTGGAGC CAAGAAATGAGTATAGGTAAGAAGCGCAATTCCACACTCTACATAACCATGTTACTCATTGTTCCAGTCATCGT CGCAGGTGCAATCATAGTACTCCTGCTTTACCTAAAAAGGCTCAAGATTATTATATTCCCTCCAATTCCTGATCC TGGCAAGATTTTTAAAGAAATGTTTGGAGACCAGAATGATGATACTCTGCACTGGAAGAAGTACGACATCTATGA GAAGCAAACCAAGGAGGAAACCGACTCTGTAGTGCTGATAGAAAACCTGAAGAAAGCCTCTCAGTGATGGAG ATAATTTATTTTTACCTTCACTGTGACCTTGAGAAGATTCTTCCCATTCTCCATTTGTTATCTGGGAACTTATTAAAT GGAAACTGAAACTACTGCACCATTTAAAAACAGGCAGCTCATAAGAGCCACAGGTCTTTATGTTGAGTCGCGC ACCGAAAAACTAAAAATAATGGGCGCTTTGGAGAAGAGTGTGGAGTCATTCTCATTGAATTATAAAAGCCAGCA GGCTTCAAACTAGGGGACAAAGCAAAAAGTGATGATAGTGGTGGAGTTAATCTTATCAAGAGTTGTGACAACTT CCTGAGGGATCTATACTTGCTTTGTGTTCTTTGTGTCAACATGAACAAATTTTATTTGTAGGGGAACTCATTTGGG GTGCAAATGCTAATGTCAAACTTGAGTCACAAAGAACATGTAGAAAACAAAATGGATAAAATCTGATATGTATTGT TTGGGATCCTATTGAACCATGTTTGTGGCTATTAAAACTCTTTTAACAGTCTGGGCTGGGTCCGGTGGCTCACG CCTGTAATCCCAGCAATTTGGGAGTCCGAGGCGGGCGGATCACTCGAGGTCAGGAGTTCCAGACCAGCCTG ACCAAAATGGTGAAACCTCCTCTCTACTAAAACTACAAAAATTAACTGGGTGTGGTGGCGCGTGCCTGTAATC CCAGCTACTCGGGAAGCTGAGGCAGGTGAATTGTTTGAACCTGGGAGGTGGAGGTTGCAGTGAGCAGAGAT CACACCACTGCACTCTAGCCTGGGTGACAGAGCAAGACTCTGTCTAAAAAACAAAACAAAACAAAACAAAAC AAAAAAACCTCTTAATATTCTGGAGTCATCATTCCCTTCGACAGCATTTTCCTCTGCTTTGAAAGCCCCAGAAAT CAGTGTTGGCCATGATGACAACTACAGAAAAACCAGAGGCAGCTTCTTTGCCAAGACCTTTCAAAGCCATTTTA GGCTGTTAGGGGCAGTGGAGGTAGAATGACTCCTTGGGTATTAGAGTTTCAACCATGAAGTCTCTAACAATGTA TTTTCTTCACCTCTGCTACTCAAGTAGCATTTACTGTGTCTTTGGTTTGTGCTAGGCCCCCGGGTGTGAAGCACA GACCCCTTCCAGGGGTTTACAGTCTATTTGAGACTCCTCAGTTCTTGCCACTTTTTTTTTTAATCTCCACCAGTCA TTTTTCAGACCTTTTAACTCCTCAATTCCAACACTGATTTCCCCTTTTGCATTCTCCCTCCTTCCCTTCCTTGTAG CCTTTTGACTTTCATTGGAAATTAGGATGTAAATCTGCTCAGGAGACCTGGAGGAGCAGAGGATAATTAGCATC TCAGGTTAAGTGTGAGTAATCTGAGAAACAATGACTAATTCTTGCATATTTTGTAACTTCCATGTGAGGGTTTTCA |
| | GCATTGATATTTGTGCATTTTCTAAACAGAGATGAGGTGGTATCTTCACGTAGAACATTGGTATTCGCTTGAGAAA AAAAGAATAGTTGAACCTATTTCTCTTTCTTTACAAGATGGGTCCAGGATTCCTCTTTTCTCTGCCATAAATGATTA ATTAAATAGCTTTTGTGTCTTACATTGGTAGCCAGCCAGCCAAGGCTCTGTTTATGCTTTTGGGGGGCATATATT GGGTTCCATTCTCACCTATCCACACAACATATCCGTATATATCCCCTCTACTCTTACTTCCCCCAAATTTAAAGA AGTATGGGAAATGAGAGGCATTTCCCCCACCCCATTTCTCTCCTCACACACAGACTCATATTACTGGTAGGAA CTTGAGAACTTTATTTCCAAGTTGTTCAAACATTTACCAATCATATTAATACAATGATGCTATTTGCAATTCCTGCT CCTAGGGGAGGGGAGATAAGAAACCCTCACTCTCTACAGGTTTGGGTACAAGTGGCAACCTGCTTCCATGGC CGTGTAGAAGCATGGTGCCCTGGCTTCTCTGAGGAAGCTGGGGTTCATGACAATGGCAGATGTAAAGTTATTC TTGAAGTCAGATTGAGGCTGGGAGACAGCCGTAGTAGATGTTCTACTTTGTTCTGCTGTTCTCTAGAAAGAATAT TTGGTTTTCCTGTATAGGAATGAGATTAATTCCTTTCCAGGTATTTTATAATTCTGGGAAGCAAAACCCATGCCTC CCCCTAGCCATTTTTACTGTTATCCTATTTAGATGGCCATGAAGAGGATGCTGTGAAATTCCCAACAAACATTGA TGCTGACAGTCATGCAGTCTGGGAGTGGGGAAGTGATCTTTTGTTCCCATCCTCTTCTTTTAGCAGTAAAATAG CTGAGGGAAAAGGGAGGGAAAAGGAAGTTATGGGAATACCTGTGGTGGTTGTGATCCCTAGGTCTTGGGAGC TCTTGGAGGTGTCTGTATCAGTGGATTTCCCATCCCCTGTGGGAAATTAGTAGGCTCATTTACTGTTTTAGGTCT AGCCTATGTGGATTTTTTCCTAACATACCTAAGCAAACCCAGTGTCAGGATGGTAATTCTTATTCTTTCGTTCAGT TAAGTTTTTCCCTTCATCTGGGCACTGAAGGGATATGTGAAACAATGTTAACATTTTTGGTAGTCTTCAACCAGG GATTGTTTCTGTTTAACTTCTTATAGGAAAGCTTGAGTAAAATAAATATTGTCTTTTTGTATGTCA |

| | **IL17RA (NM_014339.7)** |
|---|---|
| 58 | GACTCGAACTCCACCGCGGAAAAGAAAGCCTCAGAACGTTCGTTCGCTGCGTCCCCAGCCGGGGCCGAGC CCTCCGCGACGCCAGCCGGGCCATGGGGGCCGCACGCAGCCCGCCGTCCGCTGTCCCGGGGCCCCTG CTGGGGCTGCTCCTGCTGCTCCTGGGCGTGCTGGCCCCGGGTGGCGCCTCCCTGCGACTCCTGGACCAC CGGGCGCTGGTCTGCTCCCAGCCGGGGCTAAACTGCACGGTCAAGAATAGTACCTGCCTGGATGACAGCT GGATTCACCCTCGAAACCTGACCCCCTCCTCCCCAAAGGACCTGCAGATCCAGCTGCACTTTGCCCACACC CAACAAGGAGACCTGTTCCCCGTGGCTCACATCGAATGGACACTGCAGACAGACGCCAGCATCCTGTACCT CGAGGGTGCAGAGTTATCTGTCCTGCAGCTGAACACCAATGAACGTTTGTGCGTCAGGTTTGAGTTTCTGTCC AAACTGAGGCATCACCACAGGCGGTGGCGTTTTACCTTCAGCCACTTTGTGGTTGACCCTGACCAGGAATAT GAGGTGACCGTTCACCACCTGCCCAAGCCCATCCCTGATGGGGACCCAAACCACCAGTCCAAGAATTTCCT TGTGCCTGACTGTGAGCACGCCAGGATGAAGGTAACCACGCCATGCATGAGCTCAGGCAGCCTGTGGGAC CCCAACATCACCGTGGAGACCCTGGAGGCCCACCAGCTGCGTGTGAGCTTCACCCTGTGGAACGAATCTA CCCATTACCAGATCCTGCTGACCAGTTTTCCGCACATGGAGAACCACAGTTGCTTTGAGCACATGCACCACAT ACCTGCGCCCAGACCAGAAGAGTTCCACCAGCGATCCAACGTCACACTCACTCTACGCAACCTTAAAGGGT GCTGTCGCCACCAAGTGCAGATCCAGCCCTTCTTCAGCAGCTGCCTCAATGACTGCCTCAGACACTCCGCG ACTGTTTCCTGCCCAGAAATGCCAGACACTCCAGAACCAATTCCGGACTACATGCCCCTGTGGGTGTACTGG TTCATCACGGGCATCTCCATCCTGCTGGTGGGCTCCGTCATCCTGCTCATCGTCTGCATGACCTGGAGGCTA GCTGGGCCTGGAAGTGAAAAATACAGTGATGACACCAAATACACCGATGGCCTGCCTGCGGCTGACCTGAT CCCCCCACCGCTGAAGCCCAGGAAGGTCTGGATCATCTACTCAGCCGACCACCCCCTCTACGTGGACGTG GTCCTGAAATTCGCCCAGTTCCTGCTCACCGCCTGCGGCACGGAAGTGGCCCTGGACCTGCTGGAAGAGC AGGCCATCTCGGAGGCAGGAGTCATGACCTGGGTGGGCCGTCAGAAGCAGGAGATGGTGGAGAGCAACTC TAAGATCATCGTCCTGTGCTCCCGCGGCACGCGCGCCAAGTGGCAGGCGCTCCTGGGCCGGGGGGCGC CTGTGCGGCTGCGCTGCGACCACGGAAAGCCCGTGGGGGACCTGTTCACTGCAGCCATGAACATGATCCT CCCGGACTTCAAGAGGCCAGCCTGCTTCGGCACCTACGTAGTCTGCTACTTCAGCGAGGTCAGCTGTGACG GCGACGTCCCCGACCTGTTCGGCGCGGCGCCGCGGTACCCGCTCATGGACAGGTTCGAGGAGGTGTACTT CCGCATCCAGGACCTGGAGATGTTCCAGCCGGGCCGCATGCACCGCGTAGGGGAGCTGTCGGGGGACAA CTACCTGCGGAGCCCGGGCGGCAGGCAGCTCCGCGCCGCCCTGGACAGGTTCCGGGACTGGCAGGTCC GCTGTCCCGACTGGTTCGAATGTGAGAACCTCTACTCAGCAGATGACCAGGATGCCCCGTCCCTGGACGAA GAGGTGTTTGAGGAGCCACTGCTGCCTCCGGGAACCGGCATCGTGAAGCGGGCGCCCCTGGTGCGCGAG CCTGGCTCCCAGGCCTGCCTGGCCATAGACCCGCTGGTCGGGGAGGAAGGAGGAGCAGCAGTGGCAAAG CTGGAACCTCACCTGCAGCCCCGGGGTCAGCCAGCGCCGCAGCCCCTCCACACCCTGGTGCTCGCCGC AGAGGAGGGGGCCCTGGTGGCCGCGGTGGAGCCTGGGCCCCTGGCTGACGGTGCCGCAGTCCGGCTGG CACTGGCGGGGGAGGGCGAGGCCTGCCCGCTGCTGGGCAGCCCGGGCGCTGGGCGAAATAGCGTCCTC TTCCTCCCCGTGGACCCCGAGGACTCGCCCCTTGGCAGCAGCACCCCCATGGCGTCTCCTGACCTCCTTC CAGAGGACGTGAGGGAGCACCTCGAAGGCTTGATGCTCTCGCTCTTCGAGCAGAGTCTGAGCTGCCAGGC CCAGGGGGGCTGCAGTAGACCCGCCATGGTCCTCACAGACCCACACACGCCCTACGAGGAGGAGCAGCG GCAGTCAGTGCAGTCTGACCAGGGCTACATCTCCAGGAGCTCCCCGCAGCCCCCCGAGGGACTCACGGA AATGGAGGAAGAGGAGGAAGAGGAGCAGGACCCAGGGAAGCCGGCCCTGCCACTCTCTCCCGAGGACCT GGAGAGCCTGAGGAGCCTCCAGCGGCAGCTGCTTTTCCGCCAGCTGCAGAAGAACTCGGGCTGGGACAC GATGGGGTCAGAGTCAGAGGGGCCCAGTGCATGAGGGCGGCTCCCCAGGGACCGCCCAGATCCCAGCTT TGAGAGAGGAGTGTGTGTGCACGTATTCATCTGTGTGTACATGTCTGCATGTGTATATGTTCGTGTGTGAAATGT AGGCTTTAAAATGTAAATGTCTGGATTTTAATCCCAGGCATCCCTCCTAACTTTTCTTTGTGCAGCGGTCTGGTTA TCGTCTATCCCCAGGGGAATCCACACAGCCCGCTCCCAGGAGCTAATGGTAGAGCGTCCTTGAGGCTCCAT TATTCGTTCATTCAGCATTTATTGTGCACCTACTATGTGGCGGGCATTTGGGATACCAAGATAAATTGCATGCGG CATGGCCCCAGCCATGAAGGAACTTAACCGCTAGTGCCGAGGACACGTTAAACGAACAGGATGGGCCGGG CACGGTGGCTCACGCCTGTAATCCCAGCACACTGGGAGGCCGAGGCAGGTGGATCACTCTGAGGTCAGGA GTTTGAGCCAGCCTGGCCAACATGGTGAAACCCCATCTCCACTAAAAATAGAAAAATTAGCCGGGCATGGTG |
| | ACACATGCCTGTAGTCCTAGCTACTTGGGAGGCTGAGGCAGGAGAATTGCTTGAATCTGGGAGGCAGAGGTT GCAGTGAGCCGAGATTGTGCCATTGCACTGCAGCCTGGATGACAGAGCGAGACTCTATCTCAAAAAAAAAAA AAAAAAAAGATGGTCACGCGGGATGTAAACGCTGAATGGGCCAGGTGCAGTGGCTCATGCTTGTAATCCCAG CACTTTGGGAAGGCGAGGCAGGTGGATTGCTTGAGCTCAGGAGTTCAAGACCAGCCTGGGCGACATAGTGA GACCTCATCTCTACCTAAATTTTTTTTTAGTCAGTCATGGTGGCACATGCCTGTAGTCCCAGCTACTCGGGAGG CTGATGCCAGATGATCACTTGAGCCCAGGAGGTAGAGGCTGCAGTGAGCTATAATGGTACCATTGCAATCCA GCCTGGGCAGCAGAGTGAGACCCTGTCTCAAAAAAAATAAAAAAGTAGAAAGATGGAGTGGAAGCCTGCCCA GGGTTGTGAGCATGCACGGGAAAGGCACCCAGGTCAGGGGGGATCCCCGAGGAGATGCCTGAGCTGAAG GATTGTGGTTGGGGAAAGCGTAGTCCCAGCAAGGAAGCAGTTTGTGGGTAAGTGCTGGGAGGTGAGTGGAGT GAGCTTGTCAGGGAGCTGCTGGTGGAGCCTGGAGGGGAAGGAGGGAGGCAGTGAGAGAGATCGGGGTGG GGGGTGGGGGGATGTCGCCAGAGCTCAGGGGTGGGGACAGCCTTGTGCGCATCAGTCCTGAGGCCTGGG GCACCTTTCGTCTGATGAGCCTCTGCATGGAGAGAGGCTGAGGGCTAAACACAGCTGGATGTCACCTGAGTT CATTTATAGGAAGAGAGAAATGTCGAGGTGAAACGTAAAAGCATCTGGCAGGAAGGTGAGTCTGAAGCCCTG CACCCGCGTTCCGACTATCAGTGGGGAGCTGTTAGCACGTAGGATTCTTCAGAGCAGCTGGGCTGGAGCTC CCCTGAGCTCAGGAAGCCCCAGGGTGCAAGGGCAAGGAAATGAGGGGTGGTGGGTCAGTGAAGATCTGGG CAGACCTTGTGTGGGGAAGGGGTGCTGCTGTGACTTCAGGGTCTGAGGTCCAAAGACAGCATTTGAAAAGAG GCTCTGAAGCCAGTGTTTGAAGAATTTGTTCCTGAAGTACCTCCTGGGGGTAGGCTAGAGGCTTCTGGCTTCA GGGTCCTGAAGAACACATTGAGGTGCCGTCTGACACTGGAATAGGGTGCCCTTCATTCCTATGCCTGAGTCC TTAACTATATTTCCAACCTCCAGTGAGGAGGAGAAGATTCGGAAATGTGACAGGAGAGCAAACAGGACAGTTT GCATGTGTGTGTGCGCACACATACATGTGCGTGAAAGATTATCAATAAAAGTGCATAAATTTGTTGATCTGGTAA GAGTTTCTAGCAGGAAGGTCGAGCCACTTACTGTAGGTCAAGAAGTTGCTAGTTGCGGAGTTTTTTCTTGCAGT TAGACTTTACCTAGTGGTAGCAGGGCCACCAAAGCTCTGTGTCCCAGATGGTGTATGGCCCATAATCCACCC AACAGCAGCAAAGGACCAGGCAAAGGAGAACAGGAGCAGAAGCCTCCCAGCCACTAGCCTTTTGGGCTCA GTCTCTCCAATAATCCTGGAGAGGGGCTTCGTTGGGTCTGGACACCTACCATGCATTCTGTGACCTTTCCCTA GCTTCCAATAAATAACTGTTTGACGCCCAGAGTACAGGATACCACAATGCACTCTTCCTGCGTAGAGCACATG TTCCCATCTGCTCCCATTCCTCAGGAACCTTGAATTCTAGCTCTGCTGGCCTTTGAGCCCATGCCAGTAAATGT CCTGATGGGCATTGCCTACTATCTCCAGGGCAGCTGCCTTTGTCCTCCTAACAGCTTTATTGGAGTACAGTTCA CTTACCATACAATCCACAATTGACCCTGCACAATTTGATGCCGGTTTAGTATAGTCACAGTTCAGCAGCCATCA GCACAGTCAGTCTTAGAGTTTACTACCCCCAAAAGAAATCCAGCCCCCCTTAGTCACCACCCCAACCTCCCC ATCCCTAGGCACCCCTAGGCTACTTTGATCTCTGTAGACTTGCCTCTTCTGGACATGACATAGAGAAAGGAGT CATAAATTCTCCAAGGTGTCTGTTTCTTCTTTAATGTCATTCCCTGTTTCTCCTCACATTCCCTCCCCATTTCCTG GGCCCAGTCTCACACTGGTCCTTGCTTACCCTAAATGCTATTAATTCCATCACTCTGAGTATGGTGTTTGCTGTC CGCTGAATGCCAAGAGCTTCAAGAGTGTGTGTAAATAAAGCCACACCTTTATTTTTGTATTATTCTGAACCATGG CTAATAAATTGTTTCACCAAGAAATGTCTCTCTAAGAACAGGTGCCCTCCACGCTGTGCCCCTCCCACCTCTT CAGCTCGTCTCCTGAGTGTGCAGAGGTGGTTCCGGTTGGGAAAGAAGCAGCGGAGCATCTAACCATGCCTG TGTCCAGGCCGATTATGCACGCAGCCACCAACAAGCTCCCAACTCCCGCGTAGAGTTTCATGACTTTTTCCT GCCTACTATCTTGATCCTAGTTTTTTTTTTGTTTTTTTTTTTTTTAAGGAATAATTACTTTGATTCAAAACCAGTTTCTCT TTTCTGCATAGGAAGGTCCTTGAAGGTGTTTAGGGTCTAAAAAGGGTGGTGTTCGGTCTCTGAAACATCCATTC AGCAGTTTGAGCTGGGATCTCTGAATGCAAGGGTATGATGGATATACTTCTTTCTTGCTTTTGTTGTGTTTTGGTTT TTTGTTTGTTTTTAAGTCAGGGTCTCTCTGTCACCAGGCTGTATTACAGTGGTGCAATCATGGCTCACTGCAGCC TCGACCTCCCAGGCTCAAGCCATCTTTCCACCTCAGCCTGCCAGTGGCTAGAACTACAGGCGTGCACCACT GTGCCCGGCTAATTTGTGTGTATATATTTTGTAGAAATGGGGTTTCACCATGTTGTCCAGGCTGGTCACGAACTC CTGGGCTCAAGCCATCTGCCCGCCTCATCCTCCCAAAGTGCTGGGATTATAGGCCTGAGCCCACCGTGCCT GGCCTTTCCTGTTTATCTTTGAAAATTAAATAGGGCATAAGAGAGAAGAAGATGTACTTACAATGCAGTGGGTGG TTTTAACTCTATAGCCTTTGGGCTCTGTGGTTGGTGCTCCCCTTCCTAAATAAATGAGGTGTATGCAGGGCCCT CTTCTGCCTTAGCGCCCTGCCAGCTGGGACTCCAGCAAGGCCCGGGGCACCTGAGGACAGAGTGAGATGG AGGGCCGCTGCTCCAGCAGCCGGGCCTGCATCCCACAAGTCAACTGTGTCGGACAGAGGATCCTTACAAA GAAGAGGCAGCAGGGTTGGGGGCTGGCCAGCTGCTCGTCCGCCCTAGGTAGCTTGCTCATCTGTAAAGTGG GTGGGGCAGGAGTTCCCACCTCATGGGGTCCTGGCAAGCCTGCAGTATCCCCGAGTGGCACCAGCCTGCT TCTGGGGCAGAGCAGTTTGTGCCCCCTGAGGTACCACTGATCCTCTTTCCCTGCTATTAGGTATTGCTCTCTTC CTCCGGTGTTTGCCTTTTCAGATTATAGAAGTAATATGTGTTCCCATATTTGGCGTCTCTCAGGAGCTCAGGAAG TACTTGGCTGAGTGAACATGTCCATTGTGGAAAAATGGCAACAATATGGATTCCATGGGTATATTTTATAGAAGA ATATGAAGAAAAGCAGCTACCCCTAAACCCATTGCACAAGCTGTTCATGTTAATTCTGTACCCGACGCTTTCCC CACGGGGCCTCCCCTCACTCTGAAATGGCATCCAGGTCCATCTTGCCCTCCACCTCTGCATGGCTCTCCAT GCCCCATCGCCTCTCCCAGATCCTAGCACTGGGTCCACACTCTCGCCCTGTCCATTTAGGTTGATGAAAGCA GGCAGTCACCCGGGTGGGCCAGTCTTGCCTGTGGGAGGAACATGCAGTCTCCTGTCTCATGGTTTGAAGTGT GCCAGGAAGCCTGGCCCAGCCCACCTCCCCCTGGAGTCCTTCCCAGGAGGAATAACCCCTTAGGTCATTG ACTATAAGATGAGTTCGCTCACTGGATCCTTCCTCTCTGATGAGACAGGAAGAAGGTACACAGTGACCAGGTA GGAGGAGGAGAGGGAGTAGAAAGGAGGGATGCGGGTGGCTGGTCCCTGCATTTGCCTGCTTCCCTGCACG GGTGTCCCACTGGCCGCCTCTGCTCACCAGTGTCATGGGATTCTCTCAGAAGATGAAAACAGCCCCTGCTTT TTTGCTAGAATGGCTGAGCTTTCATGGAAAGGAAGCTGGACCCAAGCAACAGCCGACTACCGAAGGTTGCCT GGAGCAGTGCAGATGTGGGAGGAAGAAGGGCCTTGGTGCACACTGGCTTTTCTTCCTGACTGCAATGTGGCA TTGTGCCAGCTACCTCCTCTTTCTCGGCCTCAGGAAAATGGAGAGAAAGCAGCCCTGAAGGTGGCTGTGACG AGGGAAGGGGCAGAGGGCCTGACAGTCAACCACGCGCTATATTTTCCTGTTCTTCCTTAGGGCAAGAACTGC |
| | ATGGCCAGACTCAGGCAAGGCCTAGGTGTGGGCTGGGCATTGCCTACACGTGAAGAGATCACTCCGCGTCC CTACTGCACCTGTCACAAAGTGCCTTCTGATATGCCTGGCAAACCAAAATCGGTGAGCGCCAGCTTGCTTCC CTAGAAGACATTTCTAAATATTCATAACATGCTTGCTCAAATCAATCACCTTATTTTACATCCGCTCCAGGGAGAA ATGAAGACATGGTCCTACGTTGTTCTGTAATTATTTTCTATGTAAATTTTGTTCCTTGTTACAATTATATATGTCTTAG GGGAAAGGACCATTTCACATGTGTCACCTCATGTGATTCTCACCACAGCCCTGTGATTGCTCCTGTTTTATAAAT AATGACATAGTTCCAGTTGATGGCCAAAGCCACAGCTAACGAGAGGCAGAGAGAGCTCAGGCTCCCAGGAG CTTCCACTCTCAGACCTTGCCTCCCGGGCTGCCCTGAGTGAAACGCCTGCTTAGCATTTGGCACAGCCAGA AGCAGCAAGCTAGGGTCACAACACAGAGAGGGGCTGTGTAATACTGGCTGCCTCTGTGCTAAGAAAAAAAAA AAATCACTGTGTGTTTGTTTATTTTGGTGCAGGCCCAGTGTTCTTGCTTAGACTTAATACTACCCTTCATGTTAAAA TAAAACCAAACAAAAACCCA |

| | **GZMH (NM_033423.5)** |
|---|---|
| 59 | ATCTCCCTCCAGCATTTGTTTCTGGAAGGAGTCAACACCAACAGCTCTGACCTGGGCAGCCTTCCTGAGAAAA TGCAGCCATTCCTCCTCCTGTTGGCCTTTCTTCTGACCCCTGGGGCTGGGACAGAGGAGATCATCGGGGGC CATGAGGCCAAGCCCCACTCCCGCCCCTACATGGCCTTTGTTCAGTTTCTGCAAGAGAAGAGTCGGAAGAG GTGTGGCGGCATCCTAGTGAGAAAGGACTTTGTGCTGACAGCTGCTCACTGCCAGGGAAGCTCCATAAATGT CACCTTGGGGGCCCACAATATCAAGGAACAGGAGCGGACCCAGCAGTTTATCCCTGTGAAAAGACCCATCC CCCATCCAGCCTATAATCCTAAGAACTTCTCCAACGACATCATGCTACTGCAGCTGGAGAGAAAGGCCAAGT GGACCACAGCTGTGCGGCCTCTCAGGCTACCTAGCAGCAAGGCCCAGGTGAAGCCAGGGCAGCTGTGCA GTGTGGCTGGCTGGGGTTATGTCTCAATGAGCACTTTAGCAACCACACTGCAGGAAGTGTTGCTGACAGTGCA GAAGGACTGCCAGTGTGAACGTCTCTTCCATGGCAATTACAGCAGAGCCACTGAGATTTGTGTGGGGGATCC AAAGAAGACACAGACCGGTTTCAAGGGGGACTCCGGGGGGCCCCTCGTGTGTAAGGACGTAGCCCAAGGT ATTCTCTCCTATGGAAACAAAAAAGGGACACCTCCAGGAGTCTACATCAAGGTCTCACACTTCCTGCCCTGGA TAAAGAGAACAATGAAGCGCCTCTAACAGCAGGCATGAGACTAACCTTCCTCTGGGCCTGACCATCTCTGGG ACAGAGGCAAGAATCCCCAAGGGGTGGGCAGTCGGGGTTGCAGGACTGTAATAAATGGATCTCTGGTGTAAA TATGA |

| | **PDGFRB (NM_002609.4)** |
|---|---|
| 60 | AGCAGCCAGCAGTGACTGCCCGCCCTATCTGGGACCCAGGATCGCTCTGTGAGCAACTTGGAGCCAGAGA GGAGATCAACAAGGAGGAGGAGAGAGCCGGCCCCTCAGCCCTGCTGCCCAGCAGCAGCCTGTGCTCGCC CTGCCCAACGCAGACAGCCAGACCCAGGGCGGCCCCTCTGGCGGCTCTGCTCCTCCCGAAGGATGCTTG GGGAGTGAGGCGAAGCTGGGCCGCTCCTCTCCCCTACAGCAGCCCCCTTCCTCCATCCCTCTGTTCTCCTG AGCCTTCAGGAGCCTGCACCAGTCCTGCCTGTCCTTCTACTCAGCTGTTACCCACTCTGGGACCAGCAGTCT TTCTGATAACTGGGAGAGGGCAGTAAGGAGGACTTCCTGGAGGGGGTGACTGTCCAGAGCCTGGAACTGTG CCCACACCAGAAGCCATCAGCAGCAAGGACACCATGCGGCTTCCGGGTGCGATGCCAGCTCTGGCCCTCA AAGGCGAGCTGCTGTTGCTGTCTCTCCTGTTACTTCTGGAACCACAGATCTCTCAGGGCCTGGTCGTCACAC CCCCGGGGCCAGAGCTTGTCCTCAATGTCTCCAGCACCTTCGTTCTGACCTGCTCGGGTTCAGCTCCGGTG GTGTGGGAACGGATGTCCCAGGAGCCCCCACAGGAAATGGCCAAGGCCCAGGATGGCACCTTCTCCAGC GTGCTCACACTGACCAACCTCACTGGGCTAGACACGGGAGAATACTTTTGCACCCACAATGACTCCCGTGGA CTGGAGACCGATGAGCGGAAACGGCTCTACATCTTTGTGCCAGATCCCACCGTGGGCTTCCTCCCTAATGAT GCCGAGGAACTATTCATCTTTCTCACGGAAATAACTGAGATCACCATTCCATGCCGAGTAACAGACCCACAGC TGGTGGTGACACTGCACGAGAAGAAAGGGGACGTTGCACTGCCTGTCCCCTATGATCACCAACGTGGCTTTT CTGGTATCTTTGAGGACAGAAGCTACATCTGCAAAACCACCATTGGGGACAGGGAGGTGGATTCTGATGCCTA CTATGTCTACAGACTCCAGGTGTCATCCATCAACGTCTCTGTGAACGCAGTGCAGACTGTGGTCCGCCAGGG TGAGAACATCACCCTCATGTGCATTGTGATCGGGAATGAGGTGGTCAACTTCGAGTGGACATACCCCCGCAA AGAAAGTGGGCGGCTGGTGGAGCCGGTGACTGACTTCCTCTTGGATATGCCTTACCACATCCGCTCCATCCT GCACATCCCCAGTGCCGAGTTAGAAGACTCGGGGACCTACACCTGCAATGTGACGGAGAGTGTGAATGACC ATCAGGATGAAAAGGCCATCAACATCACCGTGGTTGAGAGCGGCTACGTGCGGCTCCTGGGAGAGGTGGG CACACTACAATTTGCTGAGCTGCATCGGAGCCGGACACTGCAGGTAGTGTTCGAGGCCTACCCACCGCCCA CTGTCCTGTGGTTCAAAGACAACCGCACCCTGGGCGACTCCAGCGCTGGCGAAATCGCCCTGTCCACGCG CAACGTGTCGGAGACCCGGTATGTGTCAGAGCTGACACTGGTTCGCGTGAAGGTGGCAGAGGCTGGCCACT ACACCATGCGGGCCTTCCATGAGGATGCTGAGGTCCAGCTCTCCTTCCAGCTACAGATCAATGTCCCTGTCC GAGTGCTGGAGCTAAGTGAGAGCCACCCTGACAGTGGGGAACAGACAGTCCGCTGTCGTGGCCGGGGCAT GCCCCAGCCGAACATCATCTGGTCTGCCTGCAGAGACCTCAAAAGGTGTCCACGTGAGCTGCCGCCCACG CTGCTGGGGAACAGTTCCGAAGAGGAGAGCCAGCTGGAGACTAACGTGACGTACTGGGAGGAGGAGCAGG AGTTTGAGGTGGTGAGCACACTGCGTCTGCAGCACGTGGATCGGCCACTGTCGGTGCGCTGCACGCTGCG CAACGCTGTGGGCCAGGACACGCAGGAGGTCATCGTGGTGCCACACTCCTTGCCCTTTAAGGTGGTGGTGA TCTCAGCCATCCTGGCCCTGGTGGTGCTCACCATCATCTCCCTTATCATCCTCATCATGCTTTGGCAGAAGAA GCCACGTTACGAGATCCGATGGAAGGTGATTGAGTCTGTGAGCTCTGACGGCCATGAGTACATCTACGTGGA CCCCATGCAGCTGCCCTATGACTCCACGTGGGAGCTGCCGCGGGACCAGCTTGTGCTGGGACGCACCCTC GGCTCTGGGGCCTTTGGGCAGGTGGTGGAGGCCACGGCTCATGGCCTGAGCCATTCTCAGGCCACGATGA AAGTGGCCGTCAAGATGCTTAAATCCACAGCCCGCAGCAGTGAGAAGCAAGCCCTTATGTCGGAGCTGAAG ATCATGAGTCACCTTGGGCCCCACCTGAACGTGGTCAACCTGTTGGGGGCCTGCACCAAAGGAGGACCCAT CTATATCATCACTGAGTACTGCCGCTACGGAGACCTGGTGGACTACCTGCACCGCAACAAACACACCTTCCT GCAGCACCACTCCGACAAGCGCCGCCCGCCCAGCGCGGAGCTCTACAGCAATGCTCTGCCCGTTGGGCT |
| | CCCCCTGCCCAGCCATGTGTCCTTGACCGGGGAGAGCGACGGTGGCTACATGGACATGAGCAAGGACGAG TCGGTGGACTATGTGCCCATGCTGGACATGAAAGGAGACGTCAAATATGCAGACATCGAGTCCTCCAACTAC ATGGCCCCTTACGATAACTACGTTCCCTCTGCCCCTGAGAGGACCTGCCGAGCAACTTTGATCAACGAGTCT CCAGTGCTAAGCTACATGGACCTCGTGGGCTTCAGCTACCAGGTGGCCAATGGCATGGAGTTTCTGGCCTCC AAGAACTGCGTCCACAGAGACCTGGCGGCTAGGAACGTGCTCATCTGTGAAGGCAAGCTGGTCAAGATCTG TGACTTTGGCCTGGCTCGAGACATCATGCGGGACTCGAATTACATCTCCAAAGGCAGCACCTTTTTGCCTTTA AAGTGGATGGCTCCGGAGAGCATCTTCAACAGCCTCTACACCACCCTGAGCGACGTGTGGTCCTTCGGGAT CCTGCTCTGGGAGATCTTCACCTTGGGTGGCACCCCTTACCCAGAGCTGCCCATGAACGAGCAGTTCTACAA TGCCATCAAACGGGGTTACCGCATGGCCCAGCCTGCCCATGCCTCCGACGAGATCTATGAGATCATGCAGA AGTGCTGGGAAGAGAAGTTTGAGATTCGGCCCCCCTTCTCCCAGCTGGTGCTGCTTCTCGAGAGACTGTTGG GCGAAGGTTACAAAAAGAAGTACCAGCAGGTGGATGAGGAGTTTCTGAGGAGTGACCACCCAGCCATCCTTC GGTCCCAGGCCCGCTTGCCTGGGTTCCATGGCCTCCGATCTCCCCTGGACACCAGCTCCGTCCTCTATACT GCCGTGCAGCCCAATGAGGGTGACAACGACTATATCATCCCCCTGCCTGACCCCAAACCCGAGGTTGCTGA CGAGGGCCCACTGGAGGGTTCCCCCAGCCTAGCCAGCTCCACCCTGAATGAAGTCAACACCTCCTCAACC ATCTCCTGTGACAGCCCCCTGGAGCCCCAGGACGAACCAGAGCCAGAGCCCCAGCTTGAGCTCCAGGTG GAGCCGGAGCCAGAGCTGGAACAGTTGCCGGATTCGGGGTGCCCTGCGCCTCGGGCGGAAGCAGAGGAT AGCTTCCTGTAGGGGGCTGGCCCCTACCCTGCCCTGCCTGAAGCTCCCCCCCTGCCAGCACCCAGCATCT CCTGGCCTGGCCTGACCGGGCTTCCTGTCAGCCAGGCTGCCCTTATCAGCTGTCCCCTTCTGGAAGCTTTCT GCTCCTGACGTGTTGTGCCCCAAACCCTGGGGCTGGCTTAGGAGGCAAGAAAACTGCAGGGGCCGTGACC AGCCCTCTGCCTCCAGGGAGGCCAACTGACTCTGAGCCAGGGTTCCCCCAGGGAACTCAGTTTTCCCATAT GTAAAATGGGAAAGTTAGGCTTGATGACCCAGAATCTAGGATTCTCTCCCTGGCTGACAGGTGGGGAGACCG AATCCCTCCCTGGGAAGATTCTTGGAGTTACTGAGGTGGTAAATTAACTTTTTTCTGTTCAGCCAGCTACCCCTC AAGGAATCATAGCTCTCTCCTCGCACTTTTATCCACCCAGGAGCTAGGGAAGAGACCCTAGCCTCCCTGGCT GCTGGCTGAGCTAGGGCCTAGCCTTGAGCAGTGTTGCCTCATCCAGAAGAAAGCCAGTCTCCTCCCTATGAT GCCAGTCCCTGCGTTCCCTGGCCCGAGCTGGTCTGGGGCCATTAGGCAGCCTAATTAATGCTGGAGGCTGA GCCAAGTACAGGACACCCCCAGCCTGCAGCCCTTGCCCAGGGCACTTGGAGCACACGCAGCCATAGCAA GTGCCTGTGTCCCTGTCCTTCAGGCCCATCAGTCCTGGGGCTTTTTCTTTATCACCCTCAGTCTTAATCCATCC ACCAGAGTCTAGAAGGCCAGACGGGCCCCGCATCTGTGATGAGAATGTAAATGTGCCAGTGTGGAGTGGCC ACGTGTGTGTGCCAGTATATGGCCCTGGCTCTGCATTGGACCTGCTATGAGGCTTTGGAGGAATCCCTCACC CTCTCTGGGCCTCAGTTTCCCCTTCAAAAAATGAATAAGTCGGACTTATTAACTCTGAGTGCCTTGCCAGCACT AACATTCTAGAGTATTCCAGGTGGTTGCACATTTGTCCAGATGAAGCAAGGCCATATACCCTAAACTTCCATCC TGGGGGTCAGCTGGGCTCCTGGGAGATTCCAGATCACACATCACACTCTGGGGACTCAGGAACCATGCCCC TTCCCCAGGCCCCCAGCAAGTCTCAAGAACACAGCTGCACAGGCCTTGACTTAGAGTGACAGCCGGTGTCC TGGAAAGCCCCCAGCAGCTGCCCCAGGGACATGGGAAGACCACGGGACCTCTTTCACTACCCACGATGAC CTCCGGGGGTATCCTGGGCAAAAGGGACAAAGAGGGCAAATGAGATCACCTCCTGCAGCCCACCACTCCA GCACCTGTGCCGAGGTCTGCGTCGAAGACAGAATGGACAGTGAGGACAGTTATGTCTTGTAAAAGACAAGAA GCTTCAGATGGGTACCCCAAGAAGGATGTGAGAGGTGGGCGCTTTGGAGGTTTGCCCCTCACCCACCAGCT GCCCCATCCCTGAGGCAGCGCTCCATGGGGGTATGGTTTTGTCACTGCCCAGACCTAGCAGTGACATCTCAT TGTCCCCAGCCCAGTGGGCATTGGAGGTGCCAGGGGAGTCAGGGTTGTAGCCAAGACGCCCCCGCACGG GGAGGGTTGGGAAGGGGGTGCAGGAAGCTCAACCCCTCTGGGCACCAACCCTGCATTGCAGGTTGGCACC TTACTTCCCTGGGATCCCCAGAGTTGGTCCAAGGAGGGAGAGTGGGTTCTCAATACGGTACCAAAGATATAAT CACCTAGGTTTACAAATATTTTTAGGACTCACGTTAACTCACATTTATACAGCAGAAATGCTATTTTGTATGCTGTT GAGTTTTTCTATCTGTGTACTTTTTTTTAAGGGAAAGATTTTAATATTAAACCTGGTGCTTCTCACTCACA |

## Claims

1. A method for identyfying a cancer, preferably a head-and-neck cancer, comprising
a) isolating small extracellular vesicles (sEVs) from a biological sample;
b) isolating RNA molecules from the sEVs obtained;
wherein said RNA molecules comprises a biomarker set of messenger RNAs (mRNA) and/or microRNAs (miRNA);
c) determining the presence and/or amount of mRNAs and/or miRNAs;
d) relating the presence and/or amount of mRNAs and/or miRNAs to a reference, wherein the cancer, preferably a head-and-neck cancer, is identified on basis of a measurable and significant difference in the presence and/or amount of mRNAs and/or miRNAs as compared to the reference.

2. A method of detecting a set of biomarkers associated with a cancer, preferably a head-and-neck cancer, comprising
a) isolating small extracellular vesicles (sEVs) from a biological sample;
b) contacting said sEVs to one or more than one reagent that specifically binds to one or more than one biomarker; and
c) determining whether or not the biomarker is differentially expressed in the sample by comparing the expression level of the biomarker in the biological sample to the expression level of said biomarker for at least one reference sample, wherein the reference sample is a comparable biological sample obtained from a disease-free subj ect.

3. A biomarker set associated with cancer, preferably a head-and-neck cancer, detected by the method according to claim 2.

4. A biomarker set comprising biomarkers represented by mRNAs and/or miRNAs, preferably wherein the biomarker set consists of biomarkers represented by mRNAs and/or miRNAs.

5. The biomarker set comprising biomarkers according to any of claims 3-4, wherein the biomarker set comprises biomarkers represented by mRNAs and miRNAs, wherein the biomarker set comprises C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, and RPS6, preferably wherein the biomarker set consists of C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, and RPS6.

6. The biomarker set comprising biomarkers according to any of claims 3-4, wherein the biomarker set comprises biomarkers represented by mRNAs, wherein the biomarker set comprises MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, and LCK, preferably comprises STAT6, TLR10, S100A8, TNFSF11, CYLD, ITGAL, BATF, ICAM3, STAT2, RPS6, CDKN1A, TMEFF2, HLA-C, LCK, and PSMB9.

7. The biomarker set according to claim 6, wherein the biomarker set consists of biomarkers represented by mRNAs, wherein the biomarker set consists of MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, and LCK, preferably consists of STAT6, TLR10, S100A8, TNFSF11, CYLD, ITGAL, BATF, ICAM3, STAT2, RPS6, CDKN1A, TMEFF2, HLA-C, LCK, and PSMB9.

8. The biomarker set comprising biomarkers according to any of claims 3-4, wherein the biomarker set comprises biomarkers represented by miRNAs, wherein the biomarker set comprises hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, and hsa-miR-216a-5p, preferably comprises hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, and hsa-miR-216a-5p.

9. The biomarker set according to claim 8, wherein the biomarker set consists of biomarkers represented by miRNAs, wherein the biomarker set consists of hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, and hsa-miR-216a-5p, preferably consists of hsa-miR-1283, hsa-miR-518b, hsa-miR-1246, hsa-miR-411-5p, hsa-miR-3127-5p, hsa-miR-590-3p, hsa-miR-548ah-5p, hsa-miR-495-3p, hsa-miR-181b-2-3p, hsa-miR-142-3p, hsa-miR-543, and hsa-miR-216a-5p.

10. The biomarker set comprising biomarkers according to any of claims 3-4, wherein the biomarker set comprises biomarkers represented by a combination of mRNAs and miRNAs, wherein the biomarker set comprises hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let7a-5p, hsa-miR-605-5p, hsa-miR-1246, hsa-miR-96-5p, C6, and hsa-miR-1286, preferably comprises hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, and hsa-miR-1286.

11. The biomarker set according to claim 10, wherein the biomarker set consists of biomarkers represented by a combination of mRNAs and miRNAs, wherein the biomarker set consists of hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let7a-5p, hsa-miR-605-5p, hsa-miR-1246, hsa-miR-96-5p, C6, and hsa-miR-1286, preferably consists of hsa-miR-1283, hsa-miR-508-3p, hsa-miR-223-3p, hsa-miR-16-5p, STAT6, hsa-miR-600, hsa-miR-1305, hsa-let-7a-5p, hsa-miR-96-5p, and hsa-miR-1286.

12. Use of a biomarker set according to any of claims 3 - 11 in the method for identification of a cancer, preferably a head-and-neck cancer according to claim 1.

13. The method for identifying a cancer, preferably a head-and-neck cancer, according to claim 1, the method of detecting a set of biomarkers according to claim 2, the biomarker set according to any of claims 3 - 11, and the use of a biomarker set according to claim 12, preferably wherein the head-and-neck cancer is a head-and-neck squamous cell carcinoma (HNSCC).

14. The methods according to any of claims 1-2, wherein said biological sample is a sample selected from the group consisting of saliva, urine, whole blood, white blood cells, blood plasma, blood serum, cerebrospinal fluid, and lymphatic fluid.

15. The methods according to any of claims 1-2, wherein said small extracellular vesicles (sEVs) are shed from cancer, preferably head-and-neck cancer cells.

16. The methods according to any of claims 1-2, wherein said small extracellular vesicles (sEVs) are isolated by use of size-exclusion chromatography, affinity selection using a binding reagent which specifically binds to an sEV surface antigen, ultrafiltration, or (ultra)centrifugation.

17. The methods according to any of claims 1-2, wherein said biomarkers are detected and/or identifed by use of nano-string analysis or polymerase chain reaction (PCR) analysis.

18. The method according to claim 2, wherein in step c) an SVM (support vector machine)-based classifier and/or a logistic regression model is developed, validated and used for training.

19. The methods according to any of claims 1, 2, and 13 - 18, wherein said methods are automated.

20. The methods according to any of claims 1-2, wherein one or more than one biomarker or biomarker set identifies a particular disease level (stage) of the cancer, preferably of the head-and-neck cancer.

21. The method according to claim 1, wherein one or more than one biomarker or biomarker set identifies the presence of a human papillomavirus (HPV) associated with the cancer, preferably with the head-and-neck cancer, and preferably wherein the HPV-specific biomarker is an HPV-specific ribonucleic acid.

22. The biomarker set comprising biomarkers according to any of claims 5 - 11, wherein the sequence identity of each biomarker represented by an individual mRNA or miRNA in the biomarker set is at least 80%, or at least 85%, or at least 90%, or at least 95%, or at least 96%, or at least 97%, or at least 98%, or at least 99% as compared to the respective nucleotide sequence listed in the present Sequence Listing.

23. A biomarker set comprising at least 60%, or at least 65%, or at least 70%, or at least 75%, or at least 80%, or at least 85%, or at least 90%, or at least 95%, or at least 97% of the group of biomarkers selected from C6, FADD, PSMB9, hsa-miR-128-1-5p, CLEC4A, IL13RA1, CD79B, hsa-miR-411-5p, hsa-miR-1204, hsa-miR-605-5p, IFNAR1, IL17RA, ITGAL, hsa-miR-508-3p, GZMH, hsa-miR-33a-5p, hsa-miR-590-5p, PDGFRB, hsa-miR-574-5p, and RPS6.

24. A biomarker set comprising at least 60%, or at least 65%, or at least 70%, or at least 75%, or at least 80%, or at least 85%, or at least 90%, or at least 95%, or at least 97% of the group of biomarkers selected from MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, and LCK.

25. A biomarker set comprising at least 60%, or at least 65%, or at least 70%, or at least 75%, or at least 80%, or at least 85%, or at least 90%, or at least 95%, or at least 97% of the group of biomarkers selected from hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, and hsa-miR-216a-5p.

26. A biomarker set comprising at least 60%, or at least 65%, or at least 70%, or at least 75%, or at least 80%, or at least 85%, or at least 90%, or at least 95%, or at least 97% of the group of biomarkers selected from hsa-miR-1283, hsa-miR-508- 3p, hsa-miR-223-3p, CD79B, hsa-miR-299-5p, hsa-miR-183-5p, hsa-miR-16-5p, MAPK14, STAT6, hsa-miR-600, hsa-miR-23a-3p, hsa-miR-325, hsa-miR-376a-3p, hsa-miR-1305, hsa-let7a-5p, hsa-miR-605-5p, hsa-miR-1246, hsa-miR-96-5p, C6, and hsa-miR-1286.

27. A biomarker set, suited for the detection of cancer, preferably of a head-and-neck cancer, comprising five or more biomarkers, selected from the group of biomarkers consisting of the miRNAs hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p, hsa-miR-223-3p, hsa-miR-183-5p, hsa-miR-16-5p, hsa-miR-23a-3p, hsa-miR-325, hsa-miR-1305, hsa-let-7a-5p, hsa-miR-605-5p, hsa-miR-96-5p, hsa-miR-1286, hsa-miR-1204, hsa-miR-33a-5p, hsa-miR-590-5p, hsa-miR-574-5p
and/or mRNAs MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK, C6, CLEC4A, IL13RA1, IL17RA, GZMH, PDGFRB.

28. Use of a biomarker set for the detection of cancer, preferably of a head-and-neck cancer, wherein the biomarker set comprises five or more biomarkers, selected from the group of biomarkers consisting of
the miRNAs hsa-miR-1283, hsa-miR-508-3p, hsa-miR-518b, hsa-miR-1246, hsa-miR-362-3p, hsa-miR-128-1-5p, hsa-miR-411-5p, hsa-miR-3127-5p, hsa-miR-590-3p, hsa-miR-548ah-5p, hsa-miR-495-3p, hsa-miR-376a-3p, hsa-miR-181b-2-3p, hsa-miR-378h, hsa-miR-142-3p, hsa-miR-543, hsa-miR-600, hsa-miR-299-5p, hsa-miR-1271-5p, hsa-miR-216a-5p, hsa-miR-223-3p, hsa-miR-183-5p, hsa-miR-16-5p, hsa-miR-23a-3p, hsa-miR-325, hsa-miR-1305, hsa-let-7a-5p, hsa-miR-605-5p, hsa-miR-96-5p, hsa-miR-1286, hsa-miR-1204, hsa-miR-33a-5p, hsa-miR-590-5p, hsa-miR-574-5p and/or the mRNAs MAPK14, STAT6, TLR10, CD79B, S100A8, FADD, TNFSF11, CYLD, ITGAL, BATF, IFNAR1, ICAM3, STAT2, RPS6, TNFRSF12A, CDKN1A, TMEFF2, PSMB9, HLA-C, LCK, C6, CLEC4A, IL13RA1, IL17RA, GZMH, PDGFRB.
